(19) Europäisches Patentamt
European Patent Office
Office européen des brevets

(11) EP 0 850 920 B1

(12) FASCICULE DE BREVET EUROPEEN

(45) Date de publication et mention de la délivrance du brevet:
**11.09.2002 Bulletin 2002/37**

(21) Numéro de dépôt: **97403187.4**

(22) Date de dépôt: **30.12.1997**

(51) Int Cl.[7]: **C07C 311/48**, C07C 311/09, C07D 307/64, C07D 303/34, C07D 407/04, C07D 207/452, C07D 213/76, C07D 285/135, C07D 251/70, C07D 219/10, C07D 311/82

# (54) Matériaux à conduction ionique contenant un sel d'amides perfluorés et leurs utilisations

Ionisch leitfähige Materialien, die Salze von perfluorierten Amiden enthalten, und ihre Verwendungen

Ionic conductive materials comprising salts of perfluorinated amides and uses thereof

(84) Etats contractants désignés:
**DE FR GB IT**

(30) Priorité: **30.12.1996 CA 2194127**
**05.03.1997 CA 2199231**

(43) Date de publication de la demande:
**01.07.1998 Bulletin 1998/27**

(73) Titulaires:
- **CENTRE NATIONAL DE LA RECHERCHE SCIENTIFIQUE (CNRS)**
  **75016 Paris (FR)**
- **HYDRO-QUEBEC**
  **Montréal Québec H2Z 1A4 (CA)**

(72) Inventeurs:
- **Armand, Michel**
  **Montreal, Québec H3T 1N2 (CA)**
- **Choquette, Yves**
  **Sainte-Julie, Québec J3E 1P4 (CA)**
- **Gauthier, Michel**
  **La Prairie, Québec J5R 1E6 (CA)**
- **Michot, Christophe**
  **38000 Grenoble (FR)**

(74) Mandataire: **Sueur, Yvette et al**
**Cabinet SUEUR & L'HELGOUALCH,**
**109, boulevard Haussmann**
**75008 Paris (FR)**

(56) Documents cités:

| | |
|---|---|
| **EP-A- 0 096 629** | **EP-A- 0 731 518** |
| **EP-A- 0 779 671** | **WO-A-88/03331** |
| **WO-A-92/16028** | **WO-A-93/16988** |
| **WO-A-95/26056** | **GB-A- 1 306 564** |

- **JOURNAL OF FLUORINE CHEMISTRY, vol. 46, no. 2, Février 1990, LAUSANNE, CH, pages 297-305, XP002062043 R.E. BANKS ET AL.: "N-Halogeno compounds. Part 11. Perfluoro-[N-fluoro-N-(4-pyridyl)-methanesulphonamide], a powerful new electrophilic fluorinating agent"**
- **JOURNAL OF FLUORINE CHEMISTRY, vol. 68, no. 3, Septembre 1994, LAUSANNE, CH, pages 277-86, XP002062044 V.A. PETROV ET AL.: "Synthesis of polyfluoro-2-alkanesulfonyl-3,3-dialkoxyaziridines"**
- **JOURNAL OF MEDICINAL CHEMISTRY, vol. 30, no. 4, Avril 1987, WASHINGTON, DC, US, pages 696-704, XP002062045 R. LIS ET AL.: "Synthesis and antiarrhythmic activity of novel 3-alkyl-1-[omega-[4-[(alkylsulfonyl)amino] phenyl]-omega-hydroxyalkyl]-1H-imidazolium salts and related compounds"**
- **INORGANIC AND NUCLEAR CHEMISTRY LETTERS, vol. 7, no. 2, 1971, pages 171-175, XP000618718 H.W. ROESKY ET AL: "Darstellung von N-Trifluormethansulfonyl-sulfonyl fluoridamid und einige Reaktionen"**
- **JOURNAL OF FLUORINE CHEMISTRY, vol. 72, no. 2, Juin 1995, LAUSANNE, CH, pages 203-208, XP002025806 D.D. DESMARTEAU: "Novel perfluorinated ionomers and ionenes"**
- **JOURNAL OF FLUORINE CHEMISTRY, vol. 52, no. 1, 1 Avril 1991, LAUSANNE, CH, pages 7-12, XP002025807 D.D. DESMARTEAU ET AL: "N-Fluoro-bis(trifluoromethanesulfonyl)imi de. An improved synthesis"**

Il est rappelé que: Dans un délai de neuf mois à compter de la date de publication de la mention de la délivrance du brevet européen, toute personne peut faire opposition au brevet européen délivré, auprès de l'Office européen des brevets. L'opposition doit être formée par écrit et motivée. Elle n'est réputée formée qu'après paiement de la taxe d'opposition. (Art. 99(1) Convention sur le brevet européen).

EP 0 850 920 B1

Printed by Jouve, 75001 PARIS (FR)

## Description

La présente invention a pour objet des matériaux à conduction ionique contenant un composé ionique dans lequel la charge anionique est délocalisée, et leurs utilisations.

Il est connu et particulièrement intéressant d'introduire des groupements ioniques dans les molécules ou les polymères organiques possédant des fonctions variées. Les forces coulombiennes correspondent, en effet, aux interactions les plus fortes disponibles au niveau moléculaire, et les groupements ioniques modifient de la manière la plus marquée les molécules auxquelles ils sont attachés. On peut citer les colorants qui sont rendus solubles dans l'eau à l'aide de fonctions sulfonates ou carboxylates.

Cependant les groupements de ce type $-CO_2^-$ $1/mM^{m+}$ ou $-SO_3^-$ $1/mM^{m+}$ ne sont pas dissociés, et ils n'induisent pas de solubilité dans les solvant autres que l'eau ou certains solvants protiques très polaires comme les alcools légers, ce qui restreint considérablement la portée de leur utilisation.

On connaît par ailleurs les sels des composés $[R_FSO_2\text{-}N\text{-}SO_2R_F]^-$ $1/mM^{m+}$ dans lesquels $R_F$ est un groupement perfluoré et $M^{m+}$ un cation à la valence m+ qui sont solubles et dissociés dans les milieux aprotiques organiques ou les polymères solvatants. Il est cependant considéré que l'existence de deux groupements perfluoroalkylesulfonyles (en particulier l'existence d'atomes de fluor sur l'atome de carbone en $\alpha$ de chacun des groupements sulfonyles) qui exercent un pouvoir attracteur important sur les électrons de la charge anionique, est une condition nécessaire à l'obtention des propriétés de solubilité et de dissociation. Pour exemple, le $pK_a$ de l'acide $H[CF_3SO_2\text{-}N\text{-}SO_2CF_3]$ n'est que de 1,95, comparable à celui de l'acide non fluoré $CH_3SO_3H$ ($pK_a$ = 0,3) et nettement inférieur à celui de l'acide perfluoré $CF_3SO_3H$ ($pK_a$ < -9) du fait de la basicité de l'atome d'azote central par rapport à l'atome d'oxygène des acides sulfoniques. On connait en outre de WO92 16028 des matériaux polymériques à conduction ionique comprenant une solution solide d'un ou plusieurs sels dans un polymère. De plus WO 95 26056 divulgue l'utilisation de composés de type fluorosulfonyl-imidure pour l'élaboration de matériaux à conduction ionique et diverses applications de ces matériaux.

D'une manière surprenante, les inventeurs ont trouvé que les excellentes propriétés de solubilité et de dissociation des groupements - ioniques $-SO_2\text{-}N\text{-}SO_2-$ étaient conservées lorsque un seul groupement sulfoné possédait des atomes de fluor sur des atomes adjacents à l'atome de soufre, laissant un choix de molécules fonctionnelles extrêmement large. D'une manière tout aussi inattendue, il a été constaté qu'il était possible, pour l'obtention des mêmes propriétés, d'omettre le . groupement $-SO_2$ fixé au groupement non perfluoré à condition que le groupe rattaché directement à l'azote ait un paramètre de Hammett $\sigma^*$ supérieur à 0,6. A titre de comparaison, le paramètre de Hammett $\sigma^*$ d'un groupement $-SO_2-$ relié à un groupement non perfluoré est de 3,5 et de 4,55 pour un groupement $CF_3SO_2-$.

Les présents inventeurs ont également trouvé que les groupements sulfonyle $-SO_2-$ pouvaient être remplacés, avec des variations mineures de propriétés, par les groupements sulfinyle -SO- ou phosphonyle -PO=.

La présente invention a par conséquent pour objet des matériaux à conduction ionique contenant un composé ionique possédant une bonne solubilité et une bonne dissociation, sans qu'il soit nécessaire de faire appel à des modifications complexes de la molécule de départ. Les précurseurs des molécules de l'invention se présentent sous forme de dérivés d'acides sulfoniques ou de groupements aminés d'une part, et des dérivés de type perfluorosulfonyle d'autre part, qui sont pour la plupart des produits industriels et/ou facilement accessibles. Il est à noter en outre que la diminution de la fraction perfluorée dans les composés de l'invention permet de réduire les coûts de production desdits composés et par conséquent le coût des applications qui en sont faites.

Un composé utilisé pour un matériau de la présente invention est un composé ionique constitué par une amide ou l'un de ses sels, comprenant une partie anionique associée à au moins une partie cationique $M^{+m}$ en nombre suffisant pour assurer la neutralité électronique de l'ensemble. Il est caractérisé en ce que $M^{m+}$ est un hydroxonium, un nitrosonium $NO^+$, un ammonium $-NH_4^+$, un cation métallique ayant la valence m, un cation organique onium ayant la valence m ou un cation organo-métallique ayant la valence m, et en ce que la partie anionique répond à la formule $R_F\text{-}SO_x\text{-}N^-Z$, dans laquelle :

- le groupement $-SO_x-$ représente un groupement sulfonyle $-SO_2-$ ou un groupement sulfinyle -SO- ;
- $R_F$ est un radical perhalogéné alkyle, alkylaryle, oxa-alkyle, aza-alkyle ou thia-alkyle, ou un radical répondant à l'une des formules $R_ACF_2-$, $R_ACF_2CF_2-$, $R_ACF_2CF(CF_3)-$ ou $CF_3C(R_A)F-$ dans lesquelles $R_A-$ représente un radical organique non perhalogéné choisi parmi les radicaux alkyle, aryle, alkylaryle ou arylalkyle ; les groupes mésomorphes ou les groupes comprenant au moins une insaturation éthylénique et/ou un groupe condensable et/ou un groupe dissociable par voie thermique, par voie photochimique ou par dissociation ionique ; les polymères conducteurs électroniques autodopés ; les alcoxysilanes hydrolysables ; les dipôle dissociants ; les couples rédox ; les ligands complexants ; les groupes chromophores ; les chaînes polymériques portant des greffons comportant un groupe carbonyle, un groupe sulfonyle, un groupe thionyle ou un groupe phosphonyle ; les zwitterions ; les acides aminés ou les polypeptides optiquement ou biologiquement actifs ; les groupements chiraux ;
- Z représente un radical électro-attracteur ayant un paramètre de Hammett au moins égal à celui d'un radical

phényle, choisi parmi :

j) -CN, $-NO_2$, -SCN, $-N_3$, $-CF_3$, $R'_FCH_2$- ($R'_F$ étant un radical perfluoré, de préférence $CF_3$-), les radicaux fluoroalkylthioxy,
jj) les radicaux comprenant un ou plusieurs noyaux aromatiques contenant éventuellement au moins un atome d'azote, d'oxygène, de soufre ou de phosphore, lesdits noyaux pouvant être éventuellement des noyaux condensés et/ou lesdits noyaux pouvant éventuellement porter au moins un substituant choisi parmi les halogènes, -CN, $-NO_2$, -SCN, $-N_3$, $-CF_3$, $CF_3CH_2$-, $CF_2$=CF-O-, $CF_2$=CF-S-, les groupes perfluoroalkyles, les groupes fluoroalkyloxy, les groupes fluoroalkylthioxy, les radicaux alkyles, alkényles, oxa-alkyles, oxa-alkényles, aza-alkyles, aza-alkényles, thia-alkyles, thia-alkényles, les radicaux polymères et les radicaux possédant au moins un groupement ionophore cationique et/ou au moins un groupement ionophore anionique ;

étant entendu qu'un substituant Z peut être un radical monovalent, une partie d'un radical multivalent portant plusieurs groupements $R_F$-$SO_X$-N-, ou un segment d'un polymère ;
ou bien

- Z est un radical $R_D$-Y- dans lequel Y est un groupe sulfonyle-, ou sulfinyle , et $R_D$ est un radical choisi dans le groupe constitué par

a) les radicaux alkyle ou alkényle, les radicaux aryle, arylalkyle, alkylaryle ou alkénylaryle, les radicaux alicycliques ou hétérocycliques, y compris les radicaux polycycliques ;
b) les radicaux alkyle ou alkényle comprenant au moins un groupe fonctionnel éther, thioéther, aminé, imine, carboxyle, carbonyle, hydroxy, silyle, isocyanate ou isothiocyanate ;
c) les radicaux aryle, arylalkyle, arylalkényle, alkylaryle ou alkénylaryle, dans lesquels les noyaux aromatiques et/ou au moins un substituant du noyau comprennent des hétéroatomes tels que l'azote, l'oxygène, le soufre ;
d) les radicaux comprenant des cycles aromatiques condensés qui comprennent éventuellement au moins un hétéroatome choisi parmi l'azote, l'oxygène, le soufre ;
e) les radicaux halogénés alkyle, alkényle, aryle, arylalkyle, alkylaryle ou alkénylaryle dans lesquels le nombre d'atomes de carbone portant au moins un halogène est au plus égal au nombre d'atomes de carbone non halogénés, le carbone en $\alpha$ du groupement Y n'étant pas halogéné lorsque Y est $-SO_2$-, lesdits radicaux comprenant éventuellement des groupes fonctionnels éther, thioéther, aminé, imine, carboxyle, carbonyle, hydroxy, silylalkyle, silylaryle, isocyanate ou isothiocyanate ;
f) les radicaux $R_CC(R')$ (R")-O- dans lesquels $R_C$ est un radical alkyle perfluoré et R' et R" sont indépendamment l'un de l'autre un atome d'hygrogène ou un radical tel que défini en a), b), c) ou d) ci-dessus [par exemple $CF_3CH_2O$-, $(CF_3)_3CO$-, $(CF_3)_2CHO$-, $CF_3CH(C_6H_5)O$-, $-CH_2(CF_2)_2CH_2$-] ;
g) les radicaux $(R_B)_2N$-, dans lesquels les radicaux $R_B$ identiques ou différents sont tels que définis en a), b), c), d) et e) ci-dessus, l'un dès $R_B$ pouvant être un atome d'hydrogène, ou bien les deux radicaux $R_B$ forment ensemble un radical divalent qui forme un cycle avec N ;
h) les radicaux constitués par une chaîne polymère,
i) les radicaux possédant un ou plusieurs groupements ionophores cationiques et/ou un ou plusieurs groupements ionophores anioniques ; étant entendu qu'un substituant $R_D$ peut être un radical monovalent, une partie d'un radical multivalent portant plusieurs groupements $R_FSO_x$-N-Y-, ou un segment d'un polymère ;

- étant entendu que, lorsque Y est un sulfonyle et que $R_D$ est un radical tel que défini en a), $R_F$ est $R_ACF_2$-, $R_ACF_2CF_2$-, $R_ACF_2CF(CF_3)$-, $CF_3C(R_A)F$- ou un radical perhaloalkyle ayant de 1 à 2 atomes de carbone ne favorisant pas une séparation de phase due à l'agrégation des segments fluorés ;
- étant entendu que lorsqu'un radical est un polymère ou partie d'un polymère, ledit polymère est un polyalkylène portant éventuellement des substituants, un polystyrène, un poly(fluoroéthylène), un polyacrylate, un polyester, un poly(oxyalkylène), un poly(alcoxysilyle, une polyaniline, un polymère ayant des unités récurrentes dérivées de 2[2-(3-thiényl)éthoxy]éthyle ou de 3-maléimidopropyle.

Dans un composé de la présente invention, le cation peut être un cation métallique choisi parmi les cations de métaux alcalins, les cations de métaux alcalino-terreux, les cations de métaux de transition, les cations de métaux trivalents, les cations de terres rares. A titre d'exemple, on peut citer $Na^+$, $Li^+$, $K^+$, $Sm^{3+}$, $La^{3+}$, $Ho^{3+}$, $Sc^{3+}$, $Al^{3+}$, $Y^{3+}$, $Yb^{3+}$, $Lu^{3+}$, $Eu^{3+}$.

Le cation peut également être un cation organo-métallique, notamment un métallocénium. A titre d'exemple, on peut citer les cations dérivés du ferrocène, du titanocène, du zirconocène, d'un indénocénium ou d'un arène métallocénium, les cations des métaux de transition complexés par des ligands de type phosphine possédant éventuellement une chiralité, les cations organométalliques possédant un ou plusieurs groupements alkyles ou aryles fixés

d'une manière covalente à un atome ou un groupe d'atome, tels les cations méthylzinc, phénylmercure, trialkylétain ou trialkylplomb. Le cation organo-métallique peut faire partie d'une chaîne polymère.

Selon une variante de l'invention, les composés de l'invention ont un cation organique choisi dans le groupe constitué par les cations $R_3O^+$ (oxonium), $NR_4^+$ (ammonium), $RC(NHR_2)_2^+$ (amidinium), $C(NHR_2)_3^+$ (guanidinium), $C_5R_6N^+$ (pyridinium), $C_3R_5N_2^+$ (imidazolium), $C_3R_7N_2^+$ (imidazolinium), $C_2R_4N_3^+$ (triazolium), $SR_3^+$ (sulfonium), $PR_4^+$ (phosphonium), $IR_2^+$ (iodonium), $(C_6R_5)_3C^+$ (carbonium). Dans un cation donné, les radicaux R peuvent être tous identiques. Mais un cation peut aussi comporter des radicaux R différents les uns des autres. Un radical R peut être un H ou bien il est choisi parmi les radicaux suivants :

- les radicaux alkyles, alkényles, oxa-alkyles, oxa-alkényles, azaalkyles, azaalkényles, thiaalkyles, thia-alkényles, silaalkyles, silaalkényles, aryles, arylalkyles, alkyl-aryles, alkényl-aryles, dialkylamino et dialkylazo ;
- les radicaux cycliques ou hétérocycliques comprenant éventuellement au moins une chaîne latérale comprenant des hétéroatomes tels que l'azote, l'oxygène, le soufre ;
- les radicaux cycliques ou hétérocycliques comprenant éventuellement des hétéroatomes dans le noyau aromatique ;
- les groupes comprenant plusieurs noyaux aromatiques ou hétérocycliques, condensés ou non, contenant éventuellement au moins un atome d'azote, d'oxygène, de soufre ou de phosphore.

Lorsqu'un cation onium porte au moins deux radicaux R différents de H, ces radicaux peuvent former ensemble un cycle aromatique ou non, englobant éventuellement le centre portant la charge cationique.

Lorsque la partie cationique d'un composé de l'invention est un cation onium, il peut se présenter soit sous la forme d'un groupe cationique indépendant qui n'est lié à la partie anionique que par la liaison ionique entre la charge positive du cation et la charge négative de la partie anionique. Dans ce cas, la partie cationique peut faire partie d'une unité récurrente d'un polymère.

Un cation onium peut également faire partie du radical Z ou du radical $R_D$ porté par le centre anionique. Dans ce cas, un composé de l'invention constitue un zwitterion.

Lorsque le cation d'un composé de l'invention est un cation onium, il peut être choisi de telle sorte à introduire dans le composé des substituants permettant de conférer audit composé des propriétés spécifiques. Par exemple, le cation $M^+$ peut être un hétérocycle cationique à caractère aromatique, comportant au moins un atome d'azote alkylé dans le cycle. A titre d'exemple, on peut citer un imidazolium, un triazolium, un pyridinium, un 4-diméthylamino-pyridinium, lesdits cations portant éventuellement un substituant sur les atomes de carbone du cycle. Parmi ces cations, ceux qui donnent un composé ionique selon l'invention dont le point de fusion est inférieur à 150°C sont particulièrement préférés. Un tel composé possédant une température de fusion basse est particulièrement utile pour l'élaboration de matériaux à conduction protonique. Un matériau à conduction protonique particulièrement préféré comprend un composé selon l'invention dans lequel le cation est formé par addition d'un proton sur l'azote d'une imidazoline, d'un imidazole ou d'un triazole, ainsi que la base azotée correspondante dans une proportion de 0,5 à 10 en rapport molaire.

Un composé de l'invention dans lequel le cation M est un groupement cationique possédant une liaison -N=N-, $-N=N^+$, un groupement sulfonium, un groupement iodonium, ou un cation arène-ferrocénium substitué ou non, éventuellement incorporé dans une trame polymérique, est intéressant dans la mesure où il est activable par une source d'énergie actinique de longueur d'onde appropriée. Comme exemples particuliers de tels composés, on peut citer ceux dans lesquels le cation est un cation diaryliodonium, un cation dialkylaryliodonium, un cation triarylsulfonium, un cation trialkylaryle sulfonium, ou un cation phénacyl-dialkyl sulfonium substitué ou non. Les cations précités peuvent faire partie d'une chaîne polymère.

Le cation M d'un composé de l'invention peut incorporer un groupement 2,2' [Azobis (2-2'-imidazolinio-2-yl) propane]$^{2+}$ ou 2,2'-Azobis(2-amidiniopropane)$^{2+}$. Le composé de l'invention est alors capable de libérer, sous l'action de la chaleur ou d'un rayonnement ionisant, des radicaux qui permettent d'initier des réactions de polymérisation, de réticulation ou, d'une manière générale, des réactions chimiques mettant en jeu des radicaux libres. De plus, ces composés sont aisément solubles dans les solvants organiques polymères et monomères même de faible polarité, contrairement aux dérivés des anions de type $Cl^-$ habituellement associés à ce type de composés. Ils présentent par ailleurs une pression de vapeur négligeable contrairement aux autres amorceurs radicalaires de type peroxyde ou azo, ce qui est un avantage considérable pour la mise en oeuvre de polymères en films minces, la volatilité de l'amorceur ayant pour conséquence une mauvaise polymérisation ou réticulation de la surface du film.

Dans un mode de réalisation de l'invention, $R_F$ est un radical alkyle perhalogéné ayant de préférence de 1 à 12 atomes de carbone, ou un radical alkylaryle perhalogéné ayant de préférence de 6 à 9 atomes de carbone. Le radical alkyle perhalogéné peut être un radical linéaire ou ramifié. On peut citer en particulier les radicaux dans lesquels l'atome de carbone qui sera en position $\alpha$ par rapport au groupe $-SO_x-$ porte au moins un atome de fluor. Comme exemple de tels radicaux, on peut citer $R_ACF_2-$, $R_ACF_2CF_2-$, $R_ACF_2CF(CF_3)-$ ou $CF_3C(R_A)F-$ dans lesquels $R_A$ représente un radical organique non perhalogéné, un groupe alkyle, un groupe aryle, un groupe alkylaryle ou arylalkyle ;

un groupe comprenant au moins une insaturation éthylénique et/ou un groupe condensable et/ou un groupe dissociable ; un groupe mésomorphe ; un groupe chromophore ; un polymère conducteur électronique autodopé ; un alcoxysilane hydrolysable ; une chaîne polymérique portant des greffons comportant un groupe carbonyle, un groupe sulfonyle, un groupe thionyle ou un groupe phosphonyle ; ; un dipôle dissociant tel qu'une amide, une sulfonamide ou un nitrile ; un couple rédox tel qu'un disulfure, une thioamide, un ferrocène, une phéno-thiazine, un groupe bis(dialkylaminoaryle), un nitroxyde ou un imide aromatique ; un ligand complexant ; un zwitterion ; un acide aminé ou un polypeptide optiquement ou biologiquement actif ; un groupement chiral.

Le choix du substituant Z permet d'ajuster les propriétés d'un composé de l'invention.

Une famille particulière de composés de l'invention est celle dans laquelle Z représente un groupe $R_DY$-. Les composés dans lesquels Y est $-SO_2-$ sont spécialement préférés.

Dans un mode de réalisation, $R_D$ est choisi parmi les radicaux alkyle, alkényle, oxa-alkyle, oxa-alkényle, aza-alkyle, aza-alkényle, thia-alkyle ou thia-alkényle ayant de 1 à 24 atomes de carbone, ou parmi les radicaux aryle, arylalkyle, alkylaryle ou alkénylaryle ayant de 5 à 24 atomes de carbones.

Dans un autre mode de réalisation, $R_D$ est choisi parmi les radicaux alkyles ou alkényles ayant de 1 à 12 atomes de carbone et comprenant éventuellement au moins un hétéroatome 0, N ou S dans la chaîne principale ou dans une chaîne latérale, et/ou portant éventuellement un groupe hydroxy, un groupe carbonyle, un groupe amine ou un groupe carboxyle.

Un substituant $R_D$ peut être un radical polymère, par exemple un radical oligo(oxyalkylène). Le composé de l'invention se présente alors sous la forme d'un polymère portant un groupe ionique $-[Y-N-SO_x-R_F]^-$, $M^+$.

$R_D$ peut être une unité récurrente d'un polymère, par exemple une unité oxyalkylène ou une unité styrène. Le composé de l'invention se présente alors sous la forme d'un polymère dans lequel une partie au moins des unités récurrentes portent un groupe latéral sur lequel est fixé un groupe ionique $-[Y-N-SO_x-R_F]^-$, $M^+$. A titre d'exemple, on peut citer un poly(oxyalkylène) dans lequel au moins certaines unités oxyalkylène portent un substituant $-[Y-N-SO_x-R_F]^-$, $M^+$, ou un polystyrène dans lequel au moins certaines unités styrène portent un substituant $-[Y-N-SO_x-R_F]^-$, $M^+$ par exemple $[styrényl-Y-N-S(O)_x-R_F]^-$.

Une catégorie particulière de composés selon l'invention comprend les composés dans lesquels le substituant $R_D$ possède au moins un groupement ionophore anionique et/ou au moins un groupement ionophore cationique. Le groupement anionique peut par exemple être une fonction carboxylate ($-CO_2^-$), une fonction sulfonate ($-SO_3^-$), une fonction sulfonimide ($-SO_2NSO_2^-$) ou une fonction sulfonamide ($-SO_2N-$). Le groupement ionophore cationique peut par exemple être un groupement iodonium, sulfonium, oxonium, ammonium, amidinium, guanidinium, pyridinium, imidazolium, imidazolinium, triazolium, phosphonium ou carbonium. Le groupement ionophore cationique peut jouer totalement ou partiellement le rôle du cation M.

Lorsque $R_D$ comporte au moins une insaturation éthylénique et/ou un groupe condensable et/ou un groupe dissociable par voie thermique, par voie photochimique ou par dissociation ionique, les composés de l'invention sont des composés réactifs qui peuvent être soumis à des polymérisations, des réticulations ou des condensations, éventuellement avec d'autres monomères. Ils peuvent également être utilisés pour fixer des groupes ionophores sur les polymères portant la fonction réactive appropriée.

Un substituant $R_D$ peut être un groupe mésomorphe ou un groupe chromophore ou un polymère conducteur électronique autodopé ou un alcoxysilane hydrolysable.

Un substituant $R_D$ peut aussi comporter un dipôle dissociant, par exemple une fonction amide, une fonction sulfonamide ou une fonction nitrile.

Un substituant $R_D$ peut aussi comporter un couple rédox, par exemple un groupe disulfure, un groupe thioamide, un groupe ferrocène, un groupe phénothiazine, un groupe bis(dialkylaminoaryle), un groupe nitroxyde ou un groupe imide aromatique.

Un substituant $R_D$ peut aussi comporter un ligand complexant, ou un groupe optiquement actif.

Une autre catégorie de composés de l'invention comprend les composés dans lesquels $R_D$-Y- représente un acide aminé, ou un polypeptide optiquement ou biologiquement actif.

Selon une autre variante, un composé selon l'invention comprend un substituant $R_D$ qui représente un radical ayant une valence v supérieure à deux, comportant lui-même au moins un groupe $R_F-S(O)_x-N-Y-$. Dans ce cas, les charges négatives présentes sur la partie anionique du composé de l'invention devront être compensées par le nombre approprié de cations ou de groupes ionophores cationiques M.

Lorsqu'un composé de la présente invention répond à la formule $R_F-S(O)_x-N-Z$, dans laquelle Z est un groupement électroattracteur non lié à l'azote portant la charge négative par un groupe Y, Z est avantageusement choisi dans le groupe constitué par -CN, $-OC_nF_{2n+1}$, $-OC_2F_4H$, $-SC_nF_{2n+1}$ et $-SC_2F_4H$, $-O-CF=CF_2$, $-SCF=CF_2$, n étant un nombre entier de 1 à 8. Z peut également être un radical $C_nF_{2n+1}CH_2-$, n étant un nombre entier de 1 à 8, ou parmi les hétérocycles, en particulier ceux dérivés de la pyridine, de la pyrazine, de la pyrimidine, de l'oxadiazole, du thiadiazole, fluorés ou non. Z peut aussi représenter une unité récurrente d'un polymère. Le composé de l'invention se présente alors sous la forme d'un polymère dans lequel au moins une partie des unités récurrentes portent un groupe

latéral sur lequel est fixé un groupe ionique -[(N-$SO_x$-$R_F$)$^-$, $M^+$]. A titre d'exemple, on peut citer un polymère comprenant l'une des unités récurrentes suivantes :

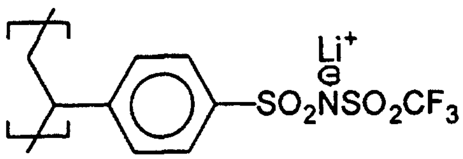

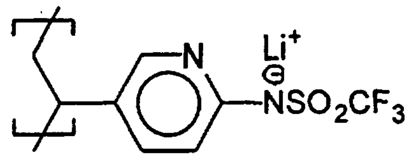

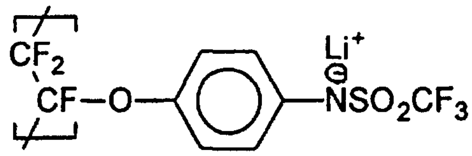

ou bien un polyzwitterion de polymère conducteur autodopé polyaniline dont l'unité récurrente est :

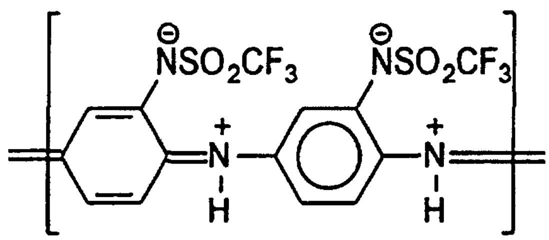

Les composés de la présente invention peuvent être obtenus par un procédé dans lequel on fait réagir un composé $R_FSO_x$-L avec un composé $[A\text{-}N\text{-}Z]^{n-}{}_m$ $nM^{m+}$, $R_F$, x, M et Z étant tels que définis précédemment, L représentant un groupe partant électronégatif tel qu'un halogène, un radical N-imidazoyle, un radical N-triazoyle, un radical $R_FSO_{x+1}$- et A représentant un cation $M^{m+}$, un groupe trialkylsilyle, un groupe trialkyle germanyle, un groupe trialkylstannyle ou un groupe tertioalkyle, dans lesquels les substituants alkyles ont de 1 à 6 atomes de carbone. A titre d'exemple, on peut citer la réaction d'un fluorure de fluorosulfonyle avec un di sel de cyanamide selon le schéma réactionnel suivant :

$$FSO_2\text{-}F + [NaNCN]^- Na^+ \Rightarrow NaF + [FSO2\text{-}NCN]^- Na^+.$$

On peut également citer la réaction d'une aniline substituée avec l'anhydride trifluorométhanesulfonique.

Les composés dans lesquels Z représente $R_DY$- peuvent être obtenus par un procédé dans lequel on fait réagir un composé $R_D$-Y-L avec un composé $[R_FSO_X\text{-}N\text{-}A]^{n-}{}_m$ $nM^{m+}$. A titre d'exemple d'un tel procédé, on peut citer la réaction d'une perfluorosulfonamide ou de l'un de ses sels avec un halogénure de sulfonyle.

L'utilisation d'un composé $[A\text{-}N\text{-}Z]^{n-}{}_m$ $nM^{m+}$ dans lequel A est un groupement tertioalkyle est avantageuse, car un tel groupement est précurseur de proton par formation de l'alcène correspondant. L'utilisation du groupe trialkylsilyle est spécialement intéressante lorsque le groupe partant est un atome de fluor, en raison de la très grande stabilité de la liaison F-Si.

Lorsque l'on utilise un composé $[A\text{-}N\text{-}Z]^{n-}{}_m$ $nM^{m+}$ dans lequel A est le proton, il est avantageux d'effectuer la réaction en présence d'une base tertiaire ou d'une base encombrée T susceptible de former le sel $L^-(HT^+)$ par combinaison avec le proton, afin de favoriser la formation du composé de l'invention. La base peut être choisie parmi les alkylamines (par exemple la triéthylamine, la di-isopropylamine, la quinuclidine), le 1,4-diazobicyclo[2,2,2]octane (DABCO) ; les pyridines (par exemple la pyridine, les alkylpryidines, les dialkylaminopyridines) ; les imidazoles (par exemple les N-alkylimidazoles, l'imidazo[1,1-a]pyridine); les amidines (par exemple le 1,5-diazabicyclo[4,3,0]non-5-ène (DBN), le 1,8-diazabicyclo[5,4,0]undec-7-ène (DBU)) ; les guanidines (par exemple la tétraméthyl guanidine, la 1,3,4,7,8-hexahydro-1-méthyl-2H-pyrimido[1,2-a]pyrimidine (HPP).

A titre d'exemple d'un tel procédé, on peut citer le procédé dans lequel on fait réagir un chlorure de sulfonyle $R_DSO_2Cl$ avec une perfluorosulfonamide en présence de DABCO.

Un composé selon l'invention peut également être obtenu en faisant réagir l'acide perfluorosulfonique ou l'un de ses sels avec un composé $(R^i)_3P{=}N{-}Z$ dans lequel les $R^i$ représentent indépendamment les uns des autres un radical alkyle, un radical aryle ou un radical dialkylamino. De la même manière, on peut faire réagir un acide $R_DSO_x$-OH ou l'un de ses sels avec un composé $(R^i)_3P{=}N{-}SO_xR_F$. A titre d'exemple, on peut citer la réaction d'un alkylsulfonate de sodium avec $R_FSO_2N{=}P(C_6H_5)_3$.

Le cation d'un composé obtenu selon l'un ou l'autre des procédés décrits ci-dessus peut être remplacé par les procédés classiques d'échange de cation, soit par des précipitations ou des extractions sélectives, soit par l'utilisation de résines échangeuses d'ions.

En outre, le substituant $R_D$ d'un composé de l'invention peut être modifié par les réactions connues. Par exemple, un substituant $R_D$ qui comprend un groupe allyle peut être transformé par réaction avec un peroxyde pour obtenir un substituant $R_D$ époxydé. Un groupe -NHR peut être transformé en groupe vinylester par réaction avec une base forte telle que le tert-butoxyde de potassium, puis avec le chloroformate de vinyle. Les procédés pour réaliser ces modifications et d'autres sont à la portée de l'homme de métier. Bien entendu, les fonctions portées par les radicaux $R_A$ et Z qui pourraient interférer avec les réactions permettant la préparation des composés de l'invention peuvent être protégées temporairement par des techniques connues. Par exemple, une fonction amine peut être protégée par un groupement t-BOC (tertiobutoxycarbonyl), stable en présence des bases T mais facilement éliminé par traitement en milieu acide.

Les composés ioniques de la présente invention comprennent au moins un groupement ionophore sur lequel sont fixés des substituants qui peuvent être très variés. Compte tenu du grand choix possible pour les substituants, les composés de l'invention permettent d'induire des propriétés de conduction ionique dans la plupart des milieux organiques, liquides ou polymères possédant une polarité, même faible. Les applications sont importantes dans le domaine de l'électrochimie, en particulier du stockage de l'énergie dans des générateurs primaires ou secondaires, dans les supercapacités, dans les piles à combustibles et dans les diodes électroluminescentes. La compatibilité des composés ioniques de l'invention avec les polymères ou les liquides organiques permet d'induire des propriétés antistatiques marquées, même lorsque la teneur en composé ionique est extrêmement faible. Les composés de l'invention qui sont des polymères, de même que des composés polymères obtenus à partir de composés de l'invention ayant la propriété de se polymériser ou de se copolymériser, présentent les propriétés énumérées ci-dessus avec l'avantage d'avoir une charge anionique immobile. C'est pourquoi un autre objet de la présente invention est constitué par un matériau à conduction ionique constitué par un composé ionique de la présente invention en solution dans un solvant.

Dans un mode de réalisation, le composé ionique utilisé pour l'élaboration d'un matériau à conduction ionique est choisi parmi les composés dont le cation est l'ammonium, ou un cation dérivé d'un métal, en particulier le lithium ou le potassium, le zinc, le calcium, les métaux des terres rares, ou un cation organique, tel qu'un ammonium substitué, un imidazolium, un triazolium, un pyridinium, un 4-diméthylamino-pyridinium, lesdits cations portant éventuellement un substituant sur les atomes de carbone du cycle. Le matériau à conduction ionique ainsi obtenu présente une conductivité et une solubilité dans les solvants élevées, du fait des interactions faibles entre la charge positive et la charge négative. Son domaine de stabilité électrochimique est étendu, et il est stable dans des milieux aussi bien réducteurs qu'oxydants. De plus, les composés qui ont un cation organique et un point de fusion inférieur à 150°C, en particulier les composés de imidazolium, de triazolium, de pyridinium, de 4-diméthylamino-pyridinium présentent une conductivité élevée intrinsèque, même en l'absence de solvant, lorsqu'ils sont en phase fondue.

Les matériaux à conduction ionique qui incorporent un composé de l'invention dans lequel $R_F$ est un radical alkyle perhalogéné ayant de 1 à 12 atomes de carbone, ou un radical alkylaryle perhalogéné ayant de 6 à 9 atomes de carbone sont intéressants dans la mesure où les faibles interactions entre les atomes de fluor de la chaîne entraînent des solubilités et une conductivité élevées, même dans le cas où le reste de la molécule contient des groupements ayant tendance à donner de fortes interactions tels les radicaux aromatiques conjugués ou les zwitterions.

Le choix d'un composé de l'invention dans lequel $R_F$ est choisi parmi les radicaux $R_ACF_2$-, $R_ACF_2CF_2$-, $R_ACF_2CF(CF_3)$- ou $CF_3C(R_A)F$- permet d'adapter de manière très précise les propriétés du matériau à conduction ionique en choisissant de manière approprié le substituant $R_A$. En particulier, il permettent de bénéficier, avec un nombre réduit d'atomes de fluor, des propriétés de dissociation et de solubilité propres aux charges anioniques des systèmes perfluorés. Ces groupements sont aisément accessibles à partir de produits industriels comme le tétrafluoroéthylène ou le tétrafluoropropylène. La quantité réduite de fluor rend ces composé moins sensibles à la réduction par les métaux très électropositifs comme l'aluminium, le magnésium ou surtout le lithium.

Les propriétés du matériau à conduction ionique peuvent également être adaptées par le choix du substituant $R_D$.

Le choix pour $R_A$ ou $R_D$ d'un groupe alkyle, d'un groupe aryle, d'un groupe alkylaryle ou d'un groupe arylalkyle, permet d'induire dans le matériau à conduction ionique des propriétés de type mésogène, en particulier les groupements alkyles de 6 à 20 atomes de carbones, les groupements arylealkyle, en particulier ceux contenant l'entité biphényle qui forment des phases de type cristal liquide. Des propriétés de conduction dans des phases de type cristal liquide, nématique, cholestérique ou discotique, sont intéressantes pour les applications relatives à l'affichages optique ou pour réduire la mobilité des anions dans les électrolytes, en particulier dans les électrolytes polymères, sans affecter la mobilité des cations. Cette particularité est importante pour les applications dans les générateurs électrochimiques, en particulier ceux mettant en jeu les cations lithium.

Lorsque le substituant $R_A$ est un groupe mésomorphe ou un groupe comprenant au moins une insaturation éthylénique et/ou un groupe condensable et/ou un groupe dissociable par voie thermique, par voie photochimique ou par dissociation ionique, ou lorsque $R_D$ est un substituant contenant l'un de ces groupes, le matériau à conduction ionique forme aisément des polymères ou copolymères qui sont des polyélectrolytes, soit intrinsèques quand le polymère porte des groupements solvatants, soit par addition d'un solvant polaire de type liquide ou polymère, ou par mélange avec un tel solvant. Ces produits ont une conductivité uniquement due au cations, ce qui constitue une propriété très utile dans les applications de type générateur électrochimique. En faible fraction molaire dans un copolymère, ils induisent des propriétés antistatiques stables et peu dépendantes de l'humidité et favorisent la fixation de colorants cationiques, cette propriété étant utile pour les fibres textiles et les lasers à colorants.

La présence d'un substituant $R_A$ ou $R_D$ qui est un polymère conducteur électronique autodopé, améliore la stabilité du matériau à conduction ionique par rapport aux agents extérieurs. La conductivité est stable dans le temps même à des températures élevées. En contact avec les métaux, ces matériaux donnent des résistances d'interface très faibles et protègent en particulier les métaux ferreux ou l'aluminium de la corrosion.

Lorsque le substituant $R_A$ ou $R_D$ est un alcoxysilane hydrolysable, le matériau à conduction ionique peut former des des polymères stables par simple mécanisme d'hydrolyse-condensation en présence d'eau, permettant ainsi de traiter les surfaces d'oxydes, de silice, de silicates, en particulier le verre, pour induire des propriétés de conduction de surface, des propriétés antistatiques, ou pour favoriser l'adhésion de polymères polaires.

Lorsque le substituant $R_A$ ou $R_D$ comprend un dipôle dissociant tel qu'une amide, une sulfonamide ou un nitrile, le matériau à conduction ionique a une conductivité améliorée dans des milieux de faible et moyenne polarité, en particulier dans les polymères solvatants, ce qui permet de minimiser, voire de supprimer l'addition de solvants ou de plastifiants volatils.

La présence d'un substituant $R_A$ ou $R_D$ qui contient un couple rédox tel qu'un disulfure, une thioamide, un ferrocène, une phéno-thiazine, un groupe bis(dialkylaminoaryle), un nitroxyde, un imide aromatique, permet d'induire dans le matériau à conduction ionique des propriétés de navette rédox utiles comme élément de protection et d'égalisation de charge des générateurs électrochimiques, dans les systèmes photoélectrochimiques, en particulier de conversion de la lumière en électricité, dans les systèmes de modulation de la lumière de type électrochrome.

La présence d'un substituant $R_A$ ou $R_D$ qui est un ligand complexant dans un matériau à conduction ionique permet de chélater les cations métalliques, en particulier ceux qui possèdent un charge élevée (2, 3 et 4), sous forme de complexe soluble dans les milieux organiques, y compris dans les milieux aprotiques, et permet le transport de ces cations en particulier sous forme de complexe anionique, dans les polymères solvatants. Les cations métalliques de charge élevée sont en effet immobiles dans les polymères solvatants. Ce type de complexant donne avec certains cations des métaux de transition (Fe, Co) ou de certaines terres rares (Ce, Eu...) des couples rédox particulièrement stables.

Les matériaux à conduction ionique contenant un composé de l'invention dans lequel $R_D$ est un substituant alkyle ou alkényle qui contient au moins un hétéroatome choisi parmi 0, N et S ont un pouvoir complexant, et plastifiant, en particulier dans les polymères polaires et tout spécialement les polyéthers. Les hétéroatomes N et S sont sélectivement complexants pour les cations des métaux de transition, Zn et Pb.

Lorsqu'un substituant $R_D$ alkyle ou alkényle porte en outre un groupe hydroxy, un groupe carbonyle, un groupe amine, un groupe carboxyle, un groupe isocyanate ou un groupe thioisocyanate, le composé ionique de l'invention peut donner par polycondensation un polymère ou un copolymère et le matériau à conduction ionique qui contient un tel polymère ou copolymère présente des propriétes de polyélectrolyte.

La présence, dans le matériau à conduction ionique de l'invention, d'un composé dans lequel $R_D$ est choisi parmi les radicaux aryle, arylalkyle, alkylaryle ou alkénylaryle, dans lesquels les chaînes latérales et/ou les noyaux aromatiques comprennent des hétéroatomes tels que l'azote, l'oxygène, le soufre, améliore la dissociation et augmente la possibilité de former des complexes suivant la position de l'hétéroatome (pyridine) ou de donner par oxydation duplicative des polymères ou copolymères conjugués (pyrrole, thiophène).

Lorsque le matériau à conduction ionique contient un composé de l'invention dans lequel $R_D$ représente une unité récurrente d'une chaîne polymère, le matériau constitue un polyélectrolyte.

Un composé de l'invention dans lequel le substituant Z est choisi dans le groupe constitué par $-OC_nF_{2n+1}$, $-OC_2F_4H$, $-SC_nF_{2n+1}$ et $-SC_2F_4H$, $-OCF=CF_2$, $-SCF=CF_2$, n étant un nombre entier de 1 à 8, est un précurseur de monomères et polymères stables, en particulier vis-à-vis de l'oxygène même à des températures supérieures à 80°C lorsqu'il s'agit des polymères. Un matériau à conduction ionique qui contient un tel composé est donc particulièrement approprié comme électrolyte d'une pile à combustible.

Le solvant d'un matériau à conduction ionique de la présente invention peut être un solvant liquide aprotique, un polymère polaire ou un de leurs mélanges.

Le solvant liquide aprotique est choisi par exemple parmi les éthers linéaires et les éthers cycliques, les esters, les nitriles, les dérivés nitrés, les amides, les sulfones, les sulfolanes, les alkylsulfamides et les hydrocarbures partiellement halogénés. Les solvants particulièrement préférés sont le diéthyléther, le diméthoxyéthane, le glyme, le tétrahydrofurane, le dioxane, le diméthyltétrahydrofurane, le formiate de méthyle ou d'éthyle, le carbonate de propylène ou d'éthylène, les carbonates d'alkyles (notamment le carbonate de diméthyle, le carbonate de diéthyle et le carbonate de méthylpropyle), les butyrolactones, l'acétonitrile, le benzonitrile, le nitrométhane, le nitrobenzène, la diméthylformamide, la diéthylformamide, la N-méthylpyrrolidone, la diméthylsulfone, la tétraméthylène sulfone et les tétraalkylsulfonamides ayant de 5 à 10 atomes de carbone.

Le polymère polaire peut être choisi parmi les polymères solvatants, réticulés ou non, portant ou non des groupes ioniques greffés. Un polymère solvatant est un polymère qui comporte des unités solvatantes contenant au moins un hétéroatome choisi parmi le soufre, l'oxygène, l'azote et le fluor. A titre d'exemple de polymères solvatants, on peut citer les polyéthers de structure linéaire, peigne ou à blocs, formant ou non un réseau, à base de poly(oxyde d'éthylène), ou les copolymères contenant le motif -oxyde d'éthylène ou oxyde de propylène ou allylglycidyléther, les polyphosphazènes, les réseaux réticulés à base de polyéthylène glycol réticulé par des isocyanates ou les réseaux obtenus par polycondensation et portant des groupements qui permettent l'incorporation de groupements réticulables. On peut également citer les copolymères à blocs dans lesquels certains blocs portent des fonctions qui ont des propriétés rédox. Bien entendu, la liste ci-dessus n'est pas limitative, et tous les polymères présentant des propriétés solvatantes peuvent être utilisés.

Un matériau à conduction ionique de la présente invention peut comprendre simultanément un solvant liquide aprotique choisi parmi les solvants liquides aprotiques cités ci-dessus et un solvant polymère polaire comprenant des unités contenant au moins un hétéroatome choisi parmi le soufre, l'azote, l'oxygène et le fluor. Il peut comprendre de 2 à 98% de solvant liquide. A titre d'exemple d'un tel polymère polaire, on peut citer les polymères qui contiennent principalement de unités dérivées de l'acrylonitrile, du fluorure de vinylidène, de la N-vinylpyrrolidone ou du méthacrylate de méthyle. La proportion de liquide aprotique dans le solvant peut varier de 2% (correspondant à un solvant plastifié) à 98% (correspondant à un solvant gélifié).

Un matériau à conduction ionique de la présente invention peut contenir en outre un sel utilisé classiquement dans l'art antérieur pour l'élaboration d'un matériau à conduction ionique. Parmi les sels utilisables en mélange avec un composé ionique selon l'invention, on préfère tout particulièrement un sel choisi parmi les perfluoroalcanesulfonates, les bis(perfluoroalkylsulfonyl) imidures, les bis(perfluoroalkylsulfonyl) méthanes et les tris(perfluoroalkylsulfonyl)méthanes.

Bien entendu, un matériau à conduction ionique de l'invention peut contenir en outre les additifs utilisés de manière classique dans ce type de matériau, et notamment des charges minérales ou organiques sous forme de poudre ou de fibres.

Un matériau à conduction ionique de l'invention peut être utilisé comme électrolyte dans un générateur électrochimique. La présente invention a ainsi pour autre objet un générateur électrochimique comprenant une électrode négative et une électrode positive séparées par un électrolyte, caractérisé en ce que l'électrolyte est un matériau à conduction ionique tel que défini ci-dessus. Selon un mode de réalisation particulier, un tel générateur comprend une électrode négative constituée par du lithium métallique, ou par l'un de ses alliages, éventuellement sous forme de dispersion nanométrique dans de l'oxyde de lithium, ou par un nitrure double de lithium et d'un métal de transition, ou par un oxyde à bas potentiel ayant pour formule générale $Li_{1+y+x/3}Ti_{2-x/3}O_4$ ($0 \leq x \leq 1$, $0 \leq y \leq 1$), ou par le carbone et les produit carbonés issus de la pyrolyse de matières organiques. Selon un autre mode de réalisation, le générateur comprend une électrode positive choisie parmi les oxydes de vanadium $VO_x$ ($2 \leq x \leq 2{,}5$), $LiV_3O_8$, $Li_yNi_{1-x}Co_xO_2$, ($0 \leq x \leq 1$ ; $0 \leq y \leq 1$), les spinelles de manganèse $Li_yMn_{1-x}M_xO_2$ (M = Cr, Al, V, Ni, $0 \leq x \leq 0{,}5$ ; $0 \leq y \leq 2$), les polydisulfures organiques, FeS, $FeS_2$, le sulfate de fer $Fe_2(SO_4)_3$, les phosphates et phosphosilicates de fer et de lithium de structure olivine, ou leurs produits de substitution du fer par le manganèse, utilisés seuls ou en mélanges. Le collecteur de l'électrode positive est de préférence en aluminium.

Un matériau à conduction ionique de la présente invention peut également être utilisé dans une supercapacité. Un autre objet de la présente invention est par conséquent une supercapacité utilisant au moins une électrode de carbone à haute surface spécifique, ou une électrode contenant un polymère rédox, dans laquelle l'électrolyte est'un matériau à conduction ionique tel que défini ci-dessus.

Un matériau à conduction ionique de la présente invention peut également être utilisé pour le dopage p ou n d'un polymère à conduction électronique et cette utilisation constitue un autre objet de la présente invention.

En outre, un matériau à conduction ionique de la présente invention peut être utilisé comme électrolyte dans un dispositif électrochrome. Un dispositif électrochrome dans lequel l'électrolyte est un matériau à conduction ionique selon l'invention est un autre objet de la présente invention.

Les inventeurs ont également trouvé que la charge anionique portée par le groupement $R_F$-$SO_x$-$N^-Z$ exerçait

un effet stabilisant sur les conducteurs électroniques de type polymères conjugués, et que l'utilisation d'un composé dans lequel le substituant Z comprenait une longue chaîne alkyle permettait de rendre ces polymères solubles dans les solvants organiques usuels même à l'état dopé. Le greffage de ces charges sur le polymère lui-même donne des polymères dont la charge globale est cationique, solubles dans les solvants organiques et présentant, outre leur stabilité, des propriétés d'anticorrosion vis-à-vis des métaux, l'aluminium et les métaux ferreux. La présente invention a aussi pour objet des matériaux à conduction électronique comprenant un composé ionique de la présente invention dans lequel la partie cationique est un polycation constitué par un polymère conjugué dopé "p". Les composés ioniques préférés pour cette application sont ceux dans lesquels le substituant Z contient au moins une chaîne alkyle ayant de 6 à 20 atomes de carbone. A titre d'exemple, on peut citer les composés dans lesquels Z est $R_DY$-, $R_D$ étant un radical alkyle. On peut également citer les composés dans lesquels $R_F$ est $R_ACF_2$-, $R_ACF_2CF_2$-, $R_ACF_2CF(CF_3)$- ou $CF_3C(R_A)F$- dans lesquelles $R_A$- représente un radical alkyle. On peut citer en outre les composés dans lesquels Z représente un noyau aromatique portant un radical alkyle.

Les colorants de type cationique (cyanines) sont utilisés de plus en plus fréquemment comme sensibilisateurs de films photographiques, pour le stockage optique d'information (disques optiques accessibles en écriture), pour les lasers. La tendance de ces molécules conjuguées à s'empiler lorsqu'elles sont en phases solides limite leur utilisation, par suite des variations des propriétés optiques par rapport à la molécule isolée. L'utilisation de composés ioniques de l'invention pour la fabrication de colorants cationiques dont les contre ions, éventuellement fixés à cette même molécule, correspondent aux fonctionnalités de l'invention, permet de réduire les phénomènes d'agrégation, y compris dans des matrices solides polymères et de stabiliser ces colorants. La présente invention a pour autre objet une composition de colorant cationique, caractérisée en ce qu'elle contient un composé ionique selon l'invention. Les composés ioniques particulièrement préférés pour cette application sont ceux dans lesquels la ou les charges négatives du groupement anionique $R_F$-$SO_x$-$N^-$-Z sont soit fixées à la molécule de colorant, soit elles constituent le contre-ion des charges positives du colorant. A titre d'exemple de tels composés, on peut citer les composés suivants :

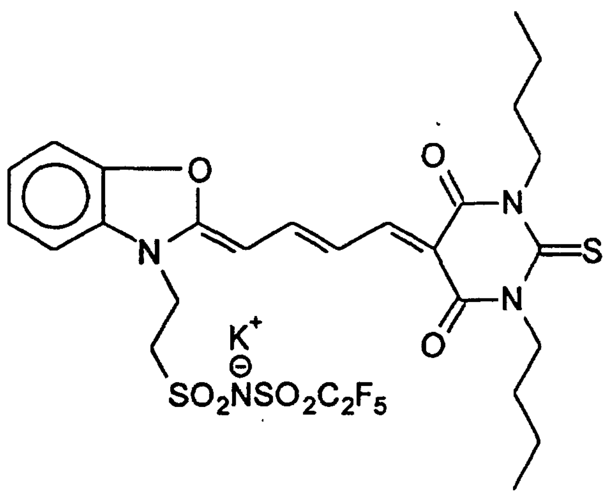

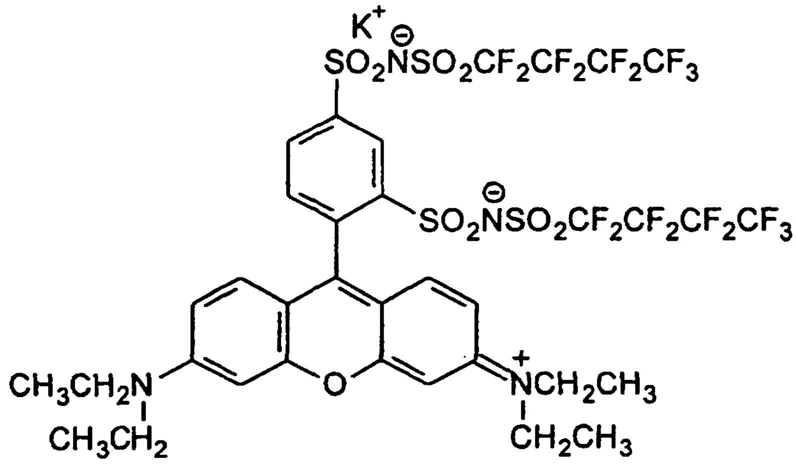

La présente invention est illustrée par les exemples suivants, auxquels elle n'est toutefois pas limitée.

Les exemples 1 à 7 décrivent la préparation de quelques composés utilisés comme réactifs pour la synthèse des composés ioniques de la présente invention. Les exemples 8 à 78 illustrent la préparation de composés selon l'invention et leurs utilisations.

**Exemple 1: Trifluorométhanesulfonamide**

A une suspension sous forte agitation de 140,53 g (1,8 moles) de carbamate d'ammonium $H_2NCO_2NH_4$ (commercialisé par Aldrich) dans 1 l de dichlorométhane à 0°C, on a ajouté goutte à goutte pendant 2 heures 282,13 g (1 mole) d'anhydride trifluorométhanesulfonique $(CF_3SO_2)_2O$ (commercialisé par Aldrich) dilués dans 250 ml de dichlorométhane. Il s'est produit un dégagement de gaz carbonique conformément à la réaction ci-dessous:

$$(CF_3SO_2)_2O + 1{,}5\ H_2NCO_2NH_4 \rightarrow CF_3SO_3^-NH_4^+ + CF_3SO_2NH^-NH_4^+ + 1{,}5\ CO_2$$

Après 3 heures à 0°C, la réaction s'est poursuivie pendant 24 heures à température ambiante, puis le dichlorométhane a été évaporé et le produit repris dans 400 ml d'eau. L'addition de 250 ml d'une solution 4 M d'acide chlorhydrique a permis de libérer la trifluorométhanesulfonamide $CF_3SO_2NH_2$ qui a été extraite par trois fractions de 200 ml d'éther. Après séchage par du sulfate de magnésium de la phase éthérée (600 ml), le produit a été récupéré après évaporation de l'éther et purifié par sublimation sous vide secondaire à 60°C. On a obtenu 137,16 g de la trifluorométhanesulfonamide $CF_3SO_2NH_2$ (92% de rendement) ayant une pureté caractérisée par RMN du proton et du fluor supérieure à 99%.

Le sel de sodium correspondant a été préparé en faisant réagir la trifluorométhanesulfonamide avec le carbonate de sodium $Na_2CO_3$ dans l'eau (20% en excès). Après évaporation de l'eau et séchage, le produit obtenu a été repris dans l'acétonitrile puis l'excès de carbonate a été éliminé par filtration. Après évaporation de l'acétonitrile et séchage, on a obtenu quantitativement le sel de sodium de la trifluorométhanesulfonamide $CF_3SO_2NHNa$.

La microanalyse a donné: H 0,52 (0,59) ; C 7,22 (7,02) ; N 8,41 (8,19) ; F 33,82 (33,32); Na 13,21 (13,44) ; S 18,65 (18,74).

Les sels de lithium $CF_3SO_2NHLi$ et de potassium $CF_3SO_2NHK$ ont été obtenus par un procédé similaire, en remplaçant le carbonate de sodium respectivement par le carbonate de lithium et de potassium.

**Exemple 2 : Fluorosulfonamide**

Le composé a été préparé dans des conditions similaires à celles décrites dans l'exemple 1, en remplaçant la trifluorométhanesulfonamide $CF_3SO_2NH_2$ par 182,11 g (1 mole) d'anhydride fluorosulfonique $(FSO_2)_2O$ (commercialisé par SST Corporation) purifiée au préalable par distillation sous vide. On a obtenu 80,25 g de la fluorosulfonamide $FSO_2NH_2$ (81% de rendement) ayant une pureté caractérisée par RMN du proton et du fluor supérieure à 99%. Le sel de sodium correspondant a été préparé en dosant une solution aqueuse à 0°C de fluorosulfonamide $FSO_2NH_2$ avec une solution titrée d'hydroxyde de sodium jusqu'au point de neutralisation déterminé par pH-métrie. Après lyophilisation et séchage sous vide pendant 24 heures, on a récupéré quantitativement le sel de sodium de la fluorosulfonamide

$FSO_2NHNa$.

La microanalyse a donné: H 0,78 (0,83) ; N 11,32 (11,57) ; F 15,12 (15,69) ; Na 18,66 (18,99) ; S 26,01 (26,48).

Les sels de lithium $FSO_2NHLi$ et de potassium $FSO_2NHK$ ont été obtenus par un procédé similaire, en remplaçant l'hydroxyde de sodium respectivement par l'hydroxyde de lithium et l'hydroxyde de potassium.

**Exemple 3 : Chlorure de pentafluoroéthanesulfonyle**

Dans 600 ml d'éther refroidi à -78°C sous argon, on a condensé 60 g (244 mmoles) de l'iodure de pentafluoroéthyle $C_2F_5I$ (commercialisé par Strem Chemicals). Sous agitation, on a alors ajouté 153 ml d'une solution 1,6 M de méthyllithium dans l'éther (244 mmoles, commercialisé par Fluka). Après 5 minutes, on a introduit ≈ 20 g (≈ 312 mmoles) de dioxyde de soufre $SO_2$ dans la solution, la réaction s'est poursuivie pendant 2 heures à -78°C. Ensuite, on a laissé remonter la solution à température ambiante durant 2 heures, puis après une heure à l'ambiante les solvants ont été évaporés. On a récupéré après séchage 44,51 g de pentafluoroéthanesulfinate de lithium $C_2F_5SO_2Li$ (96% de rendement).

On a ensuite fait passer un courant de chlore $Cl_2$ dans 200 ml d'eau contenant 28,5 g (150 mmoles) de pentafluoroéthanesulfinate de lithium $C_2F_5SO_2Li$. Il est apparu rapidement une deuxième phase plus dense que l'eau qui a été extraite par deux fractions de 25 ml de dichlorométhane anhydre. Après séchage de la phase organique par du sulfate de magnésium, on a récupéré par distillation fractionnée 26,55 g de chlorure de pentafluoroéthanesulfonyle $C_2F_5SO_2Cl$ (81% de rendement) ayant une pureté caractérisée par RMN du fluor supérieure à 99%.

**Exemple 4 : Perfluorobutanesulfonamide**

A 30,21 g (100 mmoles) de fluorure de perfluoro-1-butanesulfonyle $C_4F_9SO_2F$ (commercialisé par Aldrich) et 8,91 g (100 mmoles) de carbamate d'éthyle $C_2H_5O_2CNH_2$ dans 100 ml de tétrahydrofurane anhydre à 0°C, on a ajouté par portions 1,75 g (220 mmoles) d'hydrure de lithium LiH à 95% (commercialisé par Aldrich). Après une agitation de 72 heures sous argon, le milieu réactionnel a été centrifugé et filtré pour éliminer le précipité de fluorure de lithium LiF et l'excès d'hydrure de lithium. Le solvant a ensuite été évaporé et le produit obtenu repris dans 100 ml d'eau. Après addition de 3,5 g (200 mmoles) d'hydroxyde de lithium monohydrate, le milieu réactionnel a été porté au reflux pendant une nuit pour hydrolyser la fonction ester. Après refroidissement, le milieu réactionnel a été porté à un pH ≈ 1 par addition d'une solution d'acide chlorhydrique 10 M afin d'éliminer la fonction carboxylique présente, puis extrait par trois fractions de 50 ml d'éther après 24 heures sous agitation. Après séchage de la phase organique par du sulfate de magnésium et évaporation, on a récupéré après séchage sous vide 27,2 g de perfluoro-1-butanesulfonamide $C_4F_9SO_2NH_2$ (91% de rendement) ayant une pureté caractérisée par RMN du proton et du fluor supérieure à 99%.

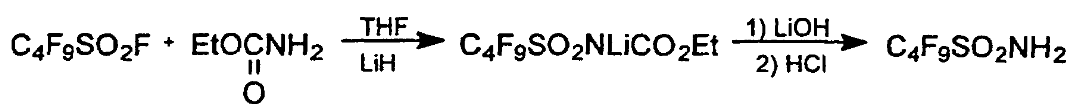


Le sel de sodium correspondant a été préparé en faisant réagir la perfluoro-1-butanesulfonamide avec le carbonate de sodium $Na_2CO_3$ dans l'eau (20% en excès). Après évaporation de l'eau et séchage, le produit obtenu a été repris dans le tétrahydrofurane puis l'excès de carbonate éliminé par filtration. Après évaporation du tétrahydrofurane et séchage, on a obtenu quantitativement le sel de sodium de la perfluoro-1-butanesulfonamide $C_4F_9SO_2NHNa$.

La microanalyse a donné: H 0,25 (0,31) ; C 15,35 (14,96) ; N 4,63 (4,36) ; F 54,1 (53,25) ; Na 7,36 (7,16) ; S 10,35 (9,98).

Les sels de lithium et de potassium ont été obtenus par un procédé similaire, en remplaçant le carbonate de lithium respectivement par le carbonate de sodium et le carbonate de potassium.

**Exemple 5 : Pentafluoroéthanesulfonamide**

On a ajouté lentement à 50 ml d'une solution 1 M de bis(triméthylsilyl)amidure de sodium $((CH_3)_3Si)_2NNa$ dans le tétrahydrofurane (50 mmoles, commercialisé par Aldrich) à -20°C, 10,93 g de chlorure de pentafluoroéthanesulfonyle $C_2F_5SO_2Cl$, préparé comme dans l'exemple 3.

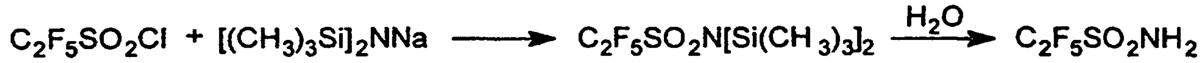

Après deux heures à -20°C, le solvant a été évaporé et le produit repris dans 50 ml d'eau, le pH amené à ≈ 2 puis la phase aqueuse extraite par deux fractions de 20 ml d'éther. Après séchage de la phase organique par le sulfate de magnésium, évaporation de l'éther et séchage, le produit recueilli a été sublimé sous vide. Après 24 heures, on a récupéré sur un doigt froid 6,17 g de pentafluoroéthanesulfonamide $C_2F_5SO_2NH_2$ (62% de rendement) ayant une pureté caractérisée par RMN du proton et du fluor supérieure à 99%.

Le sel de sodium correspondant a été préparé en faisant réagir la perfluoroéthanesulfonamide avec le carbonate de sodium $Na_2CO_3$ dans l'eau (20% en excès). Après évaporation de l'eau et séchage, le produit obtenu a été repris dans le tétrahydrofurane puis l'excès de carbonate a été éliminé par filtration. Après évaporation du tétrahydrofurane et séchage, on a obtenu quantitativement le sel de sodium de la perfluoroéthanesulfonamide $C_2F_5SO_2NHNa$.

La microanalyse a donné: H 0,42 (0,46) ; C 10,35 (10,87) ; N 6,73 (6,34) ; F 42,01 (42,97) ; Na 10,89 (10,4) ; S 14,25 (14,5).

Les sels de lithium et de potassium ont été obtenus par un procédé similaire, en remplaçant le carbonate de sodium respectivement par le carbonate de lithium et de potassium.

**Exemple 6 : Triflinate de potassium**

A une suspension dans 50 ml d'acétonitrile anhydre à -18°C de 22,64 g (100 mmoles) du di-sel de potassium du 2,2-dimercapto-1,3,4-thiadiazole (commercialisé par Aldrich), on a ajouté 17,36 g (103 mmoles) du chlorure de trifluorométhanesulfonyle $CF_3SO_2Cl$. Après 48 heures sous agitation à température ambiante, le milieu réactionnel a été filtré pour éliminer le chlorure de potassium et le poly(2,2-dimercapto-1,3,4-thiadiazole) formés au cours de la réaction suivant le schéma réactionnel ci-dessous:

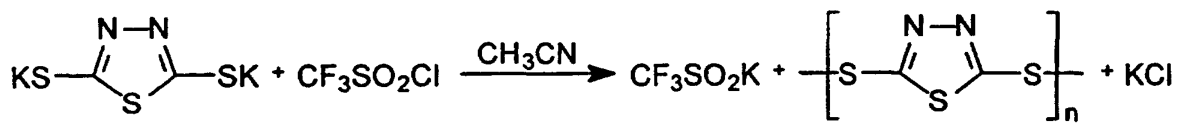


Après évaporation du solvant et séchage sous vide à température ambiante durant 24 heures, on a récupéré 16,3 g du triflinate de potassium $CF_3SO_2K$ (95% de rendement) ayant une pureté caractérisée par RMN du fluor supérieure à 98%.

La microanalyse a donné: C 6,72 (6,98) ; F 32,6 (33,11) ; S 18,32 (18,62) ; K 22,29 (22,71).

**Exemple 7 : Chlorure de 3-sulfonyl-1,2,4-triazine**

28,83 g (300 mmoles) de 3-amino-1,2,4-triazine (commercialisé par Aldrich) ont été ajoutés à un mélange sous agitation de 100 ml d'acide chlorhydrique concentré et de 30 ml d'acide acétique glacial. On a porté le milieu réactionnel à -10°C et on a alors ajouté lentement 22,42 g (325 mmoles) de nitrite de sodium $NaNO_2$ dans 35 ml d'eau. La réaction de diazotation s'est poursuivie pendant 1 heure. Dans le même temps, on a fait passer un courant de dioxyde de soufre $SO_2$ à travers un fritté dans 300 ml d'acide acétique glacial jusqu'à saturation. On a alors ajouté 7,5 g de chlorure de cuivre(I) CuCl et on a poursuivi l'addition de dioxyde de soufre jusqu'à ce que la couleur du milieu réactionnel passe du jaune-vert au bleu-vert. Après avoir porté la température du milieu réactionnel à une température inférieure à 10°C, on a ajouté le diazonium précédemment préparé sur une période de 30 min. On a ajouté un peu d'éther pour diminuer la quantité de mousse qui se forme après chaque addition. Après la fin de l'addition de diazonium, on a versé le milieu réactionnel dans un 1 litre d'un mélange eau-glace (1:1). Après fusion de la glace, on a séparé une huile jaune dans une ampoule à décanter, puis on a extrait par deux fractions de 100 ml d'éther la phase aqueuse. Après addition de la phase éthérée à l'huile recueillie, la solution a été lavée par une solution concentrée de bicarbonate de sodium jusqu'à neutralité, puis avec de l'eau, et enfin séchée par du sulfate de magnésium. Après évaporation du solvant, on a récupéré après distillation sous vide 35,1 g de 3-sulfonyl-1,2,4-triazine (65% de rendement) ayant une pureté caractérisée par RMN du proton et du fluor supérieure à 98%.

La microanalyse a donné: C 20,6 (20,1) ; H 0,6 (1,1) ; N 23,6 (23,4) ; S 17,6 (17,9) ; Cl 19,3 (19,7).

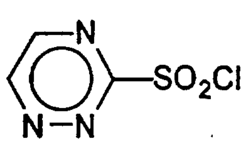

**Exemple 8 : 3-chloropropanesulfonyl(trifluorométhanesulfonyl)imide**

On a fait réagir dans 50 ml de tétrahydrofurane anhydre à 0°C, 17,7 g (100 mmoles) de chlorure de 3-chloropropanesulfonyle $Cl(CH_2)_3SO_2Cl$ et 37,44 g (200 mmoles) du sel de potassium de la trifluorométhanesulfonamide $CF_3SO_2NHK$. Après 3 heures à 0°C, puis 24 heures à l'ambiante, le tétrahydrofurane a été évaporé et le produit a été recristallisé dans 40 ml d'eau, récupéré par filtration et séché. On a ainsi obtenu 23,6 g du sel de potassium de la trifluorométhanesulfonyl(3-chloropropanesulfonyl)imide $Cl(CH_2)_3SO_2NKSO_2CF_3$ (72% de rendement) ayant une pureté caractérisée par RMN du proton et du fluor supérieure à 99%.

La microanalyse a donné: H 1,76 (1,85) ; C 14,23 (14,66) ; N 4,56 (4,27) ; F 17,78 (17,39) ; S 19,09 (19,56) ; Cl 10,28 (10,82) ; K 11,45 (11,93).

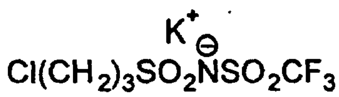

On a obtenu par un procédé similaire le sel de potassium de la fluorosulfonyl(3-chloropropanesulfonyl)imide (65% de rendement) à partir du sel de potassium de la fluorosulfonamide obtenu à l'exemple 2 et le sel de potassium de la pentafluoroéthanesulfonyl(3-chloropropanesulfonyl)imide (82% de rendement) à partir du sel de potassium de la pentafluorosulfonamide obtenu à l'exemple 5.

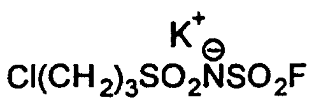

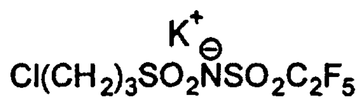

On a obtenu quantitativement les sels de lithium par traitement des sels de potassium dans le tétrahydrofurane anhydre par la quantité stoechiométrique de chlorure de lithium anhydre, filtration du milieu réactionnel, évaporation du solvant et séchage sous vide.

Ces trois sels sont solubles dans la plupart des solvants organiques usuels (tétrahydrofurane, acétonitrile, diméthylformamide, acétate d'éthyle, glymes) et dans les polymères solvatants aprotiques comme le poly (oxyde d'éthylène). Dans ce dernier solvant à une concentration O/K de 14/1, ils présentent une conductivité ionique supérieure à $10^{-3}$ $S.cm^{-1}$ à une température de 100°C.

Ces sels peuvent être greffés aisément sur différents substrats comportant un site suffisamment nucléophile tel qu'un alcoolate, un amidure ou un méthylure.

**Exemple 9: 2,2,2-trifluoroéthanesulfonyl(trifluorométhanesulfonyl)imide**

A une solution dans 30 ml d'acétonitrile anhydre à 0°C de 9,13 g (50 mmoles) de chlorure de 2,2,2-trifluoroéthanesulfonyle $CF_3CH_2SO_2Cl$ (commercialisé par Aldrich) et de 7,45 g (50 mmoles) de trifluorométhanesulfonamide $CF_3SO_2NH_2$, on a ajouté goutte à goutte 7,91 g (100 mmoles) de pyridine anhydre. Après deux heures à 0°C, la réaction s'est poursuivie pendant 48 heures à température ambiante. Le milieu réactionnel a alors été filtré pour éliminer l'hydrochlorure de pyridinium formé. On a ensuite agité le milieu réactionnel durant 48 heures avec 5,79 g (50 mmoles) de phosphate de lithium $Li_3PO_4$. Après filtration, évaporation du solvant et séchage, on a obtenu 14,6 g du sel de lithium de la trifluorométhanesulfonyl (2,2,2-trifluoroéthanesulfonyl)imide $CF_3CH_2SO_2NLiSO_2CF_3$ (rendement 97%).

La microanalyse a donné: H 0,72 (0,67) ; Li 2,48 (2,3) ; C 11,56 (11,97) ; N 4,88 (4,65) ; F 38,02 (37,86) ; S 21,56 (21,3).

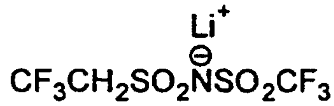

Ce sel présente une conductivité de $2,3.10^{-4}$ $S.cm^{-1}$ à 60°C dans le poly (oxyde d'éthylène) à une concentration O/Li de 12/1.

Il possède un proton présentant un caractère acide permettant d'effectuer des réactions de substitution nucléophile en présence d'une base avec des halogénures d'alkyles ou d'acides par exemple.

**Exemple 10 : N-méthyl-sulfonyl(trifluorométhanesulfonyl)imide**

Sous argon, on a ajouté goutte à goutte pendant 2 heures 100 ml d'une solution 2 M de méthylamine $CH_3NH_2$ (200 mmoles, commercialisé par Aldrich) dans le tétrahydrofurane à une solution à -20°C sous forte agitation de 13,5 g (100 mmoles) de chlorure de sulfuryle $SO_2Cl_2$ dans 50 ml de dichlorométhane anhydre. Après 3 heures à -20°C, le milieu réactionnel a été centrifugé pour éliminer le précipité d'hydrochlorure de méthylammonium $CH_3NH_3^+Cl^-$ formé. Après évaporation du tétrahydrofurane, le liquide restant a été distillé sous vide. On a obtenu 12,82 g de N-méthyl-chlorosulfonamide $ClSO_2NH(CH_3)$ (95% de rendement) ayant une pureté caractérisée par RMN du proton supérieure à 98%.

On a alors fait réagir dans 30 ml de tétrahydrofurane anhydre, 6,48 g (50 mmoles) de N-méthyl chlorosulfonamide avec 7,45 g (50 mmoles) de trifluorométhanesulfonamide, et avec 11,22 g (100 mmoles) de 1,4-diazabicyclo [2.2.2]octane (DABCO). Après 48 heures, le milieu réactionnel a été filtré pour éliminer le précipité d'hydrochlorure de DABCO formé. Après évaporation du solvant, le produit obtenu a été repris dans 20 ml d'éthanol et l'on a ajouté 14,91 g (100 mmoles) d'acétate de potassium. Il s'est alors formé un précipité. Après recristallisation, filtration et séchage, on a récupéré 9,95 g du sel de potassium de la trifluorométhanesulfonyl-(N-méthylsulfonyl)imide $CF_3SO_2NKSO_2NH(CH_3)$ (71% de rendement), dont la pureté caractérisée par RMN du proton et du fluor est supérieure à 98%.

La microanalyse a donné: H 1,31 (1,44) ; C 8,38 (8,57) ; N 9,85 (9,99) ; F 20,89 (20,34) ; S 22,35 (22,88) ; K 13,52 (13,95).

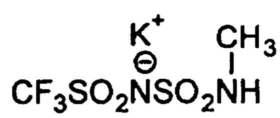

On a obtenu par un procédé similaire, le sel de potassium de la trifluorométhanesulfonyl(N-éthylsulfonyl)imide à partir de l'éthylamine et le sel de potassium de la trifluorométhanesulfonyl(N-propylsulfonyl)imide à partir de la propylamine.

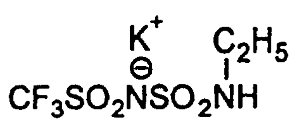

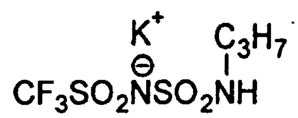

Les sels de lithium ont été préparés quantitativement par échange ionique entre les sels de potassium et le chlorure de lithium dans le tétrahydrofurane anhydre.

Ces composés possèdent un proton labile permettant d'effectuer des réactions de substitution nucléophile en présence d'une base avec des halogénures d'alkyles ou d'acides par exemple.

**Exemple 11 : 5-formyl-2-furansulfonyl(trifluorométhanesulfonyl)imide**

A 9,91 g (50 mmoles) du sel de sodium de l'acide 5-formyl-2-furansulfonique (commercialisé par Aldrich) dans 30 ml de diméthylformamide anhydre à 0°C, on a ajouté lentement 6,35 g (50 mmoles) de chlorure d'oxalyle ClCOCOCl en solution dans 20 ml de dichlorométhane anhydre, puis, après deux heures à 0°C, 18,72 g (100 mmoles) du sel de potassium de la trifluorométhanesulfonamide $CF_3SO_2NHK$. La réaction s'est poursuivie 48 heures à température ambiante, puis le solvant a été évaporé et le produit obtenu recristallisé dans 40 ml d'eau. Après filtration et séchage, on a récupéré 10,88 g du sel de potassium de la trifluorométhanesulfonyl(5-formyl-2-furansulfonyl)imide (63% de rendement) ayant une pureté déterminée par RMN du fluor et du proton supérieure à 99%.

La microanalyse a donné: H 1,01 (0,88) ; C 20,55 (20,87) ; N 4,15 (4,06) ; F 16,91 (16,51) ; S 18,17 (18,57) ; K 11,76 (11,32).

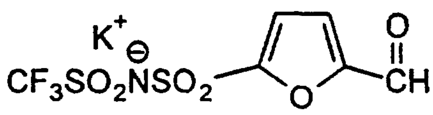

On a obtenu par le même procédé le sel de potassium de la fluorosulfonyl(5-formyl-2-furansulfonyl)imide.

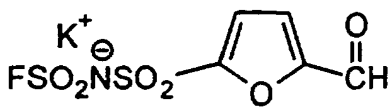

La fonction aldéhyde permet de greffer ce sel sur les substrats contenant un groupement capable de réagir avec cette fonction, par exemple un groupement amine ou une double liaisons.

**Exemple 12 : Allylsulfonyl(trifluorométhanesulfonyl)imide**

A 14,41 g (100 mmoles) du sel de sodium de l'acide 2-propène-sulfonique $CH_2=CHCH_2SO_3Na$ en suspension dans 60 ml d'acétonitrile anhydre à -20°C, on a ajouté goutte à goutte pendant 2 heures 11,9 g (100 mmoles) de chlorure de thionyle $SOCl_2$ dilués dans 20 ml de benzène. Après une nuit à -20°C, le milieu réactionnel a été centrifugé pour éliminer le chlorure de sodium formé et les solvants ont été évaporés à l'aide d'un évaporateur rotatif muni d'une pompe à membrane. Le liquide obtenu a alors été distillé sous vide sur une colonne courte pour donner 10,97 g de chlorure de 2-propène-sulfonyle $CH_2=CHCH_2SO_2Cl$ (rendement 78%) caractérisé par RMN du proton. On a alors fait réagir 7,03 g (50 mmoles) de ce composé avec 18,72 g (100 mmoles) du sel de potassium de la trifluorométhanesulfonamide $CF_3SO_2NHK$ dans 60 ml d'acétonitrile anhydre à 0°C pendant 2 heures, puis à l'ambiante pendant 24 heures. Après évaporation du solvant, le produit a été recristallisé dans 20 ml d'eau. Après filtration et séchage, on a récupéré 17,22 g du sel de potassium de la trifluorométhanesulfonyl-(2-propènesulfonyl)imide $CH_2=CHCH_2SO_2NKSO_2CF_3$ (66% de rendement) ayant une pureté caractérisée par RMN du proton et du fluor supérieure à 98%.

La microanalyse a donné: H 1,68 (1,73) ; C 16,22 (16,49) ; N 4,6 (4,81) ; F 19,12 (19,57) ; S 22,29 (22,01) ; K 13,23 (13,42).

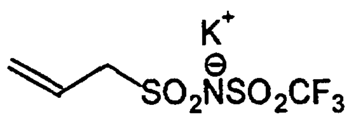

On a obtenu suivant le même procédé le sel de potassium de la pentafluoroéthanesulfonyl (2-propènesulfonyl) imide (69% de rendement) à partir du sel de potassium de la pentafluoroéthanesulfonamide obtenu à l'exemple 5.

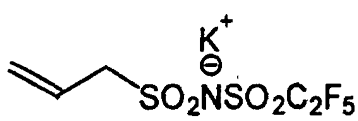

Ces sels présentent la particularité de pouvoir être homo- ou copolymérisés par une polymérisation initiée par voie radicalaire ou par un catalyseur de polymérisation des oléfines du type Ziegler-Natta, tel un zircanocène, et plus généralement de pouvoir subir les réactions chimiques propres aux liaisons éthyléniques.

**Exemple 13 : 3,4-époxypropane-1-sulfonyl(trifluorométhanesulfonyl)imide**

A 11,65 g (40 mmoles) du sel de potassium de la trifluorométhanesulfonyl(2-propènesulfonyl)imide, obtenu à l'exemple 12, dans 100 ml d'eau, on a ajouté 6,9 g (40 mmoles) de l'acide 3-chloroperoxybenzoïque obtenu suivant la procédure décrite par Scwartz & Blumbergs (J. Org. Chem., (1964), 1976). Après 1 heure sous forte agitation, le

solvant a été évaporé puis le résidu a été recristallisé dans 15 ml d'éthanol. Après filtration et séchage, on a récupéré 7,5 g du sel de potassium de la 2,3-époxypropane-1-sulfonyl(trifluorométhanesulfonyl)-imide (61% de rendement) ayant une pureté caractérisée par RMN du proton et du fluor supérieure à 98%.

La microanalyse a donné: H 1,84 (1,64) ; C 15,2 (15,63) ; N 4,99 (4,56) ; F 18,01 (18,55) ; S 20,15 (20,87) ; K 12,01 (12,72).

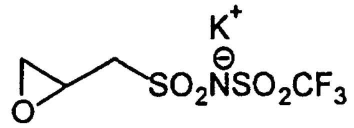

On a obtenu suivant la même procédure le sel de potassium de la 2,3-époxypropane-1-sulfonyl(pentafluoroéthanesulfonyl)-imide à partir du sel de potassium de la pentafluoroéthanesulfonyl(2-propènesulfonyl)imide obtenu à l'exemple 12.

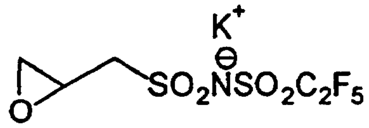

On a obtenu les sels de lithium par traitement des sels de potassium dans le tétrahydrofurane anhydre par la quantité stoechiométrique de chlorure de lithium anhydre, filtration du milieu réactionnel, évaporation du solvant et séchage sous vide.

Ces sels peuvent être homo- ou copolymérisés par une polymérisation initiée par voie anionique ou cationique. Plus généralement, ils peuvent subir les réactions chimiques propres aux oxétanes.

On a préparé l'homopolymère du sel de potassium de la 2,3-époxypropane-1-sulfonyl(trifluorométhanesulfonyl)imide par une polymérisation dans le tétrahydrofurane initiée par voie anionique par le tert-butoxyde de potassium, puis le polysel de lithium par échange ionique avec du chlorure de lithium anhydre. Ce dernier présente une conductivité en milieu gélifié (21% en poids de polyacrylonitrile, 38% de carbonate d'éthylène, 33% de carbonate de propylène, 8% de l'homopolymère) de $1,1.10^{-3}$ $S.cm^{-1}$ à 30°C. Le nombre de transport cationique dans cet électrolyte est de 0,82. De plus, cet homopolymère est soluble dans la plupart des solvants organiques usuels (tétrahydrofurane, acétonitrile, diméthylformamide, acétate d'éthyle, glymes) et dans les polymères solvatants aprotiques.

**Exemple 14: Vinylsulfonyl(trifluorométhanesulfonyl)imide**

A une solution à 0°C et sous argon de 8,15 g (50 mmoles) de chlorure de 2-chloro-1-éthanesulfonyle $ClCH_2CH_2SO_2Cl$ (commercialisé par Aldrich) et de 7,45 g (50 mmoles) de trifluorométhanesulfonamide $CF_3SO_2NH_2$ dans 25 ml de tétrahydrofurane anhydre, on a ajouté goutte à goutte pendant 30 min une solution de 16,83 g (150 mmoles) de DABCO dilués dans 25 ml de tétrahydrofurane anhydre. Après la fin de l'addition de la base, la réaction s'est poursuivie pendant 2 heures à 0°C, puis pendant 24 heures à l'ambiante. Le milieu réactionnel a alors été filtré pour éliminer l'hydrochlorure de DABCO formé. On a ensuite ajouté 2,12 g de chlorure de lithium anhydre (50 mmoles), on a agité le milieu réactionnel pendant 24 heures, et on l'a de nouveau filtré pour éliminer l'hydrochlorure de DABCO formé. Après évaporation du tétrahydrofurane et séchage, on a récupéré 11,89 g du sel de lithium de la trifluorométhanesulfonyl(vinylsulfonyl)imide $CH_2=CHSO_2NLiSO_2CF_3$ (98% de rendement) ayant une pureté caractérisée par RMN du proton et du fluor supérieure à 98%.

La microanalyse a donné: H 1,28 (1,23) ; Li 2,78 (2,83) ; C 14,91 (14,7) ; N 5,82 (5,71) ; F 22,5 (23,25) ; S 25,8 (26,16).

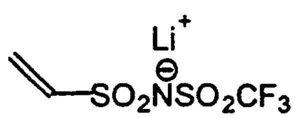

On a obtenu suivant le même procédé le sel de lithium de la perfluorobutanesulfonyl (vinylsulfonyl)imide (99%

de rendement) à partir de la perfluorobutanesulfonamide obtenu à l'exemple 4.

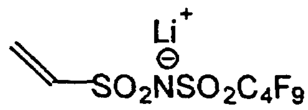

Ces sels peuvent être homo- ou copolymérisés par une polymérisation initiée par voie radicalaire. Plus généralement, ils peuvent subir les réactions chimiques propres aux liaisons vinyliques activées, en particulier des additions de Michael, avec un alcoolate par exemple.

**Exemple 15 : 7,8-octène-3,6-oxa-1-sulfonyl(trifluorométhanesulfonyl)imide**

A 2,2 g (25 mmoles) de l'éther vinylique de l'éthylène glycol $CH_2=CHO(CH_2)_2OH$ dans 60 ml de diméthylformamide anhydre, on a ajouté 6,13 g (25 mmoles) du sel de lithium de la vinylsulfonyl(trifluorométhanesulfonyl)imide, obtenu à l'exemple 14, 5,87 g de carbonate de potassium $K_2CO_3$ anhydre (42,5 mmoles) et 330 mg (1,25 mmoles) d'un éther-couronne, le 18-Crown-6 (agissant comme complexant du cation potassium). Le milieu réactionnel a alors été agité sous argon à 85°C. Après 48 heures, le milieu réactionnel a été filtré sur un verre fritté de porosité N°3, puis le solvant évaporé sous pression réduite. Après séchage, le composé a été recristallisé dans 10 ml d'eau contenant 1,86 g (25 mmoles) de chlorure de potassium KCl anhydre. Après filtration et séchage, on a récupéré 5,66 g du sel de potassium de la 7,8-octène-3,6-oxa-1-sulfonyl(trifluorométhane-sulfonyl)imide (62% de rendement) ayant une pureté caractérisée par RMN du proton et du fluor supérieure à 98%.

La microanalyse a donné: H 3,12 (3,03) ; C 23,26 (23,01) ; N 3,77 (3,83) ; F 15,89 (15,6) ; S 17,12 (17,55) ; K 10,23 (10,7).

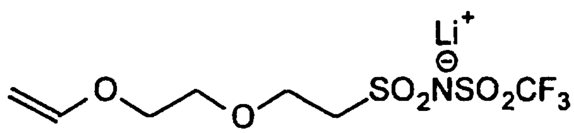

On a obtenu quantitativement le sel de lithium par traitement du sel de potassium dans le tétrahydrofurane anhydre par la quantité stoechiométrique de chlorure de lithium anhydre, filtration du milieu réactionnel, évaporation du solvant et séchage sous vide.

Ce sel peut être homopolymérisé par par voie cationique. Il peut également être copolymérisé par voie cationique, éventuellement par une polymérisation alternée avec un monomère insaturé. Plus généralement, il peut subir les réactions chimiques propres aux alkyl vinyl éthers.

L'homopolymère préparé par une polymérisation dans l'acétonitrile anhydre initiée par voie cationique par la bis(trifluorométhanesulfonyl)imide présente une conductivité à une concentration de 0,8 M dans un mélange de diméthylcarbonate et de carbonate d'éthylène (2:1) de $6.10^{-3}$ $S.cm^{-1}$ à 30°C. De plus, cet homopolymère est soluble dans la plupart des solvants organiques usuels (tétrahydrofurane, acétonitrile, diméthylformamide, acétate d'éthyle, glymes) et dans les polymères solvatants aprotiques comme le poly (oxyde d'éthylène).

**Exemple 16 : 4-styrènesulfonyl(trifluorométhanesulfonyl)imide**

Dans 100 ml de tétrahydrofurane anhydre sous argon à 0°C, on a fait réagir 20,27 g (10 mmoles) de chlorure de 4-styrènesulfonyle $CH_2=CHC_6H_4SO_2Cl$ (commercialisé par Monomer-Polymer & Dajac Laboratories), 14,91 g (10 mmoles) de trifluorométhanesulfonamide $CF_3SO_2NH_2$ et 22,44 g (20 mmoles) de (DABCO). Après 2 heures à 0°C et 48 heures à la température ambiante, la solution a été filtrée pour éliminer l'hydrochlorure de DABCO formé, puis traitée par 424 mg (10 mmoles) de chlorure de lithium anhydre, stocké et pesé en boîte à gants. Il s'est formé immédiatement un précipité d'hydrochlorure de DABCO et le milieu réactionnel a alors été de nouveau filtré après une agitation de 6 heures. Après évaporation et séchage sous vide pendant 24 heures à l'ambiante, on a récupéré 31,16 g du sel de lithium de la trifluorométhanesulfonyl(4-styrènesulfonyl)imide ayant une pureté caractérisée par RMN du proton et du fluor supérieure à 97%.

La micronalyse a donné: H 2,4 (2,2) ; Li 2,56 (2,16) ; C 33,15 (33,65) ; N 4,79 (4, 36) ; F 17,14 (17,74) ; S 19,51 (19,96).

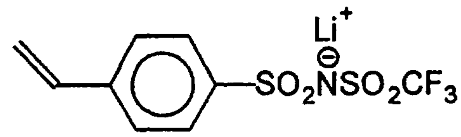

On a préparé suivant le même procédé les sels de lithium de la fluorosulfonyl(4-styrènesulfonyl)imide (98% de rendement) à partir de la fluorosulfonamide obtenu à l'exemple 2, de la pentafluoroéthanesulfonyl(4-styrènesulfonyl) imide (97% de rendement) à partir de la pentafluoroéthanesulfonamide obtenu à l'exemple 5 et de la perfluorobutanesulfonyl-(4-styrènesulfonyl)imide (99% de rendement) à partir de la perfluorobutanesulfonamide obtenu à l'exemple 4.

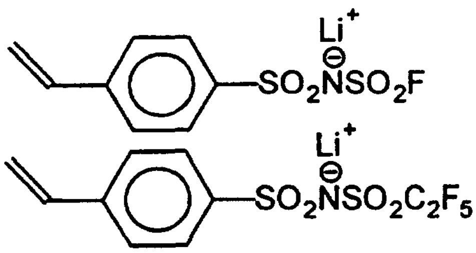
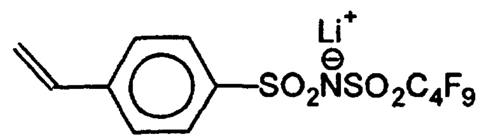

Ces sels peuvent être homo- ou copolymérisés par une polymérisation initiée par voie anionique, cationique et plus particulièrement radicalaire. On peut également les greffer sur une matrice polymère telle que le polyfluorure de vinylidène par irradiation.

Les homopolymères obtenus par polymérisation radicalaire dans l'eau désaérée, initiée par l'acide cyanovalérique à 60°C, sont solubles dans les solvants organiques usuels et dans les polymères solvatants aprotiques. Dans le poly (oxyde d'éthylène) à une concentration O/Li de 16/1, ces sels présentent une conductivité ≈ $6.10^{-4}$ $S.cm^{-1}$ à 100°C. De plus, en solution concentrée dans l'acétone (≈ 1 M en cation lithium), ces homopolymères peuvent être utilisés comme catalyseurs des réactions de Diels-Alder, en ce sens ils se comportent comme des micro-réacteurs chimiques.

**Exemple 17 : 5-(4-méthylène-1,3-dioxolane)-2-furanesulfonyl(trifluorométhanesulfonyl)imide**

On a mélangé dans 30 ml de toluène, 5,18 g (15 mmoles) du sel de potassium de la trifluorométhanesulfonyl (5-formyl-2-furansulfonyl)imide, 1,66 g (15 mmoles) de 3-chloro-1,2-propanediol $ClCH_2CH(OH)CH_2(OH)$ (commercialisé par Aldrich) et ≈ 1 mg d'acide p-toluènesulfonique monohydrate. On a alors effectué une distillation azéotropique jusqu'à ce que l'on n'observe plus d'apparition d'eau dans le Dean-Stark. Après évaporation du solvant, le produit obtenu a été recristallisé dans 10 ml d'eau. Après filtration et séchage, on a récupéré 5,13 g du sel de potassium de la 5-(4-méthylène-1,3-dioxolane)-2-furanesulfonyl(trifluorométhanesulfonyl)imide (82% de rendement) ayant une pureté déterminée par RMN du proton et du fluor supérieure à 98%.

La microanalyse a donné: C 26,65 (26,93) ; H 1,89 (1,76) ; N 3,99 (3,49) ; S 15,28 (15,98) ; F 13,8 (14,2) ; K 9,41 (9,74).

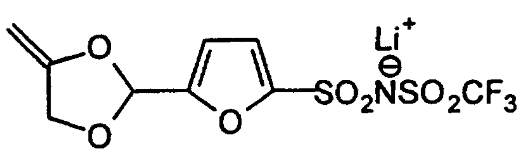

On a obtenu quantitativement le sel de lithium par traitement du sel de potassium dans le tétrahydrofurane anhydre par la quantité stoechiométrique de chlorure de lithium anhydre, filtration du milieu réactionnel, évaporation du solvant et séchage sous vide.

Ce sel peut être homo- ou copolymérisé par une polymérisation initiée par voie cationique ou radicalaire. L'homopolymère de ce sel a été obtenu par une photopolymérisation, initiée par voie cationique par l'irradiation de l'hexafluoroantimonate de tris(4-méthylphényl)sulfonium par une lampe U.V. pendant 10 min à 36°C. Il présente une

conductivité à une concentration de 0,5 M dans la tétraéthylsulfamide $(C_2H_5)_2NSO_2N(C_2H_5)$ de $4.10^{-3}$ $S.cm^{-1}$ à 20°C.

**Exemple 18:1-acryloyl-2,2,2-trifluoroéthanesulfonyl-(trifluorométhanesulfonyl)imide**

En opérant dans une boîte à gants sous argon, on a mis en solution 7,53 g (25 mmoles) du sel de lithium de trifluorométhanesulfonyl (2,2,2-trifluoroéthanesulfonyl)imide $CF_3CH_2SO_2NLiSO_2CF_3$, préparé comme dans l'exemple 9, dans 15 ml de tétrahydrofurane anhydre. Après avoir porté cette solution à -20°C, on a ajouté lentement 50 ml d'une solution 1 M (25 mmoles) dans le tétrahydrofurane de bis(triméthylsilyl)amidure de sodium $((CH_3)_3Si)_2NNa$ (commercialisé par Aldrich). Après 15 min, on a ajouté lentement 2,26 g (25 mmoles) de chlorure d'acryloyle $CH_2$=CHCOCl préalablement purifié par distillation sous vide. La réaction s'est poursuivie pendant deux heures à -20°C, puis le milieu réactionnel a été filtré pour éliminer le précipité de chlorure de sodium. On a alors évaporé le solvant et on a obtenu après séchage sous vide à température ambiante 8,7 g (98% de rendement) du sel de lithium de la trifluorométhane-sulfonyl(1-acryloyl-2, 2, 2-trifluoroéthanesulfonyl) imide $CH_2=CHCOCH(CF_3)-SO_2NLiSO_2CF_3$ ayant une pureté caractérisée par RMN du proton et du fluor supérieure à 98%.

La microanalyse a donné: H 1,26 (1,14) ; Li 1,69 (1,95) ; C 20,06 (20,29) ; N 3,79 (3, 94) ; F 32,33 (32,1) ; S 18,26 (18,05).

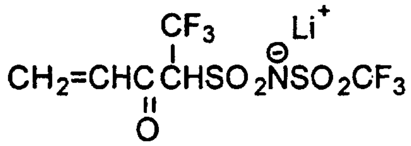

Ce sel peut être homo- ou copolymérisé par photopolymérisation en présence d'un photosensibilisateur.

On a réalisé un mélange contenant ce sel (16% en poids), du poly(éthylène glycol)diméthacrylate ayant une masse molaire de 600 g/mole (81% en poids, commercialisé par Aldrich), des particules de silice ayant une surface spécifique de 300 $m^2/g$ (3% en poids, Aérosil, commercialisé par Degussa AG) et de la xanthone. On a déposé cette solution à la tournette sur une plaque de verre recouverte d'une couche de trioxyde de tungstène $WO_3$ et d'une sous-couche conductrice d'oxyde d'étain. On a obtenu une membrane optiquement transparente dans le visible et adhérente sur le support par une photopolymérisation déclenchée par irradiation à l'aide d'une lampe U.V. pendant 10 min à 32°C. On a ensuite réalisé un système électrochrome en assemblant en boîte à gants une contre électrode constituée par le dépôt sur une plaque de verre d'une couche d'oxyde d'iridium hydrogéné $H_xIrO_2$ et d'une sous couche d'oxyde d'étain. Cet électrochrome a donné une variation de l'absorption optique de 80% (état décoloré) à 30% (état coloré) et de bonnes performances en cyclage. On a ainsi pu réaliser un nombre de cycles de coloration/décoloration supérieure à 20 000.

**Exemple 19 : 3-maléimidopropanesulfonyl(trifluorométhanesulfonyl)imide**

A une solution dans 20 ml de tétrahydrofurane anhydre à 0°C de 2,43 g de maléimide (25 mmoles), on a ajouté par portions 215 mg d'hydrure de lithium LiH (27 mmoles). Après une heure, on a ajouté à la solution filtrée 8,19 g (25 ml) du sel de potassium de la trifluorométhanesulfonyl(3-chloropropanesulfonyl)imide préparé comme à l'exemple 8. La réaction s'est poursuivie pendant 24 heures à 60°C, puis le milieu réactionnel a été filtré pour éliminer le précipité de chlorure de potassium KCl, le solvant a été évaporé et le produit séché. On a ainsi obtenu 8,37 g (rendement 94%) du sel de lithium de la trifluorométhanesulfonyl(3-maléimidopropanesulfonyl) imide (-COCH=CHCO-)N $(CH_2)_3SO_2NLiSO_2CF_3$ ayant une pureté caractérisée par RMN du proton et du fluor ≈ 96%.

La microanalyse a donné: H 2,15 (2,26) ; Li 2,15 (1,95) ; C 26,72 (26,97) ; N 7.66 (7,86) ; F 16,54 (16) ; S 18,25 (18).

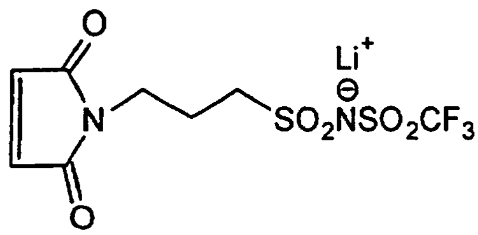

On a obtenu suivant le même procédé le sel de lithium de la pentafluoroéthanesulfonyl(3-maléimidopropanesulfonyl)imide (98% de rendement) à partir du sel de potassium de la pentafluoroéthanesulfonyl(3-chloropropanesulfonyl)imide obtenu à l'exemple 8.

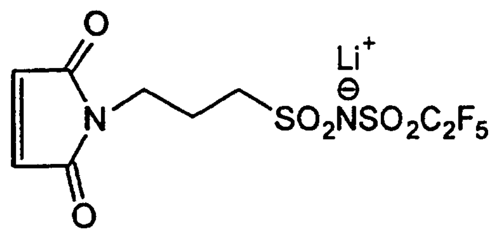

Ces sels peuvent être homopolymérisés par voie radicalaire ou par voie anionique ou copolymérisés par voie anionique ou par voie radicalaire éventuellement par une polymérisation alternée avec un monomère donneur d'électrons (N-vinyl-2-pyrrolidone, N-vinyl formamide, éther vinylique).

L'homopolymère préparé par une polymérisation dans le tétrahydrofurane anhydre à -78°C, initiée par voie anionique par le sec-butyllithium, est soluble dans les solvants organiques usuels (tétrahydrofurane, acétonitrile, diméthylformamide, acétate d'éthyle, glymes) et dans les polymères solvatants aprotiques comme le poly (oxyde d'éthylène).

**Exemple 20 : 2-(triéthoxysilyl)éthanesulfonyl(trifluorométhanesulfonyl)imide**

Dans un réacteur chimique Parr, on a introduit une solution de 9,36 g (50 mmoles) du sel de potassium de la trifluorométhanesulfonamide $CF_3SO_2NHK$ et de 264 mg d'un éther-couronne complexant du cation potassium, le 18-Crown-6, dans 60 ml d'acétonitrile anhydre. Après avoir fermé le réacteur, on a effectué un balayage d'argon pendant 15 min avant de l'isoler. On a alors introduit 6,41 g (50 mmoles) de dioxyde de soufre $SO_2$ (commercialisé par Fluka) puis, après 10 min, 9,52 g (50 mmoles) de vinyltriéthoxysilane (commercialisé par Fluka) en solution dans 20 ml d'acétonitrile anhydre. Après 6 heures à température ambiante, le réacteur a été porté à 40°C et maintenu à cette température pendant 48 heures, puis le solvant a été évaporé. Après avoir été séché sous vide, le produit a été stocké sous argon. On a récupéré quantitativement le sel de potassium de la trifluorométhanesulfonyl(2-(triéthoxysilyl)éthanesulfonyl)imide ayant une pureté caractérisée par RMN du fluor et du proton supérieure à 99%.

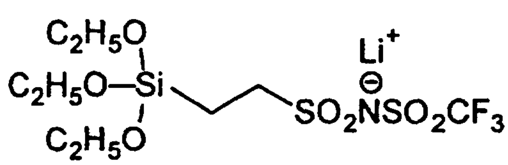

La microanalyse a donné: H 4,79 (4, 34) ; K 8,27 (8,85) ; C 24,99 (24,48) ; N 3,89 (3,17) ; F 12,15 (12,91) ; Si 6,75 (6,36) ; S 14,33 (14,52).

On a obtenu le sel de lithium par échange ionique avec le chlorure de lithium dans le tétrahydrofurane.

On a obtenu l'acide correspondant en cobroyant dans un mortier en agate, en boîte à gants, le sel de potassium avec trois équivalents (150 mmoles) d'hydrogénosulfate d'ammonium $HSO_4NH_4$ anhydre (commercialisé par Aldrich). Puis par sublimation sous vide secondaire à 40°C, on a récupéré après 24 heures sur un doigt froid porté à

une température de -40°C, la trifluorométhanesulfonyl (2-(triéthoxysilyl)éthanesulfonyl)-imide.

Ces sels permettent de former des réseaux organosiliciés par un mécanisme d'hydrolyse-polycondensation. Ils permettent également d'ensimer les matériaux à base de verre (fibre, vitrage) afin de modifier leur état de surface.

En outre, on peut obtenir les homopolymères ou des copolymères avec divers alkoxysilanes en milieu protique, éventuellement en présence d'un catalyseur (acide, base, fluorure ).

On a préparé un copolymère en polycondensant le sel de potassium de la trifluorométhanesulfonyl(2-(triéthoxysilyl)-éthanesulfonyl)imide avec le O-[2-(triméthoxysilyl)éthyl]-O'-méthylpolyéthylène glycol de masse 5000 (commercialisé par Shearwaters Polymers) (5:1 molaire) dans un mélange eau/méthanol, en utilisant comme catalyseur la trifluorométhanesulfonyl(2-(triéthoxysilyl)éthanesulfonyl)imide. Après quelques heures la solution a été concentrée. On a alors imprégné un feutre de carbone activé, préalablement dégazé:, ayant une surface spécifique de 1500 $m^2$/g (commercialisé par la société Actitex), avec le liquide visqueux obtenu. Après séchage, cette opération a été renouvelée pour parfaire l'imprégnation. Après avoir maintenu le feutre imprégné pendant une semaine dans une étuve à 50°C, on a découpé à l'emporte-pièces deux pastilles d'un diamètre de 2 cm. On a alors imprégné une feuille de papier à cigarette (commercialisée par Bolloré Technologies) par un liquide visqueux identique à celui utilisé pour imprégner le feutre de carbone. Cette feuille a été déposée entre les deux pastilles de feutre imprégné qui servent d'électrodes de carbone. Après une semaine dans une étuve à 50°C, puis 2 jours sous vide à 60°C, on a obtenu une supercapacité électrochimique "tout-solide". Cette supercapacité a donné les performances suivantes à 40°C : une densité d'énergie de 15 Wh/l (soit une capacité de 96 F/g de carbone pour une électrode), une puissance maximum de 700 W/kg et de bons résultats en cyclage (plus de 10 000 cycles de charge/décharge entre 0 et 2V). Une telle supercapacité est particulièrement intéressante pour l'électronique du fait de l'absence de liquides volatils.

On a également préparé une solution du sel de potassium de la trifluorométhanesulfonyl(2-(triéthoxysilyl) éthanesulfonyl) imide avec le O-[2-(triméthoxysilyl)éthyl]-O'-méthylpolyéthylène glycol de masse 5000 (commercialisé par Shearwaters Polymers) (3:1 molaire) dans un mélange eau/méthanol. Une plaque de verre décapée à l'acide nitrique puis séchée à 100°C a ensuite été trempée dans la solution pendant quelques minutes. Après rincage au méthanol et séchage, on a mesuré une conductivité de surface de $3.10^{-5}$ S(carré) suffisante pour donner des propriétés antistatiques à la surface du verre.

**Exemple 21 : bis[3-(triméthoxysilyl)propyl]aminosulfonyl(trifluorométhanesulfonyl)imide**

5,96 g (40 mmoles) de trifluorométhanesulfonamide $CF_3SO_2NH_2$ et 8,97 g (40 mmoles) de DABCO dans 60 ml de dichlorométhane anhydre ont été refroidis à -30°C. On a alors ajouté goutte à goutte 5,4 g (40 mmoles) de chlorure de sulfuryle $SO_2Cl_2$, puis 12,54 g (40mmoles) de bis[3-(triméthoxysilyl)propyl]amine de formule $[(CH_3O)_3Si(CH_2)_3]_2NH$. Le mélange a été agité pendant 4 heures à -30°C, puis 24 heures à température ambiante. On a alors ajouté 1,7 g de chlorure de lithium anhydre LiCl, le milieu réactionnel a été agité pendant 24 heures, puis filtré pour éliminer le précipité d'hydrochlorure de DABCO. Après évaporation du solvant et séchage sous vide, on a récupéré 21,88 g (98% de rendement) du sel de lithium de la bis[3-(triméthoxysilyl)propyl]aminosulfonyl(trifluorométhanesulfonyl)imide ayant une pureté caractérisée par RMN du fluor et du proton supérieure à 98%.

La microanalyse a donné: H 5,32 (5,41) ; Li 1,56 (1,24) ; C 27,66 (27,95) ; N 5,22 (5,01) ; F 10,56 (10,2) ; Si 10,26 (10,06) ; S 11,67 (11,48).

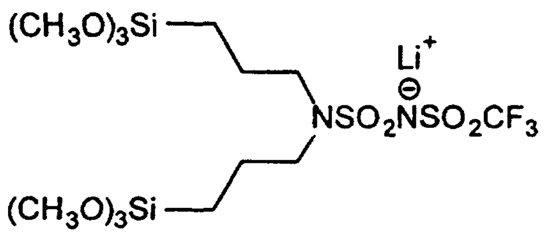

Ce composé présente des propriétés analogues à celles du composé de l'exemple 20 et peut être utilisé pour les mêmes applications.

On a effectué une polycondensation de ce composé dans un mélange eau/méthanol, en utilisant une goutte d'acide chlorhydrique concentré comme catalyseur. Après quelques heures, les solvants ont été évaporés et le liquide visqueux obtenu a été coulé sur une plaque de Téflon®. Après une semaine dans une étuve à 50°C, le matériau obtenu a été séché sous vide à 100°C pendant 48 heures, puis broyé sous argon jusqu'à obtenir une taille de particules de l'ordre du micron. On a alors préparé un électrolyte composite en mélangeant cette poudre avec du poly (oxyde d'éthylène) de masse $M_w = 3.10^5$ dans l'acétonitrile. Après avoir coulé cette dispersion dans un anneau en verre et évaporé

l'acétonitrile, on a obtenu un film d'électrolyte composite ayant une bonne tenue mécanique, et une épaisseur de 220 µm. Cet électrolyte présente une conductivité ionique supérieure à $10^{-5}$ $s^1.cm^{-1}$ à 60°C. Le nombre de transport cationique est de 0,92.

**Exemple 22 : N-méthyl-N-vinylester-sulfonyl(trifluorométhanesulfonyl)imide**

A 7,01 g (25 mmoles) du sel de potassium de la trifluorométhanesulfonyl (N-méthylsulfonyl)imide $CF_3SO_2NKSO_2NH(CH_3)$, préparé comme dans l'exemple 10, en solution dans 15 ml de tétrahydrofurane anhydre, on a ajouté lentement sous argon 25 ml d'une solution 1 M dans le tétrahydrofurane de tert-butoxide de potassium $(CH_3)_3COK$ (25 mmoles, commercialisé par Aldrich). Après quelques minutes, on a ajouté 2,66 g (25 mmoles) de vinylchloroformate $CH_2$=$CHO_2CCl$ (commercialisé par Lancaster) préalablement distillé sous vide. La réaction s'est poursuivie pendant 24 heures à température ambiante. On a ensuite filtré le milieu réactionnel pour éliminer le précipité de chlorure de potassium, évaporé le solvant et séché le produit sous vide. On a obtenu 8,58 g (98% de rendement) du sel de potassium de la trifluorométhanesulfonyl(N-méthyl-N-vinylester-sulfonyl) imide $CF_3SO_2NKSO_2N(CH_3)CO_2CH$=$CH_2$.

La microanalyse a donné: H 1,56 (1/73) ; C 17,56 (17,14) ; N 8,37 (8) ; F 17,01 (16,27) ; S 18,56 (18,3) ; K 11,46 (11,16).

On a obtenu quantitativement le sel de lithium par traitement du sel de potassium dans le tétrahydrofurane anhydre par la quantité stoechiométrique de chlorure de lithium anhydre, filtration du milieu réactionnel, évaporation du solvant et séchage sous vide.

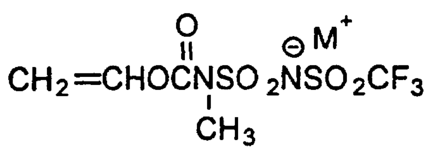

M = Li, K

Ce sel peut être homo- ou copolymérisé par une polymérisation initiée par voie radicalaire.

On a réalisé un film d'électrolyte polymère, d'une épaisseur de 30 µm, constitué par le sel de lithium en solution dans une matrice de poly (oxyde d'éthylène) possédant des insaturations éthyléniques, à une concentration O/Li = 26/1, et contenant 1% en poids d'acide cyanovalérique et 3% en poids de silice d'une surface spécifique de 300 $m^2$/g (Aérosil, commercialisé par Degussa AG). Ce polymère a été obtenu par polycondensation de polyéthylène glycol de masse 1000 avec le 3-chloro-2-chlorométhyl-1-propène selon la procédure décrite par Alloin & al. (J. Power Sources, (1995), 26, 34-39). On a réalisé d'autre part, sur un feuillard d'aluminium, une électrode composite d'une épaisseur de 90 µm contenant 45% en volume de dioxyde de vanadium ($V_2O_5$), 5% en volume de Ketjenblack® K600 (commercialisé par la société Akzo) comme additif de conduction électronique et 50% en volume d'un électrolyte polymère de même composition que celui décrit ci-dessus. En boîte à gants sous argon, on a ensuite déposé sur l'électrode composite le film d'électrolyte polymère, lui-même recouvert par un film de lithium d'une épaisseur de 30 µm déposée sur un feuillard en aluminium. L'ensemble a alors été porté à une température de 60°C pendant 24 heures en appliquant une légère pression. On a ainsi obtenu un générateur électrochimique à anions fixés, le sel de lithium co-réticulant avec les doubles liaisons de la matrice polymère. Ce générateur a donné un résultat de cyclage satisfaisant à 70°C (72% de la capacité après 10 cycles au 500ème cycle de charge/décharge). Les performances lors d'appels de puissance sont également améliorées.

**Exemple 23 : 4-perfluorovinyloxyphénylsulfonyl (pentafluorosulfonyl)imide**

Sous argon, à 9,95 g (50 mmoles) de pentafluoroéthanesulfonamide $C_2F_5SO_2NH_2$ en solution dans 40 ml de tétrahydrofurane anhydre à -20°C, on a ajouté lentement 10 ml d'une solution 10 M de butyllithium dans l'hexane $C_4H_9Li$ (100 mmoles). Après deux heures, on a ajouté 14,63 g de chlorure de sulfonyle du (3-(1,1,2,2-tétrafluoroéthoxy) benzène (50 mmoles), préparé à partir de la (3-(1,1,2,2-tétrafluoroéthoxy)aniline suivant le procédé général décrit dans l'exemple 7. La réaction s'est poursuivie pendant 24 heures à -20°C, puis on a ajouté lentement 5 ml d'une solution 10 M de butyllithium dans l'hexane $C_4H_9Li$ (50 mmoles). Après deux heures, on a laissé remonter la température à l'ambiante et les solvants ont été évaporés. Le produit a été repris dans 30 ml d'éthanol et recristallisé après addition de 4,91 g (50 mmoles) d'acétate de potassium $CH_3COOK$. On a obtenu après filtration et séchage 17,25 g du sel de potassium de la pentafluoroéthanesulfonyl(3-(1,1,2-trifluorovinyloxy)benzènesulfonyl)imide (78% de rendement) ayant une pureté caractérisée par RMN du proton et du fluor supérieure à 98%.

La microanalyse a donné: H 1,1 (0,85) ; C 25,62 (25,37) ; N 2,69 (2,96) ; F 33,1 (32,11) ; S 13,16 (13,55) ; K

8,95 (8,26).

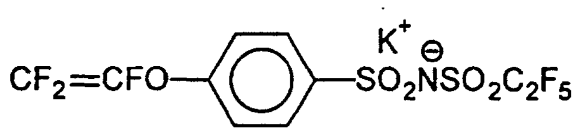

On a obtenu suivant le même procédé le sel de potassium de la trifluorométhanesulfonyl(3-(1,1,2-trifluorovinyloxy)-benzènesulfonyl)imide (98% de rendement) à partir de la trifluorométhanesulfonamide.

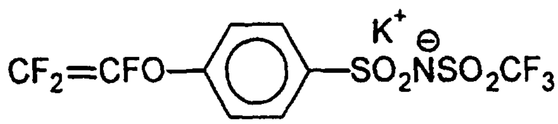

Les acides correspondant ont été obtenus par extraction à l'éther de solutions aqueuses acidifiées des différents sels de potassium. Les sels de lithium ont été obtenus par traitement des différents acides par le carbonate de lithium $Li_2CO_3$.

Ces sels peuvent être homo- ou copolymérisés par une polymérisation initiée par voie radicalaire.

On a impregné un textile poreux de GORE-TEX® d'une épaisseur de 100 µm, commercialisé par la société Gore, avec une solution concentrée dans le dichlorométhane de l'acide de la trifluorométhanesulfonyl (3-(1,1,2-trifluorovinyloxy)-benzènesulfonyl)imide contenant comme initiateur de polymérisation de l'acide cyanovalérique. Après évaporation du solvant, l'acide a été homopolymérisé au sein de la matrice textile en portant, sous argon, la température de l'ensemble à 60°C pendant 24 heures. La membrane ainsi obtenue a été utilisée comme électrolyte dans une cellule test de pile à combustible hydrogène/oxygène à électrolyte polymère. On a obtenu une durée de vie de cette membrane supérieure à 1000 heures avec de bonnes performances en puissance. Cette membrane peut aussi être utilisée pour la catalyse hétérogène de Friedel-Crafts de la réaction d'acylation du toluène par le chlorure de benzoyle.

**Exemple 24: 2,2-fluorovinylsulfonyl(trifluorométhanesulfonyl)imide**

Sous argon, à 6,02 g (20 mmoles) du sel de lithium de la trifluorométhanesulfonyl(2,2,2-trifluoroéthanesulfonyl)imide), obtenu à l'exemple 9, en solution dans 40 ml de tétrahydrofurane anhydre à -20°C, on a ajouté lentement 10 ml d'une solution 2 M de butyllithium dans le cyclohexane $C_4H_9Li$ (20 mmoles, commercialisé par Aldrich). Après deux heures à -20°C, le milieu réactionnel a été centrifugé pour éliminer le précipité de fluorure de lithium apparu au cours de la réaction. On a récupéré quantitativement après évaporation des solvants et séchage, le sel de lithium de la 2,2-fluorovinylsulfonyl(trifluorométhanesulfonyl)imide ayant une pureté déterminée par RMN du proton et du fluor supérieure à 99%.

La microanalyse a donné: H 0,47 (0,36) ; Li 2,71 (2,47) ; C 12,51 (12,82) ; N 4,72 (4,98) ; F 33,54 (33,79) ; S 22,65 (22,81).

$$CF_2{=}CHSO_2\overset{\ominus}{N}(Li^+)SO_2CF_3$$

Ce sel peut être homo- ou copolymérisé par une polymérisation initiée par voie radicalaire.

L'homopolymère préparé par une polymérisation dans le tétrahydrofurane anhydre à 66°C, initiée par voie radicalaire par le 1,1'-azobis(cyclohexanecarbonitrile), est soluble dans les solvants organiques usuels (tétrahydrofurane, acétonitrile, diméthylformamide, acétate d'éthyle, glymes) et dans les polymères solvatants aprotiques comme le poly (oxyde d'éthylène). En solution aqueuse à 25°C, il présente une conductivité 9,3.$10^{-3}$ $S.cm^{-1}$ à une concentration de 0,5 M. Il confère des propriétés antistatiques à des revêtements le contenant.

**Exemple 25 : Diméthylaminosulfonyl(trifluorométhanesulfonyl)imide**

A une solution à 0°C et sous argon de 14,36 g (100 mmoles) de chlorure de sulfamoyle $(CH_3)_2NSO_2Cl$ (commercialisé par Aldrich) et de 14,91 g de trifluorométhanesulfonamide $CF_3SO_2NH_2$ (100 mmoles) dans 60 ml de tétrahydrofurane anhydre, on a ajouté 22,44 g (200 mmoles) de DABCO en solution dans 20 ml de tétrahydrofurane anhydre à 0°C. Après 2 heures à 0°C, la réaction s'est poursuivie pendant 24 heures à l'ambiante. Le précipité d'hydrochlorure de DABCO a été éliminé par filtration sur un verre fritté de porosité N°4. On a alors ajouté 4,24 g (100 mmoles) de chlorure de lithium anhydre, on a agité le milieu réactionnel pendant 24 heures, puis on l'a de nouveau filtré pour éliminer l'hydrochlorure de DABCO formé. Après évaporation du tétrahydrofurane et séchage, on a récupéré 25,17 g (96% de rendement) du sel de lithium de la trifluorométhanesulfonyl(diméthylaminosulfonyl)imide $Me_2NSO_2NLiSO_2CF_3$ ayant une pureté caractérisée par RMN du fluor et du proton supérieure à 99%. La microanalyse a donné: H 2,34 (2,31) ; Li 2,52 (2,65) ; C 13,96 (13,65) ; N 10,75 (10,69) ; F 21,25 (21,74) ; S 24,35 (24,46).

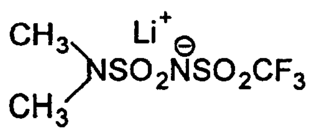

On a préparé suivant le même procédé le sel de lithium de la pentafluoroéthanesulfonyl (diméthylaminosulfonyl)imide (98% de rendement) à partir de la pentafluoroéthanesulfonamide obtenu à l'exemple 5.

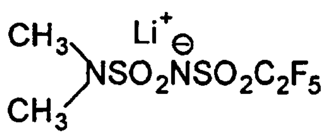

Ces sels présentent une excellente solubilité dans les solvants organiques usuels (tétrahydrofurane, acétonitrile, diméthylformamide, acétate d'éthyle, glymes) et dans les polymères solvatants aprotiques comme le poly(oxyde d'éthylène). Dans ce dernier solvant, à une concentration O/Li = 12/1, le sel de lithium de la trifluorométhanesulfonyl (diméthylaminosulfonyl)imide présente une conductivité ionique de $1,2.10^{-4}$ $S.cm^{-1}$ à 60°C. Les solutions concentrées de ces sels dans l'acétone peuvent être utilisées comme catalyseur des réactions de Diels-Alder.

On a réalisé un générateur lithium-polymère en utilisant une anode en lithium métallique, un électrolyte composé d'un terpolymère d'oxyde d'éthylène, d'allylglycidyléther et de méthylglycidyléther contenant le sel de lithium de la trifluorométhanesulfonyl(diméthylaminosulfonyl)imide à une concentration O/Li = 20/1 et une cathode composite à base d'oxyde de vanadium (40% en volume), de noir de carbone (5% en volume) et d'un électrolyte identique à celui décrit ci-dessus (50% en volume). Ce générateur a donné un profil de cyclage à 60°C équivalent à celui obtenu en utilisant un des sels les plus courant pour cette application la bis(trifluorométhanesulfonyl)imide de lithium (LiTFSI).

**Exemple 26 : Diméthylaminosulfonyl(trifluorométhanesulfonyl)imide**

A une solution à 0°C et sous argon de 14,36 g (100 mmoles) de chlorure de sulfamoyle $(CH_3)_2NSO_2Cl$ (commercialisé par Aldrich) et de 14,91 g de trifluorométhanesulfonamide $CF_3SO_2NH_2$ (100 mmoles) dans 60 ml de tétrahydrofurane anhydre, on a ajouté 22,44 g (200 mmoles) de DABCO en solution dans 20 ml de tétrahydrofurane anhydre à 0°C. Après 2 heures à 0°C, la réaction s'est poursuivie pendant 24 heures à l'ambiante. Le précipité d'hydrochlorure de DABCO a été éliminé par filtration sur un verre fritté de porosité N°4. Après évaporation du tétrahydrofurane et séchage, le produit a été solubilisé dans 25 ml d'éthanol. On a alors ajouté 9,81 g (100 mmoles) d'acétate de potassium $CH_3COOK$, puis on a recristallisé le précipité obtenu au reflux de l'éthanol. Après refroidissement, filtration et séchage, on a récupéré 24,13 g (82% de rendement) du sel de potassium de la trifluorométhanesulfonyl (diméthylaminosulfonyl) imide $Me_2NSO_2NKSO_2CF_3$ ayant une pureté caractérisée par RMN du fluor et du proton supérieure à 99%.

La microanalyse a donné: H 2,21 (2,05) ; C 12,56 (12,24) ; N 9,78 (9,52) ; F 19,89 (19,37) ; S 21,56 (21,79) ; K 13,11 (13,28).

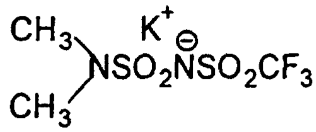

On a obtenu par le même procédé le sel de potassium de la perfluorobutanesulfonyl(diméthylaminosulfonyl) imide (85% de rendement) à partir de la perfluorobutanesulfonamide obtenu à l'exemple 4.

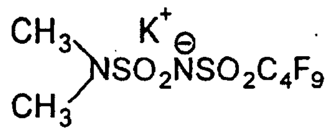

Le sel de potassium de la trifluorométhanesulfonyl-(diméthylaminosulfonyl)imide a un point de fusion de 188°C. Il présente une conductivité dans le poly (oxyde d'éthylène) à une concentration O/K = 12/1 à 60°C de $5.10^{-4}$ $S.cm^{-1}$.

On a également déterminé la conductivité d'un mélange du sel de potassium et du sel de lithium, obtenu à l'exemple 25, dans un rapport K/Li = 2/1 et pour une concentration totale en cation alcalin O/(Li+K) = 14/1 dans le poly (oxyde d'éthylène). Cette conductivité est identique à celle déterminée pour le sel de potassium seul, ce qui indique un mécanisme de transport véhiculaire du lithium dans l'électrolyte complexé par les anions et se déplaçant ainsi sous la forme d'un complexe anionique interagissant peu avec le solvant basique. Ce type de conductivité est très favorable au fonctionnement des générateurs au lithium et plus particuliérement ceux à électrolytes polymères, les performances lors d'appels de puissance en étant améliorées.

**Exemple 27 : Diméthylaminosulfonyl(trifluorométhanesulfonyl)imidede 2,2'-azobis (2-méthylpropionamidine)**

5,89 g (20 mmoles) de sel de potassium de la trifluorométhanesulfonyl (diméthylaminosulfonyl)imide $Me_2NSO_2NKSO_2CF_3$, préparé selon l'exemple 26, ont été mis en solution dans 10 ml d'eau. On a ajouté, sous agitation, 2,71 g d'hydrochlorure de 2,2'-azobis (2-méthylpropionamidine) $[=NC(CH_3)_2C(=NH)NH_2]_2$•2 HCl (10 mmoles, commercialisé par Aldrich) en solution dans 10 ml d'eau. Il s'est formé immédiatement un précipité qui a été recueilli par filtration. Après séchage sous vide à température ambiante, on a récupéré 4,36 g (96% de rendement) de diméthylaminosulfonyl(trifluorométhanesulfonyl)imide de 2,2'-azobis(2-méthylpropionamidine) $[=NC (CH_3)_2C(=NH)NH_2]_2$•2 $Me_2NSO_2NHSO_2CF_3$.

Ce sel est un amorceur de polymérisation radicalaire soluble dans la plupart des solvants organiques usuels (tétrahydrofurane, acétonitrile, diméthylformamide, acétate d'éthyle, glymes) et dans les polymères solvatants aprotiques, contrairement à l'hydrochlorure de 2,2'-azobis(2-méthylpropionamidine).

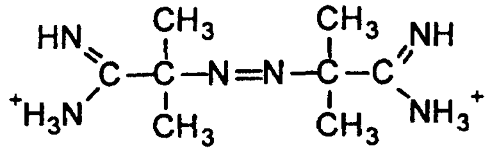 • 2 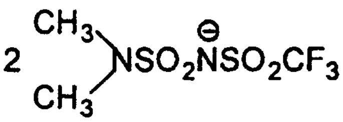

On a préparé une solution dans l'acétonitrile de 1 partie de cet initiateur et de 100 parties d'un polymère contenant des insaturations éthyléniques. Ce polymère a été obtenu par polycondensation de polyéthylène glycol de masse 1000 avec le 3-chloro-2-chlorométhyl-1-propène selon la procédure décrite par Alloin & al. (Solid States Ionics, (1993), 60, 3). On a coulé la solution visqueuse obtenue sur un film de polypropylène (PP). Après évaporation du solvant, le film de polymère d'une épaisseur de 110 µm sur PP a été stocké une semaine en boîte à gants sous argon pour le sécher. La réticulation a alors été initiée en portant la température du film à 60°C. Après une nuit, on a obtenu un film présentant de bonnes propriétés mécaniques et un faible taux d'extractibles (inférieure à 1%). La solubilité de l'initiateur utilisé dans la matrice polymère permet donc d'obtenir une réticulation efficace et homogène. De plus, cet initiateur n'est pas volatil, contrairement par exemple au 2,2'-azobisisobutyronitrile, et la quantité ajoutée peut-être optimisée au mieux pour chaque type de polymérisation.

**Exemple 28 : Dialkylaminosulfonyl(trifluorométhanesulfonyl)imide**

25,85 g (200 mmoles) de dibutylamine $(C_4H_9)_2NH$ en solution dans 50 ml de tétrahydrofurane anhydre ont été traités par 27,83 g (200 mmoles) de trioxyde de soufre complexé par la triméthylamine $(CH_3)_3N{\bullet}SO_3$. Après agitation durant 24 heures à température ambiante, le solvant a été évaporé et le produit repris dans 40 ml de méthanol. Après avoir ajouté 19,63 g (200 mmoles) d'acétate de potassium $CH_3CO_2K$ et recristallisé le précipité obtenu, on a récupéré après filtration et séchage 32,66 g du sel de potassium de l'acide sulfonique de la dibutylamine $(C_4H_9)_2NSO_3K$ (66% de rendement). A 12,37 g de ce sel (50 mmoles) dans 50 ml de tétrahydrofurane à 0°C, on a ajouté lentement 6,35 g (50 mmoles) de chlorure d'oxalyle ClCOCOCl, puis après deux heures à 0°C, 18,72 g (100 mmoles) du sel de potassium de la trifluorométhanesulfonamide $CF_3SO_2NHK$. La réaction s'est poursuivie pendant 48 heures à température ambiante, puis le solvant a été évaporé et le produit obtenu recristallisé dans 50 ml d'eau. Après filtration et séchage, on a récupéré 14,38 g du sel de potassium de la trifluorométhanesulfonyl (dibutylaminosulfonyl)imide $(C_4H_9)_2NSO_2NKSO_2CF_3$ (76% de rendement) ayant une pureté caractérisée par RMN du proton et du fluor supérieure à 99%.

La microanalyse a donné: H 4,65 (4,79) ; C 28,23 (28, 56) ; N 7,1 (7, 4) ; F 15,52 (15,06) ; S 16,45 (16,94) ; K 10,52 (10,33).

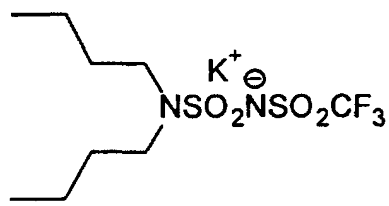

On a préparé suivant un procédé similaire les sels de potassium des amides portant des substituants diéthyle $(C_2H_5)_2NSO_2NKSO_2CF_3$ (66% de rendement) à partir de la diéthylamine, et la di-2-éthylhexyle $[C_4H_9\text{-}CH(C_2H_5)\text{-}CH_2]_2NSO_2\text{-}NKSO_2CF_3$ (70% de rendement) à partir de la di-2-éthylhexylamine, avec des puretés caractérisée par RMN du proton et du fluor supérieure à 98%.

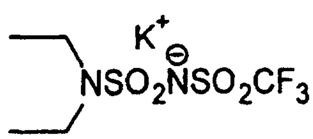

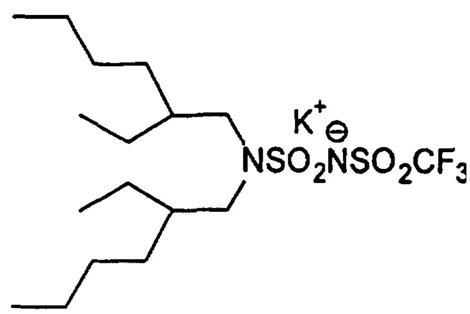

On a préparé par un procédé similaire les sels de potassium des fluorosulfonyl(dialkylaminosulfonyl)imides, à partir de la fluorosulfonamide obtenu à l'exemple 2, les sels de potassium des pentafluoroéthanesulfonyl(dialkylaminosulfonyl)-imides à partir de la pentafluoroéthanesulfonamide obtenu à l'exemple 5 et les sels de potassium des perfluorobutanesulfonyl (dialkylaminosulfonyl) imides $(C_4H_9)_2NSO_2NKSO_2C_4F_9$ à partir de la perfluorobutanesulfonamide obtenu à l'exemple 4.

Par échange ionique dans l'acétone entre le sel de potassium de la trifluorométhanesulfonyl(di-2-éthylhexylaminosulfonyl)imide avec un colorant infrarouge de la famille des cyanines, l'iodure de 3,3'-diéthylthiatricarbocyanine (commercialisé par Aldrich) suivi d'une reprécipitation dans l'eau, on a pu obtenir après filtration et séchage la 3,3'-diéthylthiatricarbocyanine de di-2-éthylhexylaminosulfonyl(trifluorométhanesulfonyl)imide.

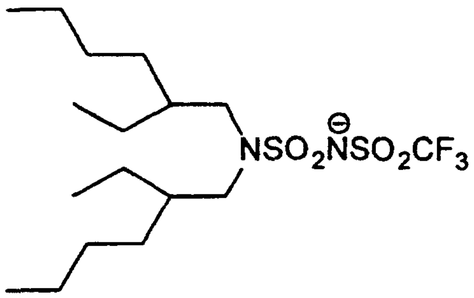 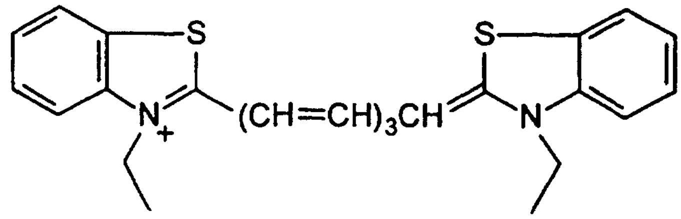

Ce sel est très soluble dans les solvants peu polaires comme le dichlorométhane ou le chlorure de méthylène ainsi que dans les matrices polymères peu polaires comme le polyméthacrylate de méthyle.

On a également constaté une très nette diminution de l'agrégation des colorants cationiques entre eux du fait du caractère "plastifiant" des groupements di-2-éthylhexylamino, ce qui est un avantage dans la mesure où le phénomène d'agrégation amène un élargissement des bandes d'absorption optiques préjudiciable à la précision de fonctionnement des systèmes utilisant ses colorants en particulier les disques optiques de stockage d'informations.

**Exemple 29 : Diméthylaminosulfonyl (trifluorométhanesulfonyl) imide imidazolium**

14,71 g (50 mmoles) du sel de potassium de la trifluorométhanesulfonyl(diméthylaminosulfonyl)imide $(CH_3)_2NSO_2NKSO_2CF_3$ préparé selon l'exemple 26, ont été cobroyés dans un mortier en agate en boîte à gants avec 17,27 g (150 mmoles) d'hydrogénosulfate d'ammonium $HSO_4NH_4$ (commercialisé par Aldrich). Par sublimation sous vide secondaire à 80°C, on a récupéré sur un doigt froid après 24 heures, 11,2 g (87% de rendement) de trifluorométhanesulfonyl(diméthylaminosulfonyl)-imide $(CH_3)_2NSO_2NHSO_2CF_3$ ayant une pureté caractérisée par RMN du fluor et du proton supérieure à 99%.

A une solution dans 15 ml d'éther de 1,36 gr d'imidazole (20 mmoles), on a ajouté 5,12 g de cet acide (20 mmoles), puis après 24 heures sous agitation, on a récupéré le précipité formé par filtration sur un verre fritté de porosité N°3. Après séchage, on a récupéré quantitativement le sel d'imidazolium de la trifluorométhanesulfonyl(diméthylaminosulfonyl)imide.

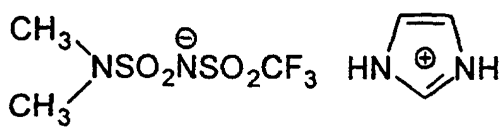

Le broyage en boîte à gants d'un mélange molaire de 7 imidazoles pour deux sels d'imidazolium a permis d'obtenir un composé d'une température de fusion inférieure à l'ambiante. Ce sel fondu présente une conductivité protonique élevée supérieure à $10^{-3}$ $S.cm^{-1}$ à 60°C. Il est possible d'obtenir un électrolyte polymère, conducteur protonique anhydre, en ajoutant du poly (oxyde d'éthylène), préférentiellement de haute masse ou pouvant être ultérieurement réticulé, au sel fondu sans que cela soit préjudiciable à la conductivité. Ces électrolytes polymères sont particulièrement intéressants pour la réalisation de systèmes de modulation de la lumière tels que les vitrages électrochromes incluant les systèmes électrochromes à colorants.

On a obtenu une membrane optiquement transparente dans le visible et ayant une bonne tenue mécanique en utilisant un électrolyte polymère composé de 80% en poids de sel fondu et de 20% en poids de poly (oxyde d'éthylène) de masse $M_w = 5.10^6$. On a ensuite réalisé un système électrochrome en boîte à gants en utilisant cet électrolyte enfermé entre une première électrode constituée par le dépôt sur une plaque de verre d'une couche d'oxyde d'iridium hydrogéné $H_xIrO_2$ et d'une sous-couche conductrice d'oxyde d'étain et une seconde électrode constituée d'une couche de trioxyde de tungstène $WO_3$ et d'une sous-couche conductrice d'oxyde d'étain. Cet électrochrome a permis une variation de l'absorption optique de 80% (état décoloré) à 30% (état coloré) et de bonnes performances en cyclage (plus de 20 000 cycles de coloration/décoloration)

On a également réalisé un électrochrome en dissolvant deux colorants complémentaires dans un tel sel fondu : en boîte à gants, on a co-broyé 1,62 g (5 mmoles) du sel d'imidazolium de la trifluorométhanesulfonyl (diméthylaminosulfonyl)imide avec 1,02 g d'imidazole (15 mmoles). Puis on a ajouté au sel fondu 16,5 mg (50 µmoles) de leucomalachite verte (état réduit incolore) et 29,5 mg (50 µmoles) du sel de 3-(4,5-diméthylthiazolyl-2-yl)-2,5-diphényl-2H-tétrazolium (MTT) de trifluorométhanesulfonyl(diméthylaminosulfonyl)imide (état oxydé incolore, (obtenu par

échange ionique dans l'eau à partir du bromure). On a alors ajouté 5% en poids de poly (oxyde d'éthylène) de masse $M_w$= $3.10^5$. Le gel obtenu a été déposé entre deux plaques de verre recouvertes d'une couche conductrice d'oxyde d'étain ($SnO_2$). Après pressage sous vide pour homogénéiser le dépôt et scellage pour le rendre étanche, on a obtenu un système électrochrome à colorants. Après avoir scellé l'ensemble pour le rendre étanche, on a appliqué à l'extérieur un potentiel de 1300 mV à l'aide d'un potentiostat. Le système s'est alors coloré, la forme oxydé de la malachite verte et la forme réduite du MTT présentant chacune une bande d'absportion intense dans le visible. En appliquant un potentiel de -500 mV, on a observé une décoloration relativement rapide du système (inférieur à 60 s). Un tel système électrochrome est facile à mettre en oeuvre, même pour des systèmes de grande taille (supérieure au $m^2$) qui utilisent aussi bien du verre qu'un polymère convenablement traité comme électrode transparente conductrice. De plus, l'énergie nécessaire pour conserver la coloration est relativement faible, inférieure à 1 $W/m^2$.

**Exemple 30 : Diméthylaminosulfonyl(trifluorométhanesulfonyl)imide**

A 2,56 g de trifluorométhanesulfonyl(diméthylaminosulfonyl)imide $(CH_3)_2NSO_2NHSO_2CF_3$ (10 mmoles), obtenue comme à l'exemple 29, en solution dans 10 ml d'eau, on a ajouté 763 mg (1,67 mmoles) de carbonate de lanthane $La_2(CO_3)_2$ anhydre. Après une nuit sous agitation, l'eau a été évaporée et on a récupéré quantitativement après séchage le sel de lanthane de la trifluorométhanesulfonyl(diméthylaminosulfonyl)imide $[(CH_3)_2NSO_2NSO_2CF_3]_3La$.

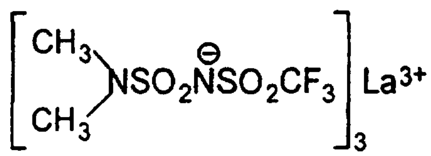

Ce sel est très soluble dans les solvants organiques usuels (tétrahydrofurane, acétonitrile, diméthylformamide, acétate d'éthyle, glymes). Il présente une interaction avec les solvants peu polaires tels que le dichlorométhane meilleure que celle du sel de lanthane de la bis(trifluorométhanesulfonyl)imide. Il peut être utilisé comme catalyseur dans les réactions de Diels-Alder.

**Exemple 31: Dialkylaminosulfonyl(trifluorométhanesulfonyl)imide**

A 3,78 g (10 mmoles) du sel de potassium de la trifluorométhanesulfonyl (diméthylaminosulfonyl) imide $(CH_3)_2NSO_2NKSO_2CF_3$, obtenu à l'exemple 26, en solution dans 10 ml de nitrométhane anhydre, on a ajouté en boîte à gants 1,17 g de tétra-fluoroborate de nitrosonium $NOBF_4$ (10 mmoles, commercialisé par Aldrich). Après une heure, le milieu réactionnel a été filtré pour éliminer le tétrafluoroborate de potassium insoluble, et on a ainsi obtenu une solution 1 M de la trifluorométhanesulfonyl (diméthylaminosulfonyl) imide $(CH_3)_2NSO_2N$ (NO) $SO_2CF_3$ dans le nitrométhane.

Par un procédé similaire, on a préparé une solution 1 M dans le nitrométhane du sel de nitrosonium de la trifluorométhanesulfonyl(dibutylaminosulfonyl)imide $(C_4H_9)_2NSO_2N(NO)SO_2$-$CF_3$, à partir du sel de potassium de la trifluorométhanesulfonyl(dibutylaminosulfonyl)imide (obtenu à l'exemple 28) et du sel de nitrosonium de la trifluorométhanesulfonyl-(N,N-di-2-éthylhexylaminosulfonyl)imide $(C_4H_9CH(C_2H_5)CH_2)_2N$-$(NO)SO_2CF_3$, à partir du sel de potassium de la trifluorométhanesulfonyl(N,N-di-2-éthylhexylaminosulfonyl)imide (obtenu à l'exemple 28).

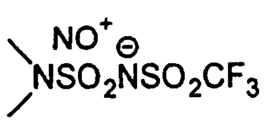
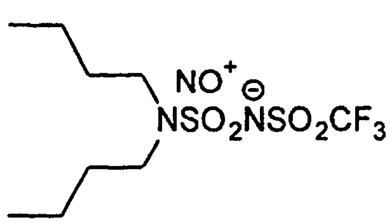
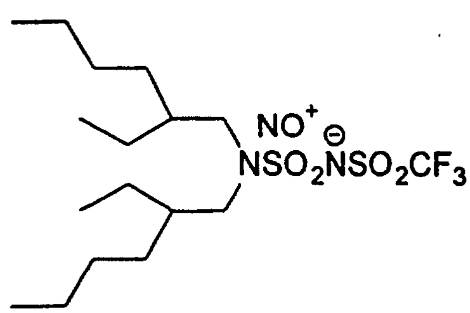

Ces sels sont particulièrement intéressants pour le dopage de polymères conjugués (polythiophène, polypyrrole) auxquels ils confèrent une conductivité électronique appréciable.

On a réalisé trois dépôts de poly(3-hexylthiophène) stéréorégulier (commercialisé par Aldrich) sur des plaques de verre à partir d'une solution dans le chloroforme. Après séchage, ces dépôts ont été dopés par un des sels en solution dans le nitrométhane. Après dopage, les trois films de poly(3-hexylthiophène) présentaient une conductivité électronique supérieure à 1 $S.cm^{-1}$ indépendamment du sel dopant.La stabilité en milieu humide de la conductivité est améliorée avec l'augmentation de la longueur des segments alkyles. Ces dépôts sont utiles pour la réalisation de masques pour l'industrie des semi-conducteurs.

**Exemple 32 : Dialkylaminosulfonyl(trifluorométhanesulfonyl)imide**

5,96 g (40 mmoles) de trifluorométhanesulfonamide $CF_3SO_2NH_2$ et 9,9 ml de pyridine dans 60 ml de dichlorométhane anhydre ont été refroidis à -20°C. On a alors ajouté goutte à goutte 5,4 g (40 mmoles) de chlorure de sulfuryle $SO_2Cl_2$ dilués dans 10 ml de dichlorométhane anhydre, puis 8,1 g (80 mmoles) de dipropylamine $(C_3H_7)_2NH$. Le mélange a été agité pendant 1 heure à -20°C puis pendant 24 heures à température ambiante. Le milieu réactionnel a ensuite été filtré, puis le solvant évaporé. Le produit récupéré a été repris dans 50 ml d'eau, acidifié à un pH ≈ 2 par une solution d'acide chlorhydrique 4 M, la phase aqueuse extraite à deux reprises par 20 ml d'éther, la phase organique séchée par du sulfate de magnésium, puis l'éther évaporé. Après sublimation du composé obtenu sous vide secondaire à 40°C, on a récupéré 11 g de trifluorométhanesulfonyl(dipropylaminosulfonyl)imide (88% de rendement) ayant une pureté caractérisée par RMN du fluor et du proton supérieure à 98%.

On a préparé le sel de lithium en dosant par pH-métrie l'acide en solution dans l'eau par une solution titrée d'hydroxyde de lithium. Après évaporation de l'eau et séchage sous vide à 60°C pendant 24 heures, on a récupéré quantitativement sous forme d'une poudre blanche le sel de lithium de la trifluorométhanesulfonyl(dipropylaminosulfonyl)-imide $(C_3H_7)_2NSO_2NLiSO_2CF_3$.

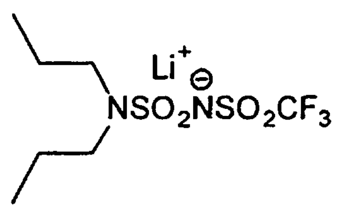

La microanalyse a donné: H 4,33 (4,43) ; Li 2,01 (2,18) ; C 26,59 (26,42) ; N 8,69 (8,8) ; F 17,33 (17,91) ; S 20,46 (20,15).

On a préparé suivant le même procédé le sel de lithium de la trifluorométhanesulfonyl(N-méthyl-N-éthylaminosulfonyl)-imide $CH_3(C_2H_5)NSO_2NLiSO_2CF_3$ ayant une pureté déterminée par RMN du proton et du fluor supérieure à 99% avec un rendement de 76%.

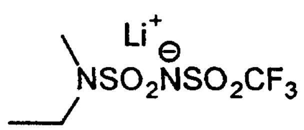

Ces sels sont solubles dans la plupart des solvants organiques usuels (tétrahydrofurane, acétonitrile, diméthylformamide, acétate d'éthyle, glymes) et dans les polymères solvatants aprotiques comme le poly (oxyde d'éthylène).

Le sel de lithium la trifluorométhanesulfonyl(dipropylaminosulfonyl)imide a une conductivité de $1,4.10^{-4}$ $S.cm^{-1}$ dans le poly(oxyde d'éthylène) à une concentration O/Li = 12/1 et le nombre de transport cationique est de 0,42.

**Exemple 33 : 3-(trifluorométhyl)phényl(trifluorométhanesulfonyl)amide**

A 48,34 g de 3-(trifluorométhyl)aniline (300 mmoles) dans 250 ml de dichlorométhane anhydre à 0°C, on a ajouté goutte à goutte pendant 2 heures, 28,21 g d'anhydride trifluorométhanesulfonique $(CF_3SO_2)_2O$ (100 mmoles) dilués dans 100 ml de dichlorométhane anhydre, puis la réaction s'est poursuivie pendant 24 heures à l'ambiante. Après évaporation du dichlorométhane, le produit obtenu a été repris dans 300 ml d'eau, puis acidifié par 25 ml d'une

solution 4 M d'acide chlorhydrique. La solution aqueuse a alors été extraite par trois fractions de 50 ml d'éther, les phases organiques ont été réunies et séchées avec du sulfate de magnésium. Après évaporation de l'éther et séchage, le produit obtenu a été purifié par sublimation sous vide secondaire à 40°C. On a récupéré après 24 heures sur un doigt froid 25 g de trifluorométhanesulfonyl(3-(trifluorométhyl)phényl)amide m-$CF_3C_6H_4NHSO_2CF_3$ (85% de rendement) sous forme d'un solide cristallisé blanc ayant une pureté caractérisée par RMN du fluor et du proton supérieure à 99%.

La microanalyse a donné H 1,65 (1,72) ; C 32,53 (32,77) ; N 4,62 (4,78) ; F 38,12 (38,88) ; S 10,72 (10,94).

On a préparé le sel de lithium en traitant l'acide obtenu par le phosphate de lithium $Li_3PO_4$ pendant 48 heures dans l'acétonitrile. Après filtration du milieu réactionnel, évaporation du solvant et séchage sous vide à 60°C pendant 24 heures, on a obtenu quantitativement le sel de lithium de la trifluorométhanesulfonyl (3-(trifluorométhyl)phényl) amide m-$CF_3C_6H_4NLiSO_2CF_3$.

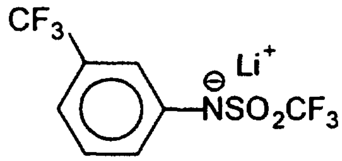

Les sels de sodium et de potassium ont été obtenus par un procédé similaire, en remplaçant le phosphate de lithium respectivement par le phosphate de sodium et de potassium.

De la même manière, on a préparé également la trifluorométhanesulfonyl(3-5-bis(trifluorométhyl)phényl)amide (I) à partir de la 3-5-bis(trifluorométhyl)aniline, la trifluorométhanesulfonyl(4-trifluorométhoxy)phényl)amide (II) à partir de la trifluorométhanesulfonyl(4-trifluorométhoxy)aniline, la trifluorométhanesulfonyl (4-aminopyridine)amide (III) à partir de la 4-aminopyridine et la trifluorométhanesulfonyl-(2,2,2-trifluoroéthyl)amide (IV) à partir de la 2,2,2-trifluoroéthylamine ainsi que les sels de lithium, sodium, potassium correspondant.

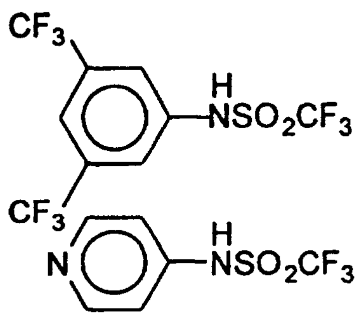
(I)

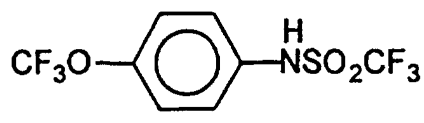
(II)

(III)

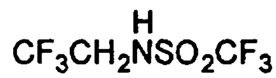
(IV)

Pour tous ces sels, on a également obtenus les dérivés du type fluorosulfonyle en utilisant l'anhydride fluorosulfonique $(FSO_2)_2O$ (commercialisé par SST corporation) à la place de l'anhydride trifluorométhanesulfonique

Ces sels sont solubles dans la plupart des solvants organiques usuels (tétrahydrofurane, acétonitrile, diméthylformamide, acétate d'éthyle, glymes) et dans les polymères solvatants aprotiques comme le poly(oxyde d'éthylène). Ils présentent un potentiel d'oxydation supérieur à 4 V vis à vis d'une anode de lithium. Les sels de lithium ou de potassium, ou leurs mélanges, peuvent donc être utilisés pour l'élaboration d'électrolytes (liquides, gels ou polymères) pour les batteries au lithium utilisant un matériau de cathode ayantun potentiel de fin de recharge inférieur à 4 V ($TiS_2$, $V_2O_5$, $Fe(PO_4)_2$). Les sels d'étain (II) peuvent être utilisés pour la catalyse des condensations aldoliques.

**Exemple 34 : Trifluoro-méthane-sulfonyl (2-trifluorométhyl-1,3,4-thiadiazole-5-amino)amide.**

En opérant dans une boîte à gants sous argon, à 16,91 g (100 mmoles) de 2-amino-5-trifluorométhyl-1,3,4-thiadiazole (commercialisé par Aldrich) en solution dans 100 ml de tétrahydrofurane anhydre à -30°C, on a ajouté goutte à goutte 100 ml d'une solution 1 M de dibutylmagnésium $(C_4H_9)_2Mg$ (100 mmoles, commercialisé par Aldrich) dans l'heptane. Après 4 heures à -30°C, on a ajouté lentement 16,85 g (100 mmoles) de chlorure de trifluorométhanesulfonyle $CF_3SO_2Cl$. La réaction s'est poursuivie pendant 2 heures à -30°C, puis pendant 24 heures à l'ambiante. Les solvants ont alors été évaporés, le produit a été repris dans l'eau et extrait par de l'éther après acidification de la solution aqueuse. Le composé obtenu après évaporation de l'éther a été sublimé sous vide secondaire à 40°C, et on

a ainsi récupéré après 24 heures sur un doigt froid, 25,73 g de trifluorométhanesulfonyl-(5-trifluorométhyl-1,3,4-thiadiazole)amide (86% de rendement).

La micronalyse a donné: H 0,39 (0,33) ; C 15,29 (15,95) ; N 13,28 (13,95) ; F 38,3 (37,85) ; S 20,62 (21,29).

Le sel de lithium a été obtenu en traitant l'acide par le carbonate de lithium $Li_2CO_3$ dans l'eau.

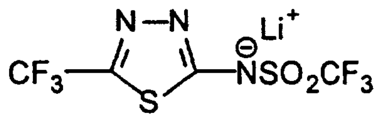

Ce sel est soluble dans la plupart des solvants organiques usuels (tétrahydrofurane, acétonitrile, diméthylformamide, acétate d'éthyle, glymes) et dans les polymères solvatants aprotiques comme le poly(oxyde d'éthylène).

Ce sel a un potentiel d'oxydation dans un mélange de carbonate d'éthylène et de diméthylcarbonate (2:1), à une concentration de 1 M, supérieur à 4 V vis à vis d'une anode de lithium.

**Exemple 35 : Trifluoro-méthane-sulfonyl (2-trifluorométhylthiadiazole-5-aminosulfonyl)imide.**

Dans un premier temps, on a préparé le chlorure de 5-trifluorométhyl-1,3,4-thiadiazole-2-sulfonyle à partir du 2-amino-5-trifluorométhyl-1,3,4-thiadiazole (commercialisé par Aldrich), suivant la procédure décrite dans l'exemple 7.

Puis, par une procédure similaire à celle utilisée dans l'exemple 25 pour la synthèse du sel de lithium de la diméthylaminosulfonyl(trifluorométhanesulfonyl)imide, on a synthétisé le sel de lithium de la trifluorométhanesulfonyl-(5-trifluorométhyl-1,3,4-thiadiazole-5-sulfonyl)imide. Le produit obtenu a une pureté déterminée par RMN du proton et du fluor supérieur à 98%.

La micronalyse a donné: Li 1,36 (1,9) ; C 13,29 (12,9) ; N 11,88 (11,3) ; F 31,4 (30,7) ; S 26,46 (25,9).

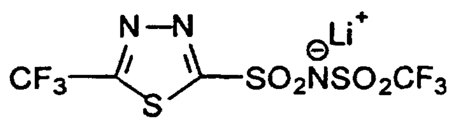

Ce sel est soluble dans la plupart des solvants organiques usuels (tétrahydrofurane, acétonitrile, diméthylformamide, acétate d'éthyle, glymes) et dans les polymères solvatants aprotiques comme le pdly (oxyde d'éthylène).

Ce sel a un potentiel d'oxydation, à une concentration 0,5 M dans l'acétonitrile, supérieure à 4,5 V vis à vis d'une anode de lithium. Il peut être utilisé pour les batteries Li-Ion à électrolytes liquides ou gels. Ainsi, on a assemblé une pile en utilisant une anode constituée par un coke de carbone (80% en volume) mélangé avec du polyfluorure de vinylidène (PVDF, commercialisé par la société Montedison) comme liant (20% en volume), un électrolyte composé d'un mélange de carbonate d'éthylène et de diméthylcarbonate (2:1), gélifié par du PVDF, contenant ce sel à une concentration 1 M et une cathode composite constituée par du noir de carbone (6% en volume), $Li_2MnO_4$ (75% en volume) et de PVDF comme liant (20% en volume). Ce générateur a donné de bonnes performances en cyclage à 25°C (1 000 cycles de charge/décharge entre 2 et 4,7 V en conservant environ 50% de la capacité au premier cycle).

**Exemple 36 : Trifluoro-méthane-sulfonyl(2-trifluorométhylthiadiazole-5-aminosulfonyl)amide.**

Dans 20 ml d'acétonitrile anhydre sous agitation, on a traité 2,29 g (10 mmoles) de trichloromélamine (commercialisé par Fluka) par 5,16 g de triflinate de potassium en présence de 6,37 g de phosphate de potassium $K_3PO_4$ (30 mmoles). Après 72 heures sous agitation, le solvant a été évaporé et le résidu recristallisé dans 40 ml d'eau. Après filtration et séchage, on a récupéré 10,69 g (56% de rendement) du trisel de potassium de la tris-[1,3,5-trifluorométhanesulfonamide]-2,4,6-triazine ayant une pureté déterminée par RMN du proton et du fluor supérieure à 99%.

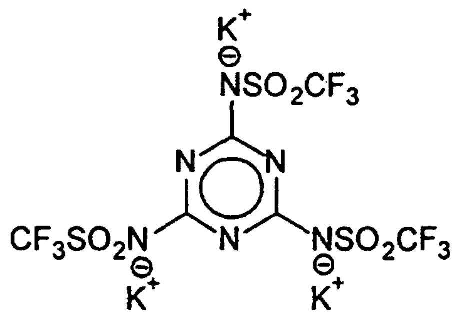

La microanalyse a donné: C 11,52 (11,32) ; N 13,61 (13,2) ; F 26,99 (26,86) ; S 15,01 (15,11) ; K 18,21 (18,43).

On a obtenu le trisel de lithium par échange ionique avec le chlorure de litihum LiCl dans le tétrahydrofurane.

Le sel de lithium est soluble dans la plupart des solvants organiques usuels (tétrahydrofurane, acétonitrile, diméthylformamide, acétate d'éthyle, glymes) et dans les polymères solvatants aprotiques comme le poly (oxyde d'éthylène). Dans ce dernier solvant contenant le sel de lithium à une concentration O/Li = 12/1, le nombre de transport cationique est de 0,52 à 60°C.

On a obtenu le tri-sel de tétrabutylammonium par traitement du tri-sel de lithium par le chlorure de tétrabutylammonium (5% en excès) dans l'eau. Le précipité obtenu a ensuite été récupéré par extraction au dichlorométhane, la solution de dichlorométhane a été lavée par de l'eau, puis évaporée. On a récupéré quantitativement le tri-sel de tritétrabutylammonium de la tris-[1,3,5-trifluorométhanesulfonamide]-2,4,6-triazine. L'addition de 3,5% en poids de ce composé à un copolymère poly(acrylonitrile-co-butadiène) contenant ≈ 20% en poids d'acrylonitrile confère au copolymère des propriétés antistatiques.

**Exemple 37 : Trifluorométhanesulfonyl(1,1,1,3,3,3-hexafluoro-2-propanoxysulfonyl)imide**

5,96 g (40 mmoles) de trifluorométhanesulfonamide $CF_3SO_2NH_2$ et 9,9 ml de pyridine dans 60 ml de dichlorométhane anhydre, ont été refroidis à -15°C. On a alors ajouté goutte à goutte 5,4 g (40 mmoles) de chlorure de sulfuryle $SO_2Cl_2$ dilués dans 10 ml de dichlorométhane anhydre, puis 6,72 g (40 mmoles) de 1,1,1,3,3,3-hexafluoro-2-propanol $(CF_3)_2CHOH$. Le mélange a été agité pendant 1 heure à -15°C, puis pendant 12 heures à température ambiante. Le milieu réactionnel a ensuite été filtré, puis le solvant évaporé. Le produit récupéré a été repris dans 50 ml d'eau, acidifié par 10 ml d'une solution d'acide chlorhydrique 4 M ; la phase aqueuse a été extraite à deux reprises par 20 ml d'éther, la phase organique a été séchée par du sulfate de magnésium et l'éther a été évaporé à l'aide d'un évaporateur rotatif. Après sublimation sous vide secondaire à 40°C du composé obtenu, on a récupéré 13,9 g de trifluorométhanesulfonyl(1,1,1,3,3,3-hexafluoro-2-propanoxysulfonyl)imide (92% de rendement) ayant une pureté caractérisée par RMN du fluor et du proton supérieure à 98%.

La microanalyse a donné: H 0,46 (0,53) ; C 12,35 (12,67) ; N 3,76 (3,69) ; F 44,3 (45,09) ; S 16,23 (16,91).

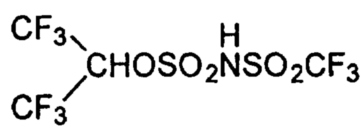

On a obtenu une solution aqueuse du sel de lithium en traitant l'acide par le carbonate de lithium $Li_2CO_3$ dans l'eau. Ensuite, par addition de chlorure de 1-éthyl-3-méthyl-1H-imidazolium (10% en excès, commercialisé par Aldrich), on a obtenu une phase liquide plus dense que l'eau. Cette phase a été récupérée par extraction au dichlorométhane. Après évaporation du dichlorométhane et séchage sous vide à 40°C du liquide obtenu, on a récupéré le sel fondu de 1-éthyl-3-méthyl-1H-imidazolium de trifluorométhanesulfonyl-(1,1,1,3,3,3-hexafluoro-2-propanoxysulfonyl)imide (91% de rendement).

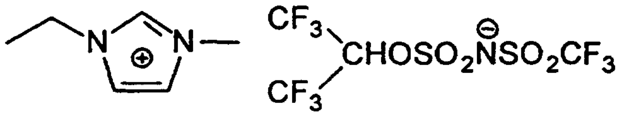

Ce sel fondu présente une conductivité de $3{,}91.10^{-3}$ $S.cm^{-1}$ et a un point de congélation inférieur à -20°C. Son large domaine de stabilité rédox en fait un électrolyte particulièrement intéressant pour les générateurs électrochimiques tels que les batteries au lithium, les supercapacités, les systèmes de modulation de la lumière et les cellules photovoltaïques.

On a réalisé une cellule photovoltaïque électrochimique en assemblant un système composé de deux électrodes séparées par un espace vide d'une épaisseur de 30 µm. La première électrode a été revêtue d'une couche nanoparticulaire de dioxyde de titane $TiO_2$ de 0,25 µm d'épaisseur sur laquelle a été adsorbée la 1,3-phénylsulfonamide-N,N'-trifluorométhanesulfonyle rhodamine B obtenue à l'exemple 55 comme sensibilisateur. L'espace entre les électrodes a été rempli avec un électrolyte composé du sel fondu dans lequel avait été solubilisé 10% en poids d'iodure de méthylhexyl imidazolium et 10 mmoles d'iode. Le courant de court-circuit de cette cellule est de 103 $\mu A.cm^{-2}$ et sa tension en circuit ouvert de 552 mV.

**Exemple 38 : Cyano(perfluorobutanesulfonyl)imide**

A 5,16 g (60 mmoles) du disel de sodium de la cyanamide (commercialisé par Aldrich) dans 30 ml de diméthoxyéthane à 0°C, on a ajouté 15,1 g de fluorure de perfluorobutanesulfonyle (50 mmoles) à 0°C. Après 3 heures à 0°C, puis 24 heures à l'ambiante, le milieu réactionnel a été centrifugé et filtré pour éliminer l'excès de cyanamide et le fluorure de sodium formé. Le produit obtenu a été repris dans 20 ml de méthanol après évaporation du diméthoxyéthane, puis l'on a ajouté 4,91 g d'acétate de potassium $CH_3COOK$ (50 mmoles) anhydre. Le précipité qui s'est formé a été recristallisé, puis récupéré par filtration. Après séchage, on a obtenu 12,5 g du sel de potassium de la perfluorobutanesulfonyl (cyano)imide $C_4F_9SO_2NKCN$ (69% de rendement) sous forme d'une poudre blanche ayant une pureté caractérisée par RMN du fluor et du proton supérieure à 97%.

La microanalyse a donné: C 16,18 (16,58) ; N 7,23 (7,73) ; F 47,98 (47,21) ; S 8,12 (8,85) ; K 11,2 (10,79).

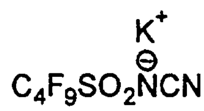

De la même manière, on a préparé les sels de potassium de la cyano(fluorosulfonyl)imide (I) à partir du chlorure de fluorosulfonyle $ClSO_2F$, de la cyano(trifluorométhanesulfonyl)-imide (II) à partir du chlorure de trifluorométhanesulfonyle $CF_3SO_2Cl$ et de la cyano(pentafluoroéthanesulfonyl)imide (III) à partir de chlorure de pentafluoroéthanesulfonyle $C_2F_5SO_2Cl$.

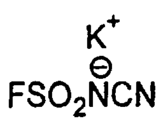
(I)

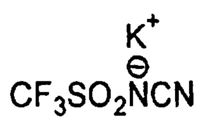
(II)

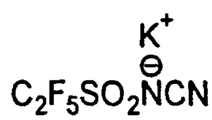
(III)

Les sels de lithium ont été préparés quantitativement par échange ionique entre le sel de potassium et le chlorure de lithium dans le tétrahydrofurane anhydre.

Ces sels sont solubles dans la plupart des solvants organiques usuels (tétrahydrofurane, acétonitrile, diméthylformamide, acétate d'éthyle, glymes) et dans les polymères solvatants aprotiques comme le poly (oxyde d'éthylène). Dans ce dernier solvant, il présente une conductivité supérieure à $2.10^{-4}$ $S.cm^{-1}$ à 60°C pour une concentration O/Li = 12/1.

On a réalisé une supercapacité électrochimique en utilisant une solution 1 M de chacun des sels de potassium ci-dessus dans l'acétonitrile comme électrolytes et des composites carbone /aluminium comme électrodes. Pour chaque supercapacité, les électrodes d'une épaisseur de 150 µm ont été placées de part et d'autre d'un polyéthylène microporeux d'une épaisseur de 40 µm imprégné par et le système complet scellé en boîte à gants dans un boitier de pile bouton. On a obtenu de bonnes performances avec ces supercapacités (plus de 100 000 cycles de charge /décharge entre 0 et 2,5 V pour une densité d'énergie supérieure à 25 Wh/l et une puissance délivrée supérieure à 1500 W/l).

**Exemple 39 : Alkylsulfonyl(trifluorométhanesulfonyl)imide**

7,83 g (50 mmoles) de chlorure de butanesulfonyle $C_4H_9SO_2Cl$ en solution dans 30 ml de tétrahydrofurane anhydre à 0°C ont été traités par 17,11 g (100 mmoles) du sel de sodium de la trifluorométhanesulfonamide $CF_3SO_2NHNa$. Après 1 heure à 0°C, puis 24 heures à température ambiante, le solvant a été évaporé et le produit repris dans 50 ml d'eau. L'addition de 3,73 g de chlorure de potassium KCl (50 mmoles) anhydre a entraîné l'apparition d'un précipité qui a été recristallisé, puis récupéré par filtration et enfin séché. 9,37 g du sel de potassium de la trifluorométhanesulfonyl(butanesulfonyl)imide (61% de rendement) $C_4H_9SO_2NKSO_2CF_3$ ont ainsi été obtenus sous forme d'une poudre blanche cristallisé, ayant une pureté déterminée par RMN du fluor et du proton supérieure à 99%.

La microanalyse a donné: H 2,75 (2.95) ; C 19,01 (19,54) ; N 4,98 (4,56) ; F 18,21 (18,54) ; S 20,25 (20,86) ; K 12,56 (12,72).

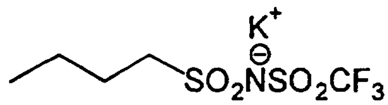

Les sels de potassium de la trifluorométhanesulfonyl-(octylsulfonyl)imide (I) et de la trifluorométhanesulfonyl (dodécylsulfonyl)imide (II) ont été obtenus dans des conditions identiques à partir respectivement du chlorure d'octylsulfonyle et du chlorure de dodécylsulfonyle.

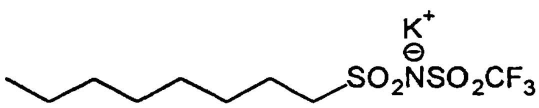
**(I)**

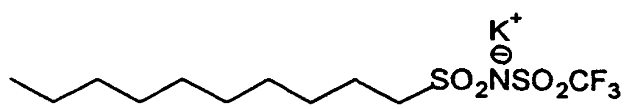
**(II)**

Les sels de lithium de ces trois dérivés ont été préparés quantitativement par échange ionique entre le sel de potassium et le chlorure de lithium dans le tétrahydrofurane anhydre. Le sel de lithium de la trifluorométhanesulfonyl (dodécylsulfonyl)imide dissous dans une matrice de poly (oxyde d'éthylène) à une concentration O/Li = 16/1 a un nombre de transport cationique d'environ 0,55. Il en résulte que, lorsque ce composé est utilisé dans l'électrolyte d'une batterie au lithium à électrolyte polymère, les gradients de concentration qui apparaissent au cours du fonctionnement de la batterie sont diminués de façon substantielle. Les performances lors d'appels de puissance sont ainsi améliorées.

**Exemple 40 : Octylsulfonyl(fluorosulfonyl)imide**

5,16 g (25 mmoles) de chlorure d'octylsulfonyle $C_8H_{17}SO_2Cl$ en solution dans 20 ml de tétrahydrofurane anhydre à 0°C ont été traités par 6,86 g (50 mmoles) du sel de potassium de la fluorosulfonamide. Après 1 heure à 0°C et 24 heures à température ambiante, le solvant a été évaporé et le produit recristallisé dans 15 ml d'eau. Après filtration et séchage, on a récupéré 5,64 g du sel de potassium de la fluorosulfonyl(butanesulfonyl) imide $C_8H_{17}SO_2NKSO_2F$ (72% de rendement) ayant une pureté déterminée par RMN du fluor et du proton supérieure à 99%. On a préparé le sel de lithium par échange ionique (métathèse) entre le sel de potassium et le chlorure de lithium dans le tétrahydrofurane anhydre.

La microanalyse a donné: H 5,27 (5,47) ; C 30,98 (30,66) ; N 4,78 (4,47) ; F 6,52 (6,06) ; S 20,96 (20,46) ; K 12,01 (12,47).

Le sel de lanthane de la fluorosulfonyl(butanesulfonyl)imide $C_8H_{17}SO_2NKSO_2F$ peut être utilisé comme catalyseur des réactions de Diels-Alder, dans le dichlorométhane en particulier.

Ces sels possèdent des propriétés plastifiantes.

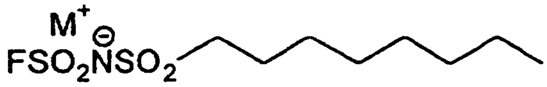
**M = Li , K**

**Exemple 41 : Triisopropylsulfonyl(fluorosulfonyl)imide**

3,96 g de fluorosulfonamide $FSO_2NH_2$ (40 mmoles) et 12,11 g de chlorure de 2,4,6-triisopropylbenzènesulfonyle (40 mmoles, commercialisé par Aldrich), ont été agités dans 40 ml de tétrahydrofurane anhydre à 0°C en présence de 8,49 gr de phosphate de potassium $K_3PO_4$ anhydre. Après 3 heures à 0°C, puis 48 heures à température ambiante, le solvant a été évaporé et le produit repris dans 25 ml d'eau froide. L'addition de 2,98 g de chlorure de potassium anhydre a permis d'obtenir un précipité qui a été recristallisé, récupéré par filtration, puis séché. On a ainsi obtenu 11,78 g du sel de potassium de la fluorosulfonyl (2,4,6-triisopropylbenzènesulfonyl)imide (73% de rendement) ayant une pureté déterminée par RMN du fluor et du proton supérieure à 99%.

La microanalyse a donné: H 5,58 (5,98) ; C 44,14 (44,53) ; N 3,78 (3,46) ; F 5,02 (4,7) ; ; S 15,23 (15, 85) ; K 10,21 (9,66).

On a préparé le sel de lithium par échange ionique (métathèse) entre le sel de potassium et le chlorure de lithium dans le tétrahydrofurane anhydre.

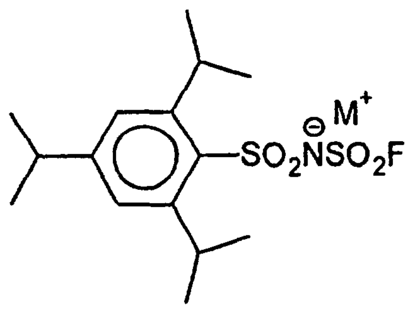

M = Li , K

**Exemple 42 : 1-dodécyl-1,1,1,3,3,3-hexafluoro-2-propanoxysulfonyl(trifluorométhanesulfonyl)imide**

En opérant dans une boîte à gants sous argon, on a mis en solution dans 20 ml de tétrahydrofurane anhydre, 18,96 g (50 mmoles) de trifluorométhanesulfonyl(1,1,1,3,3,3-hexafluoro-2-propanoxysulfonyl)imide, préparé comme à l'exemple 37. Après avoir porté cette solution à -20°C, on a ajouté lentement 100 ml d'une solution 1 M dans le tétrahydrofurane de tert-butoxide de potassium $(CH_3)_3COK$ (100 mmoles, commercialisé par Aldrich). Après 15 min, on a ajouté 12,46 g de 1-bromododécane (50 mmoles). La réaction s'est poursuivie pendant deux heures à -20°C, puis pendant 24 heures à température ambiante. Après 48 heures, le solvant a été évaporé et le résidu recristallisé dans 50 ml d'eau contenant 7,46 g (100 mmoles) de chlorure de potassium KCl. Après filtration et séchage, on a obtenu le sel de potassium de la 1-dodécyl-1,1,1,3,3,3-hexafluoro-2-propanoxysulfonyl(trifluorométhanesulfonyl)imide d'une pureté caractérisée par RMN du proton et du fluor supérieure à 99%.

La microanalyse a donné: H 4,8 (4,5) ; C 34,5 (34,1) ; N 2,8 (2,3) ; F 28,1 (28,5) ; S 10,1 (10,7) ; K 6,1 (6,5).

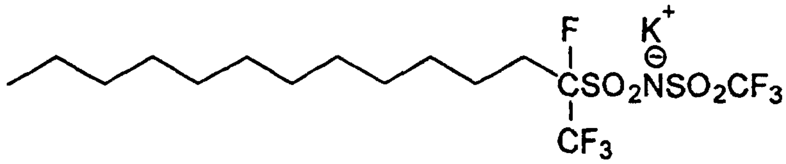

On a obtenu quantitativement le sel de lithium par traitement du sel de potassium dans le tétrahydrofurane anhydre par la quantité stoechiométrique de chlorure de lithium anhydre, filtration du milieu réactionnel, évaporation du solvant et séchage sous vide.

Ces sels peuvent être utilisés comme additifs pour le laminage du lithium et pour l'extrusion de polymères, en particulier l'extrusion du poly (oxyde d'éthylène). Ils présentent des propriétés plastifiantes.

**Exemple 43 : Igepal® CA-520-propylsulfonyl(trifluorométhanesulfonyl)imide.**

Dans 30 ml de tétrahydrofurane, 4,27 g d'Igepal® CA-520 (10 mmoles, commercialisé par Aldrich) ont été traités par 3,28 g (10 mmoles) de trifluorométhanesulfonyl(3-chloropropanesulfonyl)imide obtenu à l'exemple 8, en

présence de 4,24 g de phosphate de potassium $K_3PO_4$ (20 mmoles). Après 72 heures sous agitation à 60°C, le milieu réactionnel a été filtré afin d'éliminer le phosphate de potassium et le chlorure de potassium formé au cours de la réaction. On a récupéré après évaporation du solvant et séchage, 7,18 g du sel de potassium de l'Igepal® CA-520-propylsulfonyl(trifluorométhanesulfonyl)imide ayant une pureté determinée par RMN du proton et du fluor supérieure à 96%.

La microanalyse a donné: H 6,89 (6,6) ; C 46,45 (46,85) ; N 1,69 (1,95) ; F 7,66 (7,94) ; S 8,72 (8,93) ; K 5,75 (5,45).

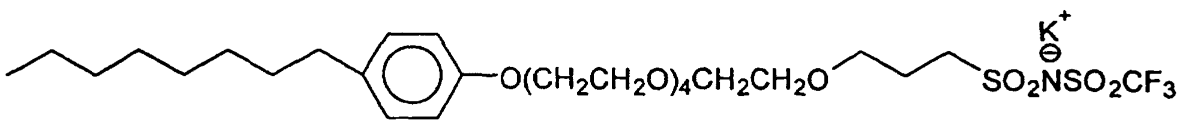

Ce sel est un excellent additif pour l'extrusion du poly (oxyde d'éthylène). Il permet également de plastifier un grand nombre de polymères contenant des unités polaires (éther, amide, nitrile, ester), tout en leur conférant une conductivité ionique élevée.

**Exemple 44 : Toluènesulfonyl(trifluorométhanesulfonyl)imide**

En opérant dans une boîte à gants sous argon, on a ajouté par portions 3,23 g de dichlorotriphénylphosphorane $(C_6H_5)_3PCl_2$ (10 mmoles) à une solution dans 20 ml d'acétonitrile de 2,24 g (20 mmoles) de DABCO et de 1,49 g de trifluorométhanesulfonamide $CF_3SO_2NH_2$ (10 mmoles). Après trois heures sous agitation, le milieu réactionnel a été filtré pour éliminer le précipité de chlorure de DABCO formé, puis le solvant évaporé. On a récupéré quantitativement la triphénylphosphoranylidènesulfonyl-trifluorométhyle $CF_3SO_2N{=}P(C_6H_5)_3$. Ensuite, on a fait réagir ce composé avec 1,94 g (10 mmoles) du sel de sodium de l'acide p-toluènesulfonique dans 10 ml de diméthylformamide à 60°C. Après 48 heures sous agitation, le solvant a été évaporé et le résidu recristallisé dans 10 ml d'eau contenant 1 g de chlorure de potassium KCl. Après filtration et séchage, on a récupéré 2,47 g du sel de sodium de la p-toluènesulfonyl (trifluorométhanesulfonyl)imide (76% de rendement) ayant une pureté déterminée par RMN du proton et du fluor supérieure à 99%.

La microanalyse a donné: H 2,07 (2,17) ; C 29,88 (29,54) ; N 4,01 (4,31) ; F 17,23 (17,52) ; Na 7,15 (7,07) ; S 19,21 (19,71).

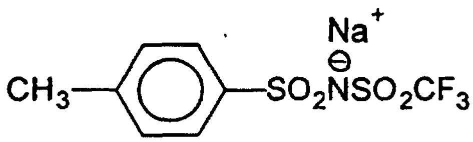

**Exemple 45 : O,O'-[propylsulfonyl(trifluorométhanesulfonyl)imide]polyéthylène glycol.**

Un oligomère sulfoné de poly(oxyde d'éthylène) a été préparé de la manière suivante : 12 g de poly(éthylène glycol) de masse 600 (≈ 40 mmoles de fonctions hydroxyles) ont été séchés par distillation azéotropique avec du benzène et par lyophilisation. Après addition de 50 ml de tétrahydrofurane anhydre, les groupements hydroxyles terminaux ont été métallés par le potassium-naphtalène. La stoechiométrie a été déterminée par colorimétrie, la fin de la réaction étant indiquée par la persistance de la couleur verte intense du radical anion du naphtalène. On a alors ajouté 4,89 g de 1,3-propane sultone (40 mmoles). Après évaporation du solvant, le polymère $\alpha,\omega$-disulfoné a été obtenu sous forme de poudre et le naphtalène résiduel a été éliminé par lavage à l'hexane. 8,44 g du produit ainsi formé (≈ 20 mmoles de $-SO_3H$), mis en suspension dans 20 ml d'acétonitrile anhydre, ont été traités par 2,82 g de chlorure de (chlorométhylène)diméthylammonium $[(CH_3)_2N{=}CHCl]^+$, $Cl^-$ (22 mmoles, commercialisé par Aldrich). Un précipité de chlorure de potassium s'est formé après environ 1 h. A cette suspension ont été ajoutés 3,28 g de trifluorométhanesulfonamide (22 mmoles) et 2,47 g de DABCO (22 mmoles). Après filtration, le mélange réactionnel a été agité en présence de 3,4 g de phosphate de lithium $Li_3PO_4$ pendant 24 heures. Une nouvelle filtration suivie d'une reprécipitation dans 200 ml d'éther à 0°C a permis de récupérer un fluide visqueux très légérement coloré caractérisé par RMN du proton et du fluor comme le di-sel de lithium du poly(éthylène glycol) $\alpha,\omega$-trifluorométhanesulfonyl(propanesulfonyl)imide :

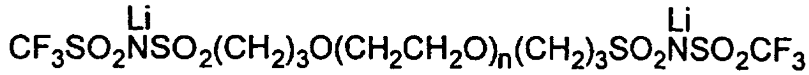

Ce sel est soluble dans la plupart des solvants organiques polaires (acétonitrile, tétrahydrofurane, DMF) et il permet de plastifier un grand nombre de polymères contenant des unités polaires (éther, amide, nitrile, ester), tout en leur conférant une conductivité ionique élevée.

**Exemple 46 : Trifluorométhanesulfonyl(R(-)-1-phényl-2,2,2-trifluoroéthanoxysulfonyl)imide**

5,96 g (40 mmoles) de trifluorométhanesulfonamide et 9,9 ml de pyridine dans 60 ml de dichlorométhane anhydre ont été refroidis à -15°C. On a alors ajouté goutte à goutte 5,4 g (40 mmoles) de chlorure de sulfuryle dilués dans 10 ml de dichlorométhane anhydre, puis 7,05 g (40 mmoles) de R(-)-1-phényl-2,2,2-trifluoroéthanol (commercialisé par Fluka). Le mélange a été agité pendant 1 heure à -15°C, puis pendant 4 heures à température ambiante (25°C). Le mélange réactionnel a été filtré et le solvant a été éliminé à l'aide d'un évaporateur rotatif. Le produit récupéré a été repris dans 20 ml d'éthanol. Un précipité s'est formé après addition de 3,93 g (40 mmoles) d'acétate de potassium. Après recristallisation, filtration et séchage, on a obtenu 12,25 g du sel de potassium de la trifluorométhanesulfonyl-(R(-)-1-phényl-2,2,2-trifluoroéthanoxysulfonyl)imide (72% de rendement) ayant une pureté caractérisée par RMN du fluor et du proton supérieure à 98%.
La microanalyse a donné: H 1,65 (1,42) ; C 25,21 (25,41) ; N 3,55 (3,29) , F 26,21 (26,8) ; S 15,65 (15,07) ; K 9,56 (9,19).

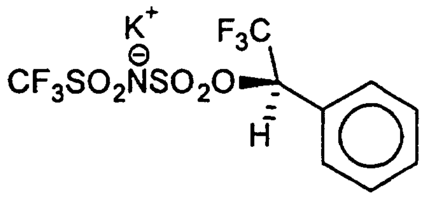

De la même manière, on a obtenu le sel de potassium de la trifluorométhanesulfonyl(S(+)-1-phényl-2,2,2-trifluoroéthanoxysulfonyl)imide (rendement 66%) à partir du R(-)-1-phényl-2,2,2-trifluoroéthanol.
On a obtenu les sels de lithium par échange ionique (métathèse) dans le tétrahydrofurane avec le chlorure de lithium.
On a obtenu les sels de lanthane en traitant les sels de potassium par une quantité stoechiométrique de perchlorate de lanthane $La(ClO_4)_3$, $6H_2O$ dans un mélange d'acétonitrile et d'orthoformate d'isopropyle destiné à éliminer l'eau de cristallation du sel de lanthane. Après filtration pour éliminer le précipité de perchlorate de potassium $KClO_4$ et évaporation du solvant, on a récupéré quantitativement les sels de lanthane des deux énantiomères de la trifluorométhanesulfonyl(1-phényl-2,2,2-trifluoroéthanoxysulfonyl)imide.
Ces sels sont solubles dans la plupart des solvants organiques polaires (acétonitrile, tétrahydrofurane, DMF) et dans les polymères solvatants aprotiques.

**Exemple 47 : Trifluorométhanesulfonyl(N-méthoxybutyl-N-2-butyl-3-méthyl)aminosulfonyl)imide**

Les deux énantiomères du sel de potassium de la trifluorométhanesulfonyl (N-méthoxybutyl-N-2-butyl-3-méthyl)-aminosulfonyl)imide ont été obtenus par un procédé similaire à celui décrit dans l'exemple 28, à partir de la N-méthoxybutyl-N-2-butyl-3-méthyl-amine (commercialisé par Air Products) avec une pureté supérieure à 99% et un rendement de 62%.
On a également obtenu par le même procédé les sels de potassium des deux énantiomères de la la fluorosulfonyl-(N-méthoxybutyl-N-2-butyl-3-méthyl)aminosulfonyl)imide.
On a obtenu les sels de lithium par échange ionique (métathèse) dans le tétrahydrofurane avec le chlorure de lithium.
On a obtenu par un procédé similaire à celui décrit dans l'exemple 46, les sels de lanthane des deux énantiomères de la trifluorométhanesulfonyl(N-méthoxybutyl-N-2-butyl-3-méthyl)-aminosulfonyl)imide et des deux énantiomères de la fluorosulfonyl(N-méthoxybutyl-N-2-butyl-3-méthyl)aminosulfonyl)imide.

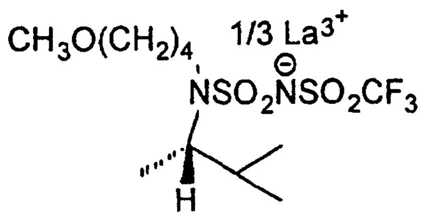

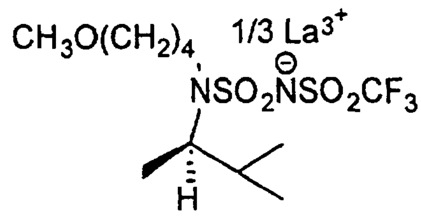

Ces sels sont solubles dans la plupart des solvants organiques polaires (acétonitrile, tétrahydrofurane, DMF) et dans les polymères solvatants aprotiques.

**Exemple 48 : Camphorsulfonyl(trifluorométhanesulfonyl)imide.**

Suivant un procédé similaire à celui décrit dans l'exemple 39, on a obtenu le sel de potassium de la (1R)-(-)-10-camphorsulfonyl(trifluorométhanesulfonyl)imide à partir du chlorure de (1R)-(-)-10-camphorsulfonyle (commercialisé par Aldrich), et le sel de potassium de la (1S)-(+)-10-camphorsulfonyl(trifluorométhanesulfonyl)imide à partir du chlorure de (1S) - (+)-10-camphorsulfonyle (commercialisé par Aldrich) avec des rendements supérieurs à 70%. La pureté des composés obtenus, déterminée par RMN du proton et du fluor, est supérieure à 99%.
On a obtenu de même les sels de potassium de la (1R)-(-)-10-camphorsulfonyl(perfluorobutanesulfonyl)imide et de la (1S)-(+)-10-camphorsulfonyl(perfluorobutanesulfonyl)imide à partir de la perfluorobutanesulfonamide $C_4F_9SO_2NH_2$ obtenu à l'exemple 4.
On a obtenu les sels de lithium par échange ionique (métathèse) dans le tétrahydrofurane avec le chlorure de lithium.
On a obtenu par un procédé similaire à celui décrit dans l'exemple 46, les sels de lanthane de la (1R)-(-)-10-camphorsulfonyl(perfluorobutanesulfonyl)imide et de la (1S)-(+)-10-camphorsulfonyl(perfluorobutanesulfonyl) imide :

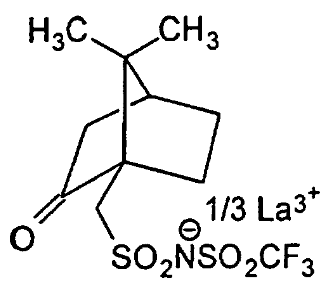

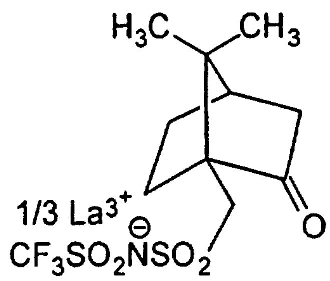

Ces sels sont solubles dans la plupart des solvants organiques polaires (acétonitrile, tétrahydrofurane, DMF) et dans les polymères solvatants aprotiques.

**Exemple 49 : N-(1S)-(+)-kétopinique-acétylméthylsulfonyl (trifluorométhanesulfonyl) imide**

En opérant dans une boîte à gants sous argon, à 2,8 g (10 mmoles) du sel de potassium de la trifluorométhanesulfonyl(N-méthyl-sulfonyl) imide $CF_3SO_2NKSO_2NH(CH_3)$ obtenu à l'exemple 10, en solution dans 5 ml de tétrahydrofurane anhydre, on a ajouté lentement 10 ml d'une solution 1 M dans le tétrahydrofurane de tert-butoxide de potassium $(CH_3)_3COK$ (10 mmoles).
Dans le même temps, à 1,82 g (10 mmoles) de l'acide (1S)-(+)-kétopinique (commercialisé par Aldrich) en solution dans 10 ml d'acétonitrile anhydre, on a ajouté 80 mg d'hydrure de lithium LiH (10 mmoles), puis après quelques minutes, 1,27 g de chlorure d'oxalyle ClCOCOCl (10 mmoles). Après centrifugation, on a versé dans cette solution la solution de THF. La réaction s'est poursuivie 24 heures à température ambiante. Le milieu réactionnel a alors été filtré pour éliminer le précipité de chlorure de potassium, puis le solvant a été évaporé et le produit recristallisé dans 6 ml d'eau. Après filtration et séchage, on a obtenu 2,76 g du sel de potassium de la trifluorométhanesulfonyl(N-(1S)-(+)-kétopinique-acétyl-N-méthylsulfonyl) imide (62% de rendement), ayant une pureté caractérisée par RMN du proton et du fluor supérieure à 99%.

On a obtenu le sel de scandium en traitant le sel de potassium par une quantité stoechiométrique de tétrafluoroborate de scandium $Sc(BF_4)_3$ dans l'acétonitrile. Après filtration pour éliminer le précipité de tétrafluoroborate de potassium $KBF_4$ et évaporation du solvant, on a récupéré quantitativement, après séchage, le sel de scandium de la trifluorométhanesulfonyl(N-(1S)-(+)-kétopinique-acétyl-N-méthylsulfonyl)imide.

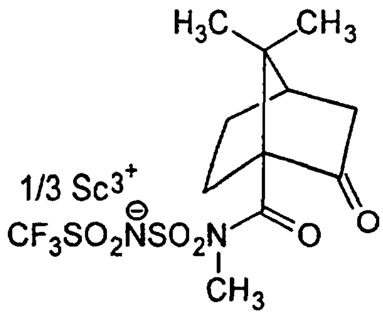

**Exemple 50 : Dialkylaminosulfonyl(trifluorométhanesulfonyl)imidediphényliodonium**

1,58 g (5 mmoles) de chlorure de diphényliodonium $(C_6H_5)_2ICl$ et 1,89 g du sel de potassium de la trifluorométhanesulfonyl (dibutylaminosulfonyl)imide $(C_4H_9)_2NSO_2NKSO_2CF_3$ (5 mmoles) ont été agités ensemble durant 24 heures dans l'eau. Par extraction de la phase aqueuse par du dichlorométhane, on a récupéré après évaporation du dichlorométhane et séchage, 3,01 g du sel de trifluorométhanesulfonyl-(dibutylaminosulfonyl) imide du diphényliodonium (97% de rendement) ayant une pureté caractérisée par RMN du proton et du fluor supérieure à 99%.

On a préparé par le même procédé le sel de trifluorométhanesulfonyl(N,N-di-2-éthylhexylaminosulfonyl)imide du diphényliodonium avec un rendement de 98% et ayant une pureté caractérisée par RMN du proton et du fluor supérieure à 99%.

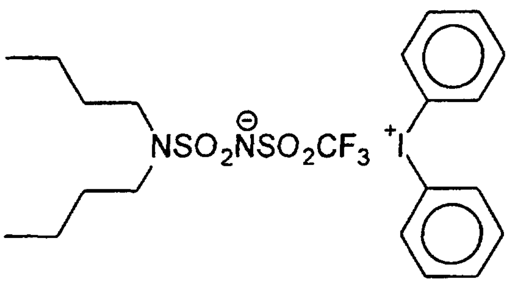

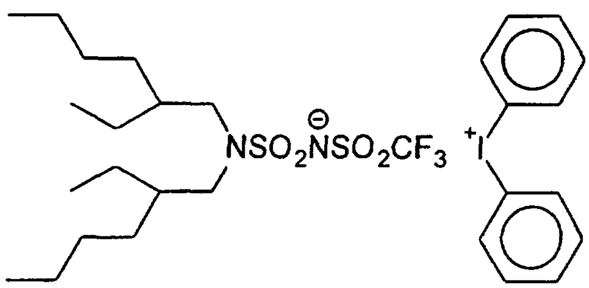

Ces sels permettent d'amorcer sous l'effet d'un rayonnement actinique (lumière, rayons $\gamma$, faisceaux d'électrons) la réaction de réticulation cationique de monomères riches en électrons (éthers vinyliques, alkyls vinyls éthers).

Ils sont solubles dans la plupart des solvants organiques usuels (tétrahydrofurane, acétonitrile, diméthylformamide, acétate d'éthyle, glymes) et dans les polymères solvatants aprotiques comme le poly (oxyde d'éthylène). Ils sont aussi solubles à plus de 10% en poids dans les solvants réactifs comme le triéthylèneglycol divinyl éther. On a testé les propriétés de photo-amorçage de ces sels en irradiant par un rayonnement U.V. à 254 nm, d'une puissance de 1900 $mW/cm^2$, une solution de triéthylèneglycol divinyl éther les contenant à 1% en poids. Après quelques secondes sous irradiation, le solvant réactif a pris en masse, cette réaction étant très exothermique.

**Exemple 51 : (4-butoxybenzène)-[trifluorométhanesulfonyl-(4-phénylsulfonyl)imide)]-iodonium**

A une solution dans 10 ml de tétrahydrofurane anhydre à 0°C de 4,49 g (40 mmoles) de DABCO et de 2,98 g de trifluorométhanesulfonamide $CF_3SO_2NH_2$ (20 mmoles), on a ajouté 6,05 g de chlorure de 4-iodobenzenesulfonyle $IC_6H_4SO_2Cl$ (20 mmoles, Aldrich) dilués dans 5 ml de tétrahydrofurane anhydre. Après deux heures à 0°C, la réaction s'est poursuivie durant 24 heures à l'ambiante. L'hydrochlorure de DABCO formé au cours de la réaction a été éliminé par filtration sur un verre fritté de porosité N°4. Après évaporation de l'acétonitrile de la solution filtrée, le produit a été repris dans 15 ml d'eau froide et l'on a ajouté lentement 1,49 g de chlorure de potassium anhydre (20 mmoles) en

solution dans 5 ml d'eau. Il est apparu un précipité qui a été recueilli par filtration sur un verre fritté de porosité N°4. Après séchage, on a récupéré 7,89 g du sel de potassium de la trifluorométhanesulfonyl(4-iodobenzenesulfonyl)imide (87% de rendement) caractérisé par RMN du proton et du fluor.

Ce composé a été oxydé en $CF_3SO_2NKSO_2C_6H_4I(O_2CCH_3)_2$ (iodosoacétate) par le mélange acide acétique, anhydride acétique et peroxyde d'hydrogène selon la méthode de Yamada & al (Die Makromolecular Chemie, (1972), 152, 153-162). 5,71 g du composé ainsi préparé (10 mmoles) ont été mis en suspension dans un mélange de 15 ml d'acide méthanesulfonique et de 4,51 g de butoxybenzène (30 mmoles) maintenu à 0°C pendant 4 heures. Le produit de réaction a été versé dans 300 ml d'éther et le précipité séparé par filtration, lavé par de l'éther et séché. On a ainsi obtenu 4,62 g (82% de rendement) du zwitterion du (4-butoxybenzène)-[trifluorométhanesulfonyl-(4-phénylsulfonyl) imide)]iodonium [$CF_3SO_2N^-SO_2C_6H_4I^+C_6H_4OC_4H_9$] ayant une pureté caractérisée par RMN du proton et du fluor supérieure à 97%.

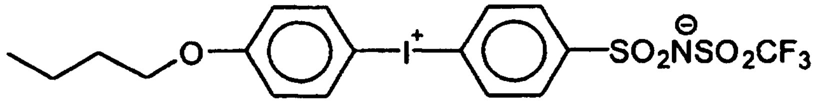

On a obtenu par un procédé similaire le (4-butoxybenzène)-[pentafluoroéthanesulfphonyl-(4-phénylsulfonyl) imide)]iodonium à partir de la pentafluoroéthanesulfonamide et le (4-butoxybenzène)-[perfluorobutanesulfonyl-(4-phénylsulfonyl)imide)]iodonium à partir de la perfluorobutanesulfonamide-.

Ces composés présentent des propriétés analogues à celles des composés de l'exemple 50 et peuvent être utilisés pour les mêmes applications.

**Exemple 52 : Tétrakis(acétonitrile)palladium(II) trifluorométhanesulfonyl-(N,N-di-2-éthylhexylaminosulfonyl) imide**

2,22 g (5 mmoles) de tétra-fluoroborate de tétrakis(acétonitrile)-palladium(II) $(CH_3CN)_4Pd(BF_4)_2$ (commercialisé par Aldrich), dans 30 ml de tétrahydrofurane, ont été traités par 4,91 g du sel de potassium de la trifluorométhanesulfonyl (N,N-di-2-éthylhexylaminosulfonyl)imide (10 mmoles) obtenu à l'exemple 28. Après 24 heures sous agitation, le milieu réactionnel a été filtré pour éliminer le précipité de tétrafluoroborate de potassium $KBF_4$, puis le solvant évaporé. On a obtenu quantitativement la trifluorométhanesulfonyl(N,N-di-2-éthylhexylaminosulfonyl)imide de tétrakis (acétonitrile)-palladium(II).

Ce sel est utile comme catalyseur de la polymérisation vinylique du norbornène. Ainsi on a réalisé la polymérisation à température ambiante du norbornène dans le nitrométhane en présence de 300 ppm de ce sel. Après 2 heures, on a reprécipité le milieu réactionnel dans le méthanol. On a obtenu le polynorbornène d'une masse moyenne en nombre de 420 000 avec un rendement de 82%.

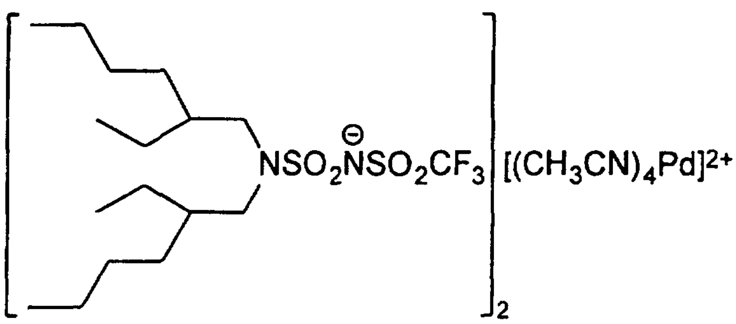

**Exemple 53 : 5-diméthylamino-1-naphtalènesulfonyl(trifluorométhanesulfonyl)imide**

Dans 20 ml d'acétonitrile anhydre, on a fait réagir 5,39 g (20 mmoles) du chlorure de 5-diméthylamino-1-naphtalènesulfonyle (commercialisé par Aldrich), 2,98 g (20 mmoles) de trifluorométhanesulfonamide $CF_3SO_2NH_2$ et 4,49 g (40 mmoles) de DABCO. Après 24 heures sous agitation à température ambiante, le milieu réactionnel a été filtré pour éliminer l'hydrochlorure de DABCO formé, puis l'acétonitrile a été évaporé. Le produit obtenu a été recristallisé dans 20 ml d'eau additionnée de 2,98 g (40 mmoles) de chlorure de potassium. Après filtration et séchage, on a récupéré 5,63 g du sel de potassium de la trifluorométhanesulfonyl(5-diméthylamino-1-naphtalènesulfonyl)imide (69%

de rendement) ayant une pureté caractérisée par RMN du fluor et du proton supérieure à 99%.

La microanalyse a donné: H 2,96 (2,88) ; C 37,23 (37,14) ; N 6,41 (6,66) ; F 13,98 (13,56) ; S 15,65 (15,25) ; K 9,46 (9, 3).

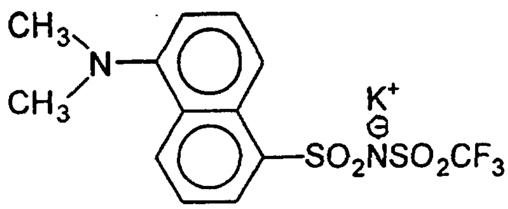

Ce sel fluorescent est soluble dans la plupart des solvants organiques polaires (acétonitrile, tétrahydrofurane, DMF).

**Exemple 54 : N-trifluorométhanesulfonyl-2-aminoacridine**

Par un procédé similaire à celui décrit dans l'exemple 34, on a obtenu le sel de magnésium de la N-trifluorométhanesulfonyl-2-aminoacridine par action du chlorure de trifluorométhanesulfonyle sur le magnésien de la 2-aminoacridine dans le tétrahydrofurane. Après évaporation des solvants, le produit a été repris dans l'eau et traité par du bromure de tétraéthylammonium (10% en excès) dans l'eau, il est alors apparu un précipité. Après filtration et séchage, on a obtenu le sel de tétraéthylammonium de la N-trifluorométhanesulfonyl-2-aminoacridine (rendement 66%) ayant une pureté déterminée par RMN du proton et du fluor supérieure à 99%.

La microanalyse a donné: H 6,11 (6,39) ; C 59,25 (59,85) ; N 6,89 (6,34) ; F 12,25 (12,91) ; S 7,95 (7,26).

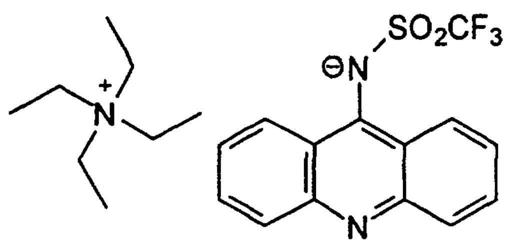

On a obtenu de la même façon le sel de tétraéthylammonium de la N-fluorosulfonyl-2-aminoacridine à partir de la fluorosulfonamide.

Ce sel est soluble dans la plupart des solvants organiques usuels (tétrahydrofurane, acétonitrile, diméthylformamide, acétate d'éthyle, glymes) et dans les polymères solvatants aprotiques tel le poly (oxyde d'éthylène), ainsi que dans les polymères peu polaires comme le polyméthacrylate de méthyle.

Ces sels présentent une fluorescence très marquée. On a réalisé une solution dans le dichloroéthane de polyméthylméthacrylate (PMMA) et de l'un ou l'autre de ces sels d'ammonium (97:3). On a obtenu des dépôts fluorescents sur de nombreux supports (verre, polymère).

**Exemple 55 : 1,3-phénylsulfonamide-N,N'-perfluoro-1-butanesulfonyle rhodamine B**

A 2,9 g de sulforhodamine B (5 mmoles, commercialisé par Aldrich) dans 15 ml de diméthylformamide anhydre, on a ajouté 941 mg du sel de potassium de l'acide trifluorométhanesulfonique $CF_3SO_3K$ (5 mmoles). Après deux heures sous agitation, on a ajouté lentement 1,27 g de chlorure d'oxalyle ClCOCOCl (10 mmoles) en solution dans 10 ml de dichlorométhane anhydre. La réaction s'est poursuivie pendant une nuit sous argon, puis on a ajouté 6,74 g du sel de potassium de perfluoro-1perfluoro-1-butanesulfonamide $C_4F_9SO_2NHK$ (20 mmoles4. Après 48 heures, la diméthylformamide a été évaporée et le résidu recristallisé dans 40 ml d'eau. Après filtration et séchage, on a obtenu 4,11 g (71% de rendement) du sel de potassium de la 1,3-phénylsulfonamide-N,N'-(perfluoro-1-butanesulfonyle) rhodamine B ayant une pureté caractérisée par RMN du proton et du fluor supérieure à 99%. La microanalyse a donné: H 2,76 (2,52) ; C 36,56 (36,27) ; N 4,96 (4,83) ; F 29,99 (29,51) ; S 11,55 (11,07) ; K 3,17 (3,37).

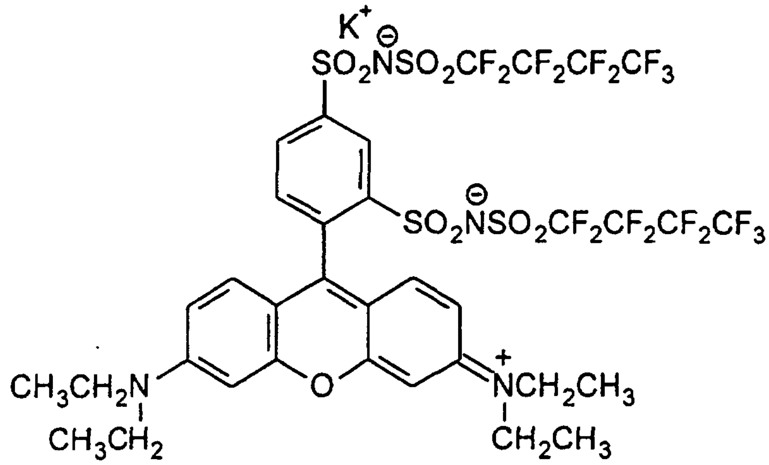

Suivant le même procédé, on a obtenu le disel de potassium de la 1,3-phénylsulfonamide-N,N'-fluorosulfonyle rhodamine B à partir du sel de potassium de la fluorosulfonamide , le disel de potassium de la 1,3-phénylsulfonamide-N, N'-trifluorométhanesulfonyle rhodamine B à partir du sel de potassium de la trifluorométhanesulfonamide et le disel de potassium de la 1,3-phénylsulfonamide-N,N'-pentafluoroéthanesulfonyle rhodamine B à partir de la pentafluorosulfonamide.

Les sels de lithium ont été obtenus par métathèse avec le chlorure de lithium dans le tétrahydrofurane.

Ces zwitterions possèdent des propriétés colorantes intenses. Ils sont solubles dans les polymères polaires et permettent la constitution de lasers à colorants. Les groupements sulfonimides leurs permettent également de s'adsorber sur des oxydes en particulier le dioxyde de titane nano-particulaire ; ils jouent alors un rôle de sensibilisateur au rayonnement visible, en particulier dans les applications aux cellules photovoltaïques.

**Exemple 56 : trifluorométhanesulfonyl(anthracènyl-9-éthanesulfonyl)imide**

Dans un réacteur chimique Parr, on a introduit une solution de 9,36 g du sel de potassium de la trifluorométhanesulfonamide $CF_3SO_2NHK$ (50 mmoles) et de 264 mg d'un éther couronne, le 18-Crown-6 (agissant comme complexant du cation potassium), dans 60 ml d'acétonitrile anhydre. Après avoir fermé le réacteur, on a effectué un balayage d'argon pendant 15 min avant de l'isoler. On a alors introduit 6,41 g de dioxyde de soufre $SO_2$ (50 mmoles, commercialisé par Fluka) puis, après 10 min, 10,21 g de 9-vinylanthracène (50 mmoles, commercialisé par Lancaster) en solution dans 20 ml de dichlorométhane anhydre. Après 6 heures à température ambiante, le réacteur a été porté à 50°C et maintenu à cette température pendant 48 heures, puis le solvant a été évaporé et le produit séché. On a récupéré quantitativement le sel de potassium de la trifluorométhanesulfonyl(anthracényl-9-éthanesulfonyl)imide ayant une pureté caractérisée par RMN du fluor et du proton supérieure à 99%.

La microanalyse a donné: H 2,78(2,88) ; C 44,53 (44,83) ; N 3,33 (3,07) ; F 12,01 (12,51) ; S 14,36 (14,08) ; K 8,99 (8,58).

On a obtenu suivant le même procédé le sel de potassium de la fluorosulfonyl(anthracényl-9-éthanesulfonyl) imide.

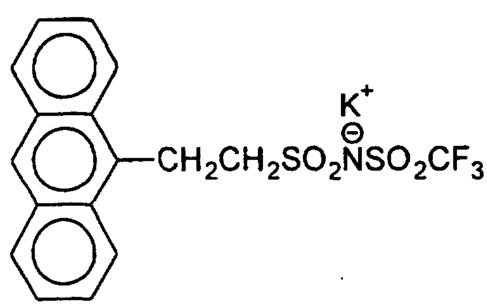

Ce sel est soluble dans la plupart des solvants organiques usuels (tétrahydrofurane, acétonitrile, diméthylformamide, acétate d'éthyle, glymes) et dans les polymères polaires.

**Exemple 57 : N,N',N",N"'-perfluorobutanesulfonyl-nickel(II) phtalocyaninetétrasulfonamide**

A 4,9 g du sel de sodium de l'acide Nickel(II) phtalocyaninetétrasulfonique (5 mmoles, commercialisé par Aldrich) dans 40 ml de diméthylformamide anhydre, on a ajouté lentement 2,54 g de chlorure d'oxalyle ClCOCOCl (20 mmoles) en solution dans 10 ml de dichlorométhane anhydre. Après 4 heures sous agitation, le milieu réactionnel a été centrifugé, le liquide surnageant éliminé, puis le produit décanté repris dans 40 ml de diméthylformamide anhydre. On a alors ajouté 13,5 g du sel de potassium de la perfluoro-1-butanesulfonamide $C_4F_9SO_2NHK$ (40 mmoles). Après 48 heures, la diméthylformamide a été évaporée et le résidu recristallisé dans 50 ml d'eau additionnée de 1,49 g (20 mmoles) de chlorure de potassium anhydre. Après filtration et séchage, on a obtenu 9,54 g (81% de rendement) du sel de potassium de la N,N',N",N"'-perfluorobutanesulfonyl-Nickel (II) phtalocyaninetétrasulfonamide ayant une pureté caractérisée par RMN du proton et du fluor supérieure à 99%.

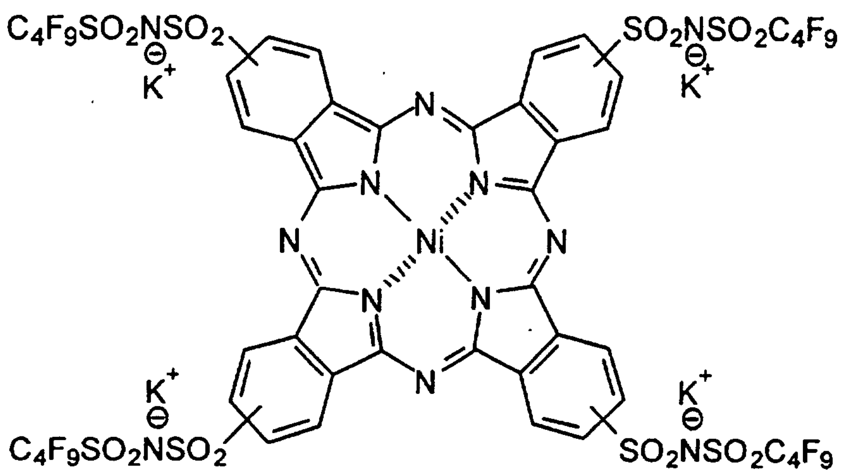

Ce sel est soluble dans la plupart des solvants organiques usuels (tétrahydrofurane, acétonitrile, diméthylformamide, acétate d'éthyle, glymes) et dans les polymères polaires auxquels ils donnent une couleur bleue intense et stable vis à vis de la lumière. Ce sel, de même que les sels analogues de nickel, de fer ou de manganère sont utiles comme catalyseurs de la réduction de l'oxygène

**Exemple 58**

Dans 30 ml de THF, on a fait réagir 6,76 g de pentafluoropyridine (40 mmoles, commercialisé par Aldrich) avec 7,49 gr (40 mmoles) du sel de potassium de la trifluorométhanesulfonamide $CF_3SO_2NHK$ en présence de 4,49 g (40 mmoles) de DABCO. Après 48 heures sous agitation, le solvant a été évaporé et le résidu recristallisé dans 20 ml d'eau. Après filtration et séchage, on a obtenu 8,03 g du sel de potassium de la trifluorométhanesulfonyl(4-aza-pentafluoropyridine)amide (76% de rendement), ayant une pureté déterminée par RMN du fluor supérieure à 99%.

Suivant le même procédé, on a obtenu le sel de potassium de la trifluorométhanesulfonyl((4,6-dinitro-2-trifluorométhyl)phényl)amide à partir de 10,82 g du 2-chloro-3,5-dinitrobenzotrifluorure (40 mmoles, commercialisé par Aldrich), ayant une pureté déterminée par RMN du fluor, du proton et du carbone supérieure à 99%.

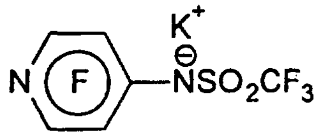

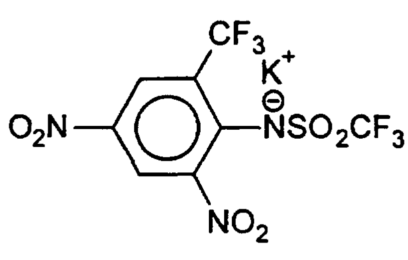

On a obtenu les sels de lithium par échange ionique avec le chlorure de lithium dans le THF.

Ces sels sont solubles dans la plupart des solvants organiques usuels (tétrahydrofurane, acétonitrile, diméthylformamide, acétate d'éthyle, glymes) et dans les polymères solvatants aprotiques.

**Exemple 59 : N,N'-trifluorométhanesulfonyl-3,4-amino-3-cyclobutène-1,2-dione**

En boîte à gants sous argon, à 5,61 g (30 mmoles) de trifluorométhanesulfonamide en solution dans 40 ml de tétrahydrofurane anhydre à -30°C, on a ajouté goutte à goutte 30 ml d'une solution 1 M de dibutylmagnésium $(C_4H_9)$ 2Mg (30 mmoles, commercialisé par Aldrich) dans l'heptane. Après 4 heures à -30°C, on a ajouté lentement 2,55 g (15 mmoles) de 3,4-diéthoxy3-cyclobutène-1,2-dione. La réaction s'est poursuivie pendant 2 heures à -30°C, puis pendant 24 heures à l'ambiante. Les solvants ont alors été évaporés, le produit a été repris dans l'eau et extrait par de l'éther après acidification de la solution aqueuse. Le composé obtenu après évaporation de l'éther a été sublimé sous vide secondaire à 40°C, et on a récupéré après 24 heures sur un doigt froid, 5,02 g de N,N'-trifluorométhanesulfonyl-3,4-amino-3-cyclobutène-1,2-dione (89% de rendement).

La microanalyse a donné: H 0,78 (0,54) ; C 18,89 (19,16) ; N 7,04 (7,45) ; F 29,88 (30,3) ; S 16,71 (17,04).

Le sel de lithium a été obtenu en traitant l'acide par le carbonate de lithium $Li_2CO_3$ dans l'eau.

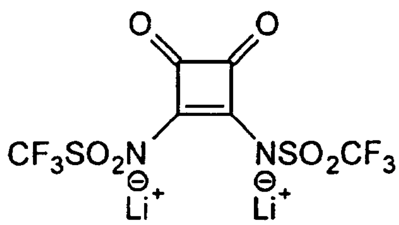

Ces sels sont solubles dans la plupart des solvants organiques usuels (tétrahydrofurane, acétonitrile, diméthylformamide, acétate d'éthyle, glymes) et dans les polymères polaires.

Ces sels présentent deux couples rédox réversibles, ils sont réoxydés à l'état neutre au potentiel d'oxydation de $LiCoO_2$ en fin de charge. Par dissolution dans un électrolyte liquide, gel ou polymère, ils permettent d'effectuer une protection en surcharge, jouant ainsi un rôle de navette rédox. Ils permettent aussi de réaliser des systèmes électrochromes à colorants.

**Exemple 60 : 1,1'-(propylsulfonamide-N-trifluorométhanesulfonyl) ferrocène**

Au cours d'une première étape, on a préparé le dilithien du ferrocène complexé par la tétraméthyléthylènediamine (TMEDA) de la manière suivante: En opérant dans une boîte à gants sous argon, on a placé 37 ml de TMEDA (247 mmoles) fraîchement distillé et 40 ml d'hexane anhydre dans un ballon de 1 1. On a ensuite ajouté goutte à goutte 154 ml d'une solution 1,6 M de butyllithium dans l'hexane (247 mmoles, commercialisé par Aldrich). Après 10 mn, on a ajouté goutte à goutte 18,6 g de ferrocène (100 mmoles) en solution dans 500 ml d'hexane anhydre tout en maintenant une forte agitation de la solution. Après une nuit, il est apparu des cristaux oranges dans la solution, qui ont été récupérés par filtration de la solution sur un verre fritté de porosité N°4. Après séchage sous vide, on a obtenu 28,4 g de 1,1'-dilithioferrocène•2 TMEDA (66% de rendement) que l'on a conservés sous argon.

8,61 g de ce composé (20 mmoles) dans 30 ml d'acétonitrile anhydre ont ensuite été traités par 4,89 g de 1,3-propane sultone (40 mmoles) en boîte à gants. Après 24 heures à température ambiante, on a ajouté deux gouttes de diméthylformamide dans le milieu réactionnel, puis l'on a ajouté lentement 5,08 g de chlorure d'oxalyle ClCOCOCl (40 mmoles) en solution dans 15 ml de dichlorométhane anhydre. Après 4 heures à température ambiante, on a ajouté 14,97 g du sel de potassium de la trifluorométhanesulfonamide (80 mmoles). La réaction s'est poursuivie pendant 24 heures, puis le solvant a été évaporé. Le composé recueilli a alors été recristallisé dans 30 ml d'eau contenant 3 g de chlorure de potassium. Après filtration et séchage, on a récupéré 10,2 g du disel de potassium du 1,1'-(propylsulfonamide-N-trifluorométhanesulfonyl)ferrocène (66% de rendement) dont la pureté caractérisée par RMN du proton et du fluor est supérieure à 97%.

La microanalyse a donné: H 2,38 (2,62) ; C 28,72 (28,13) ; N 3,13 (3,64) ; F 15,16 (14,83) ; S 17,12 (16,68) ; K 10,56 (10,17) ; Fe 7,65 (7,27).

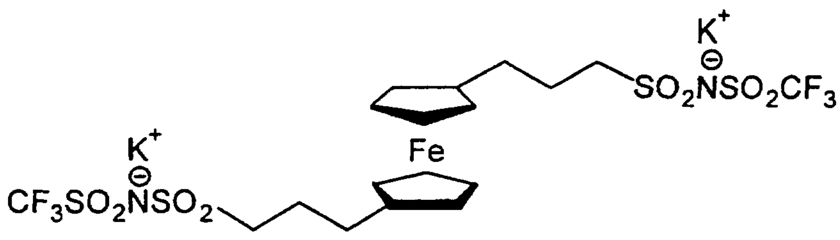

On a obtenu par un procédé similaire le di-sel de potassium de la 1,1'-(propylsulfonamide-N-fluorosulfonyl)-ferrocène.

Les sels de lithium ont été obtenus en traitant l'acide par le carbonate de lithium $Li_2CO_3$ dans l'eau.

Ces sels sont solubles dans la plupart des solvants organiques usuels (tétrahydrofurane, acétonitrile, diméthylformamide, acétate d'éthyle, glymes) et dans les polymères polaires.

Ces sels présentent un couple rédox réversible. Dans le poly (oxyde d'éthylène), on a détermineé, sur une électrode de platine d'un diamétre de de 125 µm, un potentiel réversible ≈ 3,4 V vis à vis d'une électrode de lithium.

Par dissolution dans un électrolyte liquide, gel ou polymère, les sels permettent d'effectuer une protection en surcharge jouant ainsi un rôle de navette rédox. Ils permettent aussi de réaliser des systèmes électrochromes à colorants.

**Exemple 61 : 9-10-( propylsulfonamide-N-trifluorométhanesulfonyl) phénazine**

En opérant dans une boîte à gants sous argon, on a introduit dans un flacon en Nalgène de 30 ml, 1,8 g de phénazine (10 mmoles) et 139 mg de lithium métallique. On a alors ajouté 20 ml de tétrahydrofurane anhydre et des billes en agate. Après avoir fermé le flacon, on l'a mis en rotation sur lui-même, à l'extérieur de la boîte à gants, sur l'axe d'un moteur. Le tétrahydrofurane a rapidement pris une couleur mauve foncé, caractéristique de la phénazine mono-lithiée. Après 24 heures, on a obtenu en suspension un précipité orange de 9,10-di-Li-dihydrophénazine. On a alors ajouté sous argon 6,56 g du sel de potassium de la trifluorométhanesulfonyl-(3-chloropropanesulfonyl)imide (20 mmoles), obtenu à l'exemple 8. Le flacon a alors été remis en rotation sur lui-même pendant 24 heures, puis le milieu réactionnel a été filtré sous argon pour éliminer le précipité de chlorure de potassium formé au cours de la réaction et les billes en agate.

Après évaporation du solvant, on a récupéré 6,52 g du disel de lithium de la 9-10-(propylsulfonamide-N-trifluorométhanesulfonyl)phénazine.

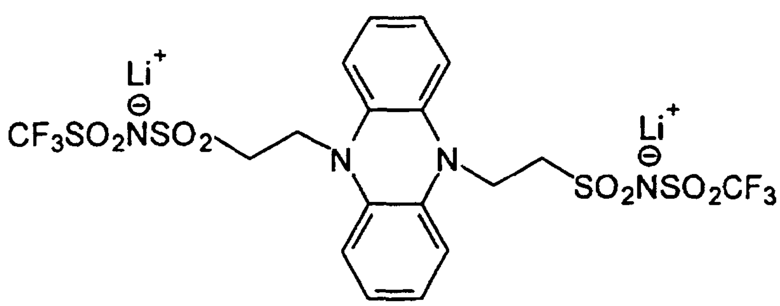

Ce sel est soluble dans la plupart des solvants organiques usuels (tétrahydrofurane, acétonitrile, diméthylformamide, acétate d'éthyle, glymes,...) et dans les polymères polaires.

Ce sel présente deux couples rédox réversibles. Dans le poly (oxyde d'éthylène), on a mis en évidence, sur une électrode de platine d'un diamètre de 125 µm, un premier couple rédox d'un potentiel ≈ 3,2 V et un second couple rédox d'un potentiel ≈ 3,8 V, ces potentiels étant mesurés vis à vis d'une électrode de lithium.

Par dissolution dans un électrolyte liquide, gel ou polymère, ce sel permet d'effectuer une protection en surcharge jouant ainsi un rôle de navette rédox.

Ce sel peut aussi être utilisé dans des systèmes électrochromes à colorants. On a ainsi réalisé un vitrage électrochrome en déposant sur une plaque de verre recouverte d'une couche conductrice d'ITO (oxyde d'indium et d'étain), une solution dans l'acétone de ce composé et de poly(benzodiimide-co-oxyde d'éthylène) ayant une masse

molaire ≈ 1100 g/mole. Après évaporation du solvant et séchage, on a déposé en boîte à gants sur la couche de copolymère, une deuxième électrode de verre recouverte d'une couche conductrice d'ITO (oxyde d'indium et d'étain). Après avoir scellé l'ensemble pour le rendre étanche, on a appliqué à l'extérieur un potentiel de 1250 mV à l'aide d'un potentiostat. Le système s'est alors coloré en bleu intense. En appliquant un potentiel de -500 mV, on a observé une décoloration relativement rapide du système (inférieur à 60 s).

Un tel système électrochrome est facile à mettre en oeuvre, même pour des systèmes de grande taille (supérieure au $m^2$) qui utilisent aussi bien du verre qu'un polymère convenablement traité comme électrode transparente conductrice. De plus, l'énergie nécessaire pour conserver la coloration est relativement faible, inférieure à 1 $W/m^2$.

**Exemple 62 : 2,2'-Azinobis(3-éthylbenzothiazoline-6(sulfonyl (trifluorométhanesulfonyl)imide).**

Dans un premier temps, on a préparé le disel de sodium de l'acide 2,2'-azinobis(3-éthylbenzothiazoline-6-sulfonique) à partir de son disel d'ammonium (commercialisé par Aldrich), en le traitant par une solution titrée d'hydroxyde de sodium. Après évaporation et séchage, on a récupéré quantitativement le disel de sodium. A 1,12 g de ce dernier (2 mmoles) dans 10 ml d'acétonitrile anhydre, on a ajouté lentement 508 mg de chlorure d'oxalyle ClCOCOCl (4 mmoles) en solution dans 1 ml de dichlorométhane anhydre. Après 4 heures sous agitation, on a ajouté 2,7 g du sel de potassium de la perfluoro-1-butanesulfonamide $C_4F_9SO_2NHK$ (4 mmoles). Après 48 heures, l'acétonitrile a été évaporé et le résidu recristallisé dans 10 ml d'eau. Après filtration et séchage, on a obtenu 1,81 g du composé suivant :

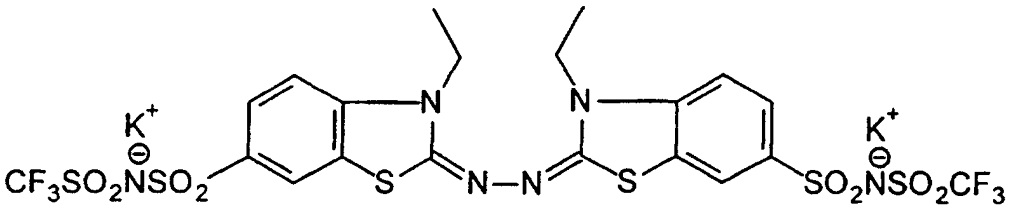

On a préparé le disel de tétraéthylammonium en traitant ce produit par le chlorure de tétraéthylammonium dans l'eau. Le di-sel de tétraéthylammonium a ensuite été récupéré par extraction au dichlorométhane.

Ce composé donne par oxydation un radical et un biradical qui sont des zwitterions stables. En outre, il est utile comme catalyseur d'oxydation entre une phase aqueuse oxygénée et une phase organique non miscible contenant les espèces à oxydées.

**Exemple 63 : Poly(N-2-trifluorométhanesulfonyl-aniline)**

A 21,63 g de 1,2-phénylènediamine $C_6H_4(NH_2)_2$ (200 mmoles), dans 200 ml de dichlorométhane anhydre à -20°C, on a ajouté goutte à goutte 56,42 g d'anhydride trifluorométhanesulfonique $(CF_3SO_2)_2O$ (200 mmoles) en solution dans 50 ml de dichlorométhane anhydre. Après une nuit à -20°C et 4 heures à température ambiante, le dichlorométhane a été évaporé. Le résidu a alors été recristallisé dans 100 ml d'une solution 4 M d'hydroxyde de potassium KOH. Après filtration et séchage, on a récupéré 47,87 g du sel de potassium de la N-2-trifluorométhanesulfonyl-1,2-phénylènediamine (86% de rendement). Ce composé (172 mmoles) a alors été agité dans 86 ml d'une solution 2 M d'acide chlorhydrique. Après 24 heures, on a récupéré après filtration et séchage, 26,85 g (65% de rendement) du sel d'ammonium de la N-2-trifluorométhanesulfonyl-1,2-phénylènediamine $C_6H_4(NSO_2CF_3)$ $(NH_3^+)$. 12,01 g de ce composé (50 mmoles) ont ensuite été dissous dans 200 ml d'eau, on a ajouté 136 mg de nitrate d'argent (800 μmoles), puis la solution a été portée à 0°C. On a préparé également une solution de 11,4 g de persulfate d'ammonium $(NH_4)_2S_2O_8$ (50 mmoles) dans 100 ml d'eau et on a porté cette solution à 0°C. On a alors ajouté sous agitation, la solution de persulfate en quelques minutes à la solution du sel d'aniline. Après une dizaine de minutes, la solution a commencé à se colorer. Après trois heures à une température inférieure à 5°C, la solution a été concentrée à un volume ≈ 100 ml et additionnée de 3,73 g de chlorure de potassium. Le précipité présent dans la solution a alors été récupéré par filtration. Après séchage, on a obtenu 5,9 g d'une poudre noire du composé suivant :

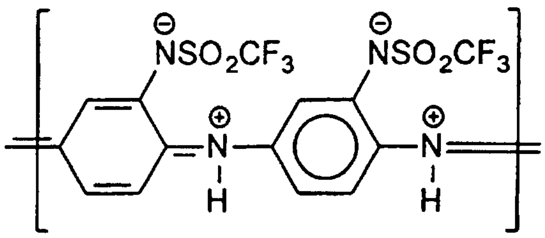

Ce polymère conducteur électronique présente une conductivité électronique déterminée par la méthode des quatres pointes de 8,7 $S.cm^{-1}$. Cette conductivité est stable même lorsque le matériau est exposé à l'air.

**Exemple 64: 1-dodécyl-1,1,1,3,3,3-hexafluoro-2-propanoxysulfonyl(trifluorométhanesulfonyl)imide**

A 18,11 g (100 mmoles) de 6-bromo-1-hexanol et 11,22 g (100 mmoles) de DABCO dans 100 ml de THF anhydre à -20°C, on a ajouté lentement 19,06 g (100 mmoles) de chlorure de tosyle. Après 24 heures sous agitation à -20°C, le milieu réactionnel a été filtré pour éliminer le précipité d'hydrochlorure de DABCO. Après évaporation du solvant, on a récupéré quantitativement le tosylate du 6-bromo-1-hexanol $CH_3\Phi SO_2O(CH_2)_6Br$. Ce composé a ensuite été dissous dans 200 ml de THF avec 40 g d'aniline $\Phi NH_2$ et cette solution portée au reflux pendant une nuit. Après refroidissement, on a ajouté 300 ml d'eau et la phase organique a été extraite avec de l'éther. Après avoir été lavée avec de l'eau, la phase éthérée a été séchée par du sulfate de magnésium. On a obtenu après évaporation et séchage, 23 g de la N-(6-bromohexyl)aniline.

En opérant dans une boîte à gants sous argon, on a mis en solution 18,96 g (50 mmoles) de trifluorométhanesulfonyl-(1,1,1,3,3,3-hexafluoro-2-propanoxysulfonyl)imide, préparé comme à l'exemple 37,, dans 10 ml de tétrahydrofurane anhydre. Après avoir porté cette solution à -20°C, on a ajouté lentement 50 ml d'une solution 1 M dans le tétrahydrofurane de tert-butoxide de potassium $(CH_3)_3COK$ (50 mmoles, commercialisé par Aldrich). Après 15 min, on a ajouté 12,81 g (50 mmoles) de N-(6-bromohexyl)aniline. La réaction s'est poursuivie deux heures à -20°C, puis pendant 24 heures à température ambiante. Après 48 heures sous agitation, le solvant a été évaporé et le résidu recristallisé dans 30 ml d'eau. Après filtration et séchage, on a obtenu le sel de potassium du 1-(6-anilino-1-hexyl)-1,1,1,3,3,3-hexafluoro-2-propanoxysulfonyl(trifluorométhanesulfonyl)imide, ayant une pureté caractérisée par RMN du proton, du carbone et du fluor supérieure à 97%.

12,13 g de ce composé (20 mmoles) ont ensuite été dissous dans 200 ml d'eau, on a ajouté 68 mg de nitrate d'argent (400 µmoles), puis la solution a été portée à 0°C. On a préparé également une solution de 4,56 g de persulfate d'ammonium $(NH_4)_2S_2O_8$ (20 mmoles) dans 100 ml d'eau et on a porté cette solution à 0°C. On a alors ajouté la solution de persulfate sur en minutes à la solution sous agitation du sel d'aniline. Après une dizaine de minutes, la solution a commencé à prendre une couleur bleu-vert. Après trois heures à une température inférieure à 5°C, la solution a été concentrée à un volume ≈ 60 ml, et additionnée de 1,49 g de chlorure de potassium. Le précipité présent dans la solution a alors été récupéré par filtration. Après séchage, on a obtenu 3,9 g d'une poudre noire du composé suivant :

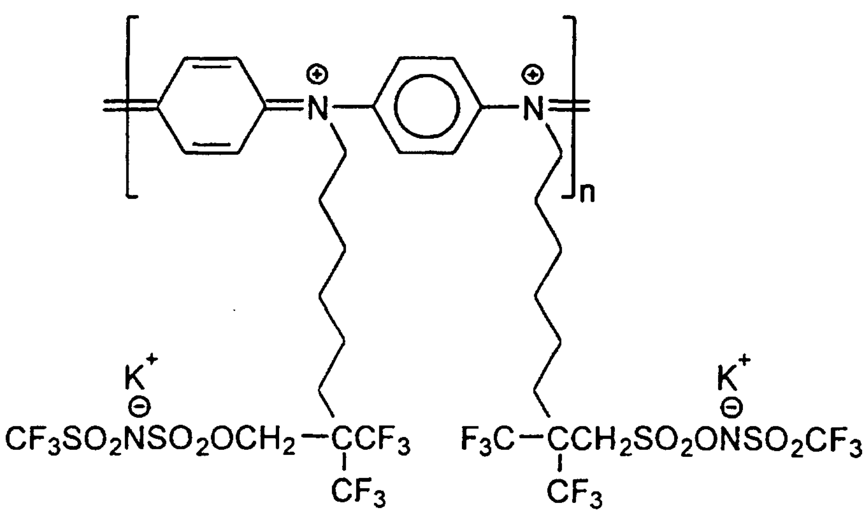

Ce composé polymère qui comprend un anion dopant très délocalisé dans sa structure, présente des propriétés de conducteur électronique (PCE). La faible basicité de cet anion améliore la stabilité du polymère, en particulier en milieu humide. La conductivité déterminée par une mesure quatres points, avant exposition du PCE à une atmosphère humide, était de l'ordre de 4 $S.cm^{-1}$.

On a testé ce matériau comme cathode de batterie. La batterie avait la constitution suivante :

- une cathode composite constituée par 40% en volume du composé polymère obtenu dans le présent exemple et 60% en volume de poly (oxyde d'éthylène) de masse $3.10^5$ ;
- un électrolyte constitué par un film de poly (oxyde d'éthylène) de masse $5.10^6$ contenant le sel de lithium de la trifluorométhanesulfonyl(butanesulfonyl)imide, obtenu à l'exemple 39, à une concentration O/Li = 20/1 ;
- une anode de lithium métallique.

Après assemblage de l'ensemble dans un boîtier de pile bouton, la batterie obtenue a été cyclée à une température de 60°C entre 3 V et 3,9 V. Plus de 1000 cycles de charge/décharge ont pu être effectués en conservant 80% de la capacité du premier cycle.

En outre, le composé polymère du présent exemple est un bon inhibiteur de corrosion des métaux ferreux en milieu acide ou chlorure. Le traitement des surfaces à protéger s'effectue simplement par dépôt d'une solution du PCE dans un mélange d'eau et de diméthylformamide, sous la forme d'une peinture, suivi d'un séchage et d'un traitement thermique à 100°C. Ce composé polymère permet également d'obtenir des dépôts conducteurs adhérents dont la conductivité est stable à l'air sur des plastiques traités par effet Corona.

**Exemple 65 : Poly(2-[2-(3-thiényl)éthoxy]-éthanesulfonyl(trifluorométhanesulfonyl)imide)**

Par un procédé similaire à celui utilisé pour la synthèse du 7,8-octène-3,6-oxa-1-sulfonyl(trifluorométhanesulfonyl)-imide (exemple 15), on a synthétisé, à partir du 2-(3-thiényl)éthanol, le sel de potassium de la 2-[2-(3-thiényl)-éthoxy]-éthanesulfonyl(trifluorométhanesulfonyl)imide. Le produit obtenu a une pureté, déterminée par RMN du carbone et du proton, supérieure à 98%.

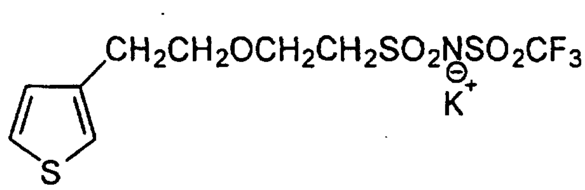

On a préparé 10 ml d'une solution $5.10^{-2}$ M du sel dans l'acétonitrile et l'on a effectué une électropolymérisation dans le compartiment anodique d'une cellule électrochimique sur électrode de platine. On a obtenu un film souple conducteur de :

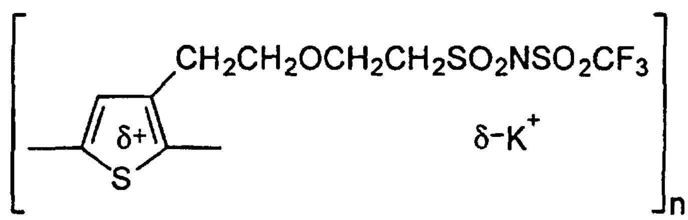

dont le dopage (oxydation) est assuré par échange de cations et d'électrons avec l'extérieur. La conductivité de ce matériau est de l'ordre de 10 $S.cm^{-1}$ et elle est stable à l'atmosphère ambiante et en milieu humide. L'électropolymérisation effectuée en présence de pyrrole non substitué ou possédant des chaînons oxyéthylène en position N ou 3 donne des copolymères également stables dont le changement de couleur peut-être utilisé pour la constitution de systèmes électrochromes.

**Exemple 66 : Polyaniline dopée**

Dans 100 ml d'eau, on a mis en suspension 2,54 g du chlorure de polyaniline (AC&T, St Égrève, France):

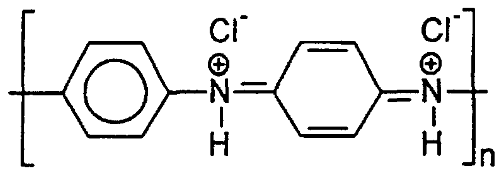

On a alors ajouté 9,81 g du sel de potassium de la trifluorométhanesulfonyl(di-2-éthylhexylaminosulfonyl) imide obtenu à l'exemple 28 :

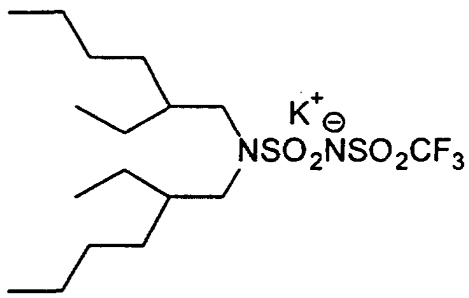

Après 48 heures sous agitation, on a récupéré la polyaniline dopée par la trifluorométhanesulfonyl(di-2-éthylhexylaminosulfonyl)imide. Sous cette forme, elle est soluble dans le toluène. Une solution dans le toluène de la polyaniline dopée a été utilisée pour réaliser un film qui est un polymère conducteur électronique dont la conductivité, mesurée par la méthode des quatres pointes, est de 6 S/cm, avec une bonne stabilité en milieu humide.

On a également réalisé à partir de cette solution un film sur un support de polypropylène (PP) traité par effet Corona. Après séchage sous vide à 60°C pendant 48 heures, on a obtenu un dépôt conducteur et adhèrant de polyaniline d'une épaisseur inférieur au micron. Ce type de traitement sur des plastiques est particulièrement intéressant pour réaliser par des contacteurs électriques souples ou des systèmes de protections électromagnétiques.

**Exemple 67 : Poly(4-styrènesulfonyl (trifluroro méthane sulfonyl) imide)**

20,62 g de poly(4-styrènesulfonate de sodium) ayant une masse moyenne en poids de $10^6$ g/mole (100 mmoles de -$SO_3Na$), (commercialisé par Aldrich) en suspension dans 100 ml de diméthylformamide anhydre ont été traités par 14,08 g (110 mmoles) de chlorure de (chlorométhylène)diméthylammonium (commercialisé par Aldrich) à température ambiante. Après 72 heures, la solution est devenue visqueuse, le poly(chlorure de 4-styrènesulfonyle) passant en solution dans la diméthylformamide. On a alors ajouté au milieu réactionnel 16,4 g (110 mmoles) de trifluorométhanesulfonamide et 24,68 g (220 mmoles) de 1 (DABCO). Après 24 heures, le solvant a été évaporé, le produit obtenu repris dans 50 ml d'eau, puis traité par 8,2 g de chlorure de potassium anhydre. Après 24 heures, le milieu réactionnel a été filtré et le produit ainsi récupéré a été recristallisé dans 50 ml d'eau. On a obtenu après séchage 26,1 g du sel de potassium de la poly(4-styrènesulfonyl(trifluorométhanesulfonyl)imide) (74% de rendement) ayant une pureté caractérisée par RMN du proton et du fluor supérieure à 99%.

Le sel de lithium correspondant a été préparé quantitativement par échange ionique (métathèse) entre le sel de potassium et le chlorure de lithium dans le tétrahydrofurane anhydre.

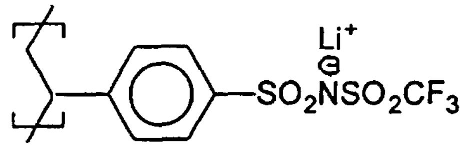

Ce polyélectrolyte est soluble dans la plupart des solvants organiques usuels (tétrahydrofurane, acétonitrile, diméthylformamide, acétate d'éthyle, glymes) et dans les polymères polaires.

En utilisant un cation approprié, ce poyélectrolyte peut être un dopant des polymères conducteurs électroniques conjugués comme le polypyrrole ou la polyaniline.

**Exemple 72 : Copolymère acrylonitrile/4-styrènesulfonyl (trifluorométhanesulfonyl)imide**

Une solution dans 100 ml de tétrahydrofurane anhydre de 19,27 g du sel de lithium de la 4-styrènesulfonyl (trifluorométhanesulfonyl)imide (60 mmoles), de 2,12 g (40 mmoles) d'acrylonitrile et de 100 mg de 1,1'-azobis(cyclohexanecarbonitrile) (1 mmoles) a été dégazée par un balayage d'argon sec. On a alors effectué sous argon la copolymérisation de l'acrylonitrile avec le dérivé du styrène en chauffant le milieu réactionnel à 60°C pendant 48 heures. Après refroidissement, la solution a été concentrée, puis le polymère récupéré par reprécipitation dans l'éther. Après filtration et séchage, on a récupéré 17,54 g du sel de lithium du poly(acrylonitrile-co-4-styrènesulfonyl(trifluorométhanesulfonyl)imide (PANSDTFSI) avec un rendement de 82%.

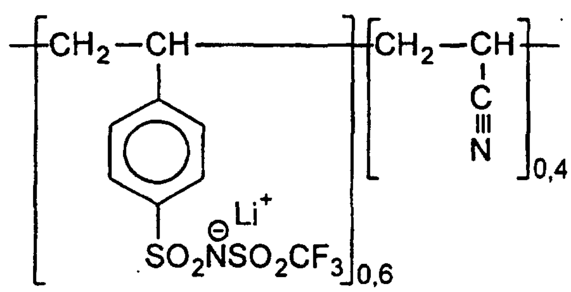

Ce polymère peut être utilisé pour l'élaboration de électrolytes polymères gélifiés à anions fixés, le polymère assurant la double fonctionnalité de matrice permettant d'obtenir le gel et de polyélectrolytes.

On a préparé un électrolyte gélifié constitué par 30% en poids du polyélectrolyte, 35% de carbonate d'éthylène et 35% de carbonate de propylène. Ce gel présente de bonnes propriétés mécaniques et une conductivité de $9,6.10^{-4}$ $S.cm^{-1}$ à 30°C. Le nombre de transport cationique dans cette électrolyte est de 0,85. On a assemblé un générateur électrochimique comprenant une anode constituée par un coke de carbone (80% en volume) mélangé avec le copolymère (PANSDTFSI) comme liant (20% en volume), l'électrolyte gélifié précité, et une cathode composite constituée par du noir de carbone (6% en volume), $LiNiO_2$ (75% en volume) et le copolymère (PANSDTFSI) (20% en volume). Ce générateur présente de bonnes performances en cyclage à 25°C (1 000 cycles de charge/décharge entre 3 et 4,2 V en conservant une capacité supérieure à 80% de la capacité au premier cycle). Il a également de très bonnes performances lors d'appel de puissance du fait de l'utilisation d'anions fixés. L'utilisation d'anions fixés a également permis d'améliorer l'évolution de la résistance d'interface.

**Exemple 73 : Copolymère acrylonitrile/4-styrènesulfonyl (trifluorométhanesulfonyl)imide**

Suivant un procédé similaire à celui utilisé dans l'exemple 76, on a synthétisé un copolymère d'acrylonitrile (3% en moles) et du sel de lithium de la 4-styrènesulfonyl(trifluorométhanesulfonyl)imide (97% en moles).

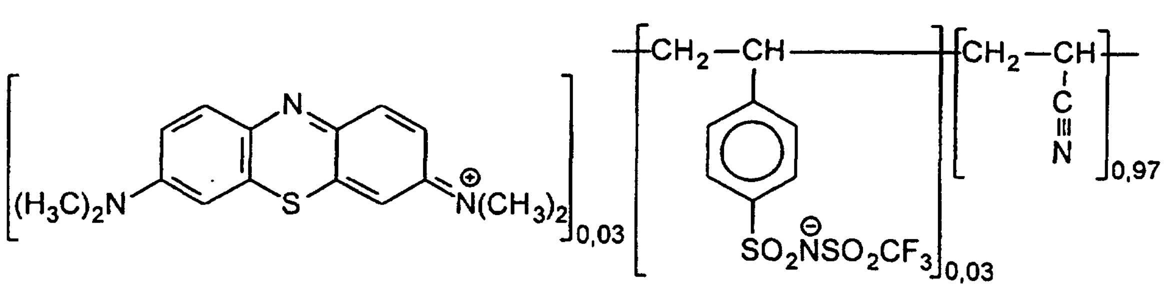

Ce copolymère présente des propriétés antistatiques, contrairement au polyacrylonitrile (PAN) qui, sous la forme du sel d'alcalin ou d'ammonium, est largement utilisé sous forme de fibre pour le textile. De plus, le filage de ce

copolymère est plus aisé que celui du PAN non modifié.

Le copolymère présente de très bonnes interactions avec les colorants cationiques comme le bleu de méthylène, ce qui en fait un matériau d'intérêt pour les fibres textiles colorées, La stabilité de la couleur étant nettement améliorée par rapport au copolymère classique d'acrylonitrile et de méthallylsulfonate.

**Exemple 74 : Copolymère fluorure de vinylidène/2,2-fluorovinylsulfonyl(trifluorométhanesulfonyl)imide**

Dans un réacteur chimique, on a introduit une solution dans 100 ml de tétrahydrofurane anhydre de 8,43 g (30 mmoles) de 2,2-fluorovinylsulfonyl(trifluorométhanesulfonyl)imide obtenu à l'exemple 24 et 100 mg de 1,1'-azobis (cyclohexanecarbonitrile). Après avoir purgé le réacteur sous argon, on a introduit à l'aide d'un sas 4,48 g de fluorure de vinylidène $CF_2CH_2$ (70 mmoles, commercialisé par l'Air Liquide). On a alors effectué sous argon la copolymérisation en chauffant le milieu réactionnel à 60°C pendant 48 heures. Après refroidissement, la solution a été concentrée, puis le polymère récupéré par reprécipitation dans l'éther. Après filtration et séchage, on a récupéré 10,2 g du sel de lithium du poly(fluorure de vinylidène-co-2,2-éthanesulfonyl(trifluorométhanesulfonyl)imide (PFVESTFSI) avec un rendement de 79%.

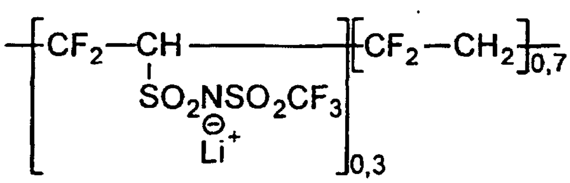

Ce polymère permet de réaliser des électrolytes polymères gélifiés à anions fixés, le polymère assurant la double fonctionnalité de matrice permettant d'obtenir le gel et de polyélectrolytes.

On a réalisé une batterie du même type que celle décrite à l'exemple 72 et on a obtenu des performances analogues

**Exemple 75 : Copolymère AGE/Epoxy-demiTFSI/OE**

Dans un réacteur chimique, on a introduit une solution dans 100 ml de tétrahydrofurane anhydre de 15,37 g (50 mmoles) du sel de potassium du 3,4-époxybutane-1-sulfonyl(trifluorométhanesulfonyl)imide, préparé comme à l'exemple 13, et de 685 mg (6 mmoles) d'allylglycidyléther. Après avoir purgé le réacteur avec de l'argon, on a introduit à l'aide d'un sas 6,34 g (146 mmoles) de 1,2-époxyde puis 100 µl d'une solution $10^{-2}$ M de t-butoxyde de potassium dans le THF. On a alors effectué sous argon la polymérisation en chauffant le milieu réactionnel à 60°C pendant 48 heures. Après refroidissement, la solution a été concentrée, puis le polymère récupéré par reprécipitation dans l'éther. Après filtration et séchage, on a récupéré 15,9 g (71% de rendement) du sel de potassium du poly(oxyde d'éthylène-co-3,4-époxybutanesulfonyl(trifluorométhanesulfonyl)imide-co-allylglycidyléther).

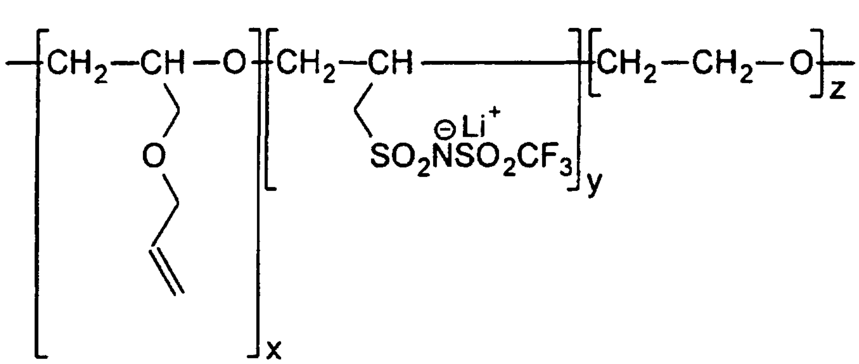

Ce polymère permet de réaliser des électrolytes polymères ou gélifiés à anions fixés, le polymère assurant la double fonctionnalité de matrice permettant d'obtenir le gel et de polyélectrolytes. Il peut être réticulé au cours du processus de fabrication du système électrochimique qui le contient.

On a réalisé avec ce polyélectrolyte une batterie analogue à celle décrite à l'exemple 22, qui a donné des

performances similaires.

**Exemple 76 : Polysiloxane à anions fixés**

Dans un ballon tricol équipé d'un réfrigérant, d'une agitation mécanique et d'une entrée de gaz neutre (Argon), 9,5 g d'un copolymère de diméthylsiloxane et d'(hydrogéno) (méthyl)-siloxane (HMS 301 25% SiH, $M_w$ 1900 Gelest Inc., Tullytown, PA, USA) ont été mis en solution dans du tétrahydrofurane ; on a alors ajouté 9,13 g du sel de lithium de la vinylsulfonyl(trifluorométhanesulfonyl)imide et 70 mg d'acide chloroplatinique $H_2PtCl_6$. Le mélange a été chauffé au reflux pendant 4 heures. Le polymère a ensuite été reprécipité dans l'éthanol.

On a ainsi obtenu le copolymère de diméthylsiloxane et du sel de lithium du (N-trifluorométhanesulfonyl-éthylsulfonamide)(méthyl)-siloxane.

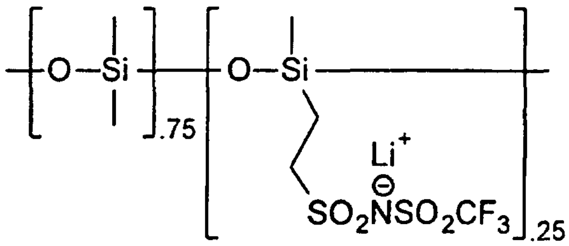

Ce polymère est soluble dans la plupart des solvants organiques, y compris à des teneurs > 2% dans les huiles ou les matériaux siliconés, leur conférant ainsi des propriétés antistatiques.

**Exemple 77 : Batterie Li/POE/$V_2O_5$**

Le sel de lithium de la diméthylaminosulfonyl(trifluorométhanesulfonyl)imide, préparé selon l'exemple 25, a été testé dans un générateur électrochimique selon la technologie lithium-polymère. Le générateur a été réalisé en superposant les couches suivantes :

- un collecteur de courant en acier inoxydable ayant une épaisseur de 2 mm ;
- une cathode constituée par une pastille d'un film de matériau composite ayant une épaisseur de 89 µm et constitué par du dioxyde de vanadium (45% en volume), du noir de Shawinigan (5% en volume) et un polyoxyde d'éthylène de masse Mw = 3.105 (50% en volume) ;
- un électrolyte constitué par une pastille d'un film de polyoxyde d'éthylène de masse Mw = 5.106 contenant le sel de lithium de la diméthylaminosulfonyl(trifluorométhanesulfonyl)-imide à une concentration O/Li = 15/1 ;
- une anode constituée par une feuille de lithium métallique ayant une épaisseur de 50µm ;
- un collecteur de courant analogue au collecteur précité.

Les pastilles constituant les électrodes et l'électrolyte ont été découpées en boîte à gants et empilées dans l'ordre indiqué ci-dessus. Les collecteurs ont ensuite été déposés de part et d'autre de l'empilement obtenu.

On a scellé le tout dans un boîtier de pile bouton, qui permet à la fois de protéger le générateur de l'atmosphère et d'excercer une contrainte mécanique sur les films. La batterie a alors été placée dans une enceinte sous argon installée dans une étuve à une température de 60°C. Elle a ensuite été cyclée entre 1,8 et 3,3 V à un régime de charge et de décharge de C/10 (capacité nominale chargée ou déchargée en 10 heures).

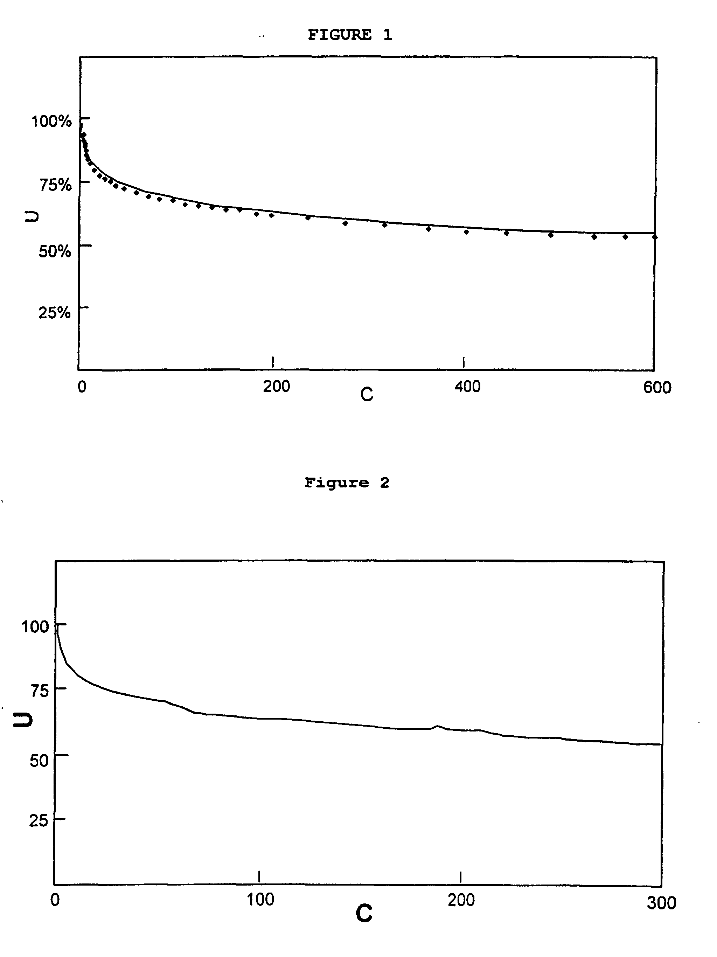

La courbe de cyclage est donnée sur la figure 1. Sur cette figure, l'utilisation, U, exprimée en % est donnée en ordonnée, et le nombre de cycles C est donné en abscisse.

**Exemple 78 : Extrusion**

On a introduit dans une extrudeuse Warner & Pfilder sous atmosphère d'argon, du poly (oxyde d'éthylène) de masse $M_w = 10^5$ sous forme de pastilles de 2mm de diamètre et un mélange de sel de lithium de la dodécylsulfonyl (trifluorométhanesulfonyl)imide préparé selon un procédé analogue à celui de l'exemple 39, de sel de potassium de l'Igepal® CA-520-propylsulfonyl(trifluorométhanesulfonyl)imide préparé selon un procédé analogue à celui de l'exemple 43, d'oxyde de vanadium $V_2O_5$ broyé en grains de taille inférieure à 5 µm et de noir de Shawinigan. L'ensemble des composants ont été introduits dans des proportions telles que l'oxyde de vanadium représente 40% du volume total, le noir de Shawinigan 5%, le sel de potassium de l'Igepal® CA-520-propylsulfonyl(trifluorométhanesulfonyl)imide

2%, et l'ensemble poly (oxyde d'éthylène) /sel de lithium de la dodécylsulfonyl(trifluorométhanesulfonyl)imide 53%, le sel de lithium étant à une concentration O/Li = 15/1. L'ensemble a alors été extrudé à une température de 100°C sous la forme d'une bande de 14 cm de large et d'une épaisseur de 63 µm. Ce film utilisable comme cathode, a été directement déposé sur une feuille d'acier inoxydable de 8 µm d'épaisseur.

Ce film de cathode composite a lui-même été recouvert par un film d'électrolyte de 30 µm d'épaisseur obtenu par extrusion d'un mélange de poly (oxyde d'éthylène) de masse $M_w = 3.10^5$ et de sel de lithium de la dodécylsulfonyl-(trifluorométhanesulfonyl)imide à une concentration O/Li = 45/1.

L'ensemble a ensuite été laminé avec un film de lithium de 20 µm d'épaisseur. On a ainsi obtenu un générateur électrochimique selon la technologie lithium-polymère.

La courbe de cyclage de ce générateur à un régime de charge et de décharge de C/10 est représentée sur la figure 2. Sur cette figure, l'utilisation, U, exprimée en % est donnée en ordonnée, et le nombre de cycles C est donné en abscisse.

Les sels de la présente invention contenant de longues chaînes alkyles tels que le sel de potassium de l'Igepal® CA-520-propylsulfonyl(trifluorométhanesulfonyl)imide ou le sel de lithium de la dodécylsulfonyl(trifluorométhanesulfonyl)-imide, permettent de plastifier le poly (oxyde d'éthylène). Ils rendent ainsi plus aisée l'extrusion des films de cathodes ou d'électrolytes utilisés lors de la fabrication de batteries selon la technologie lithium-polymère en film mince. Leur stabilité électrochimique permet également d'obtenir de bonnes performances lors du cyclage de ces batteries.

## Revendications

1. Matériau à conduction ionique comprenant un composé ionique en solution dans un solvant, ledit composé ionique étant constitué par une amide ou l'un de ses sels, comprenant une partie anionique associée à au moins une partie cationique $M^{+m}$ en nombre suffisant pour assurer la neutralité électronique de l'ensemble, ledit matériau étant **caractérisé en ce que** le cation $M^{+m}$ est un hydroxonium, un nitrosonium $NO^+$, un ammonium $-NH_4^+$, un cation métallique ayant la valence m, un cation organique onium ayant la valence m ou un cation organo-métallique ayant la valence m et **en ce que** la partie anionique répond à la formule $R_F\text{-}SO_x\text{-}N^-\text{-}Z$
dans laquelle :

- le groupement $-S(O)_x-$ représente un groupement sulfonyle $-SO_2-$ ou un groupement sulfinyle -SO- ;
- $R_F$ est un radical perhalogéné alkyle, alkylaryle, oxa-alkyle, aza-alkyle ou thia-alkyle, ou un radical répondant à l'une des formules $R_ACF_2-$, $R_ACF_2CF_2-$, $R_ACF_2CF(CF_3)-$ ou $CF_3C(R_A)F-$ dans lesquelles $R_A-$ représente un radical organique non perhalogéné choisi parmi les radicaux alkyle, aryle, alkylaryle ou arylalkyle ; les groupes mésomorphes ou les groupes comprenant au moins une insaturation éthylénique et/ou un groupe condensable et/ou un groupe dissociable par voie thermique, par voie photochimique ou par dissociation ionique ; les polymères conducteurs électroniques autodopés ; les alcoxysilanes hydrolysables ; les dipôle dissociants ; les couples rédox ; les ligands complexants ; les groupes chromophores ; les chaînes polymériques portant des greffons comportant un groupe carbonyle, un groupe sulfonyle, un groupe thionyle ou un groupe phosphonyle ; les zwitterions ; les acides aminés ou les polypeptides optiquement ou biologiquement actifs ; les groupements chiraux ;
- Z représente un radical électro-attracteur ayant un paramètre de Hammett au moins égal à celui d'un radical phényle, choisi parmi :

  j) -CN, $-NO_2$, -SCN, $-N_3$, $-CF_3$, $R'_FCH_2-$ ($R'_F$ étant un radical perfluoré), les radicaux fluoroalkylthioxy,
  jj) les radicaux comprenant un ou plusieurs noyaux aromatiques contenant éventuellement au moins un atome d'azote, d'oxygène, de soufre ou de phosphore, lesdits noyaux pouvant être éventuellement des noyaux condensés et/ou lesdits noyaux pouvant éventuellement porter au moins un substituant choisi parmi les halogènes, -CN, $-NO_2$, -SCN, $-N_3$, $-CF_3$, $CF_3CH_2-$, $CF_2=CF-O-$, $CF_2=CF-S-$, les groupes perfluoroalkyles, les groupes fluoroalkyloxy, les groupes fluoroalkylthioxy, les radicaux alkyles, alkényles, oxa-alkyles, oxa-alkényles, aza-alkyles, aza-alkényles, thia-alkyles, thia-alkényles, les radicaux polymères, les radicaux possédant au moins un groupement ionophore cationique et/ou au moins un groupement ionophore anionique ;
  étant entendu qu'un substituant Z peut être un radical monovalent, une partie d'un radical multivalent portant plusieurs groupements $R_F\text{-}S(O)_x\text{-}N-$, ou un segment d'un' polymère ;

  ou bien
- Z est un radical $R_D\text{-}Y-$ dans lequel Y est un groupe sulfonyle ou sulfinyle, et $R_D$ est un radical choisi dans le

groupe constitué par :

a) les radicaux alkyle ou alkényle, les radicaux aryle, arylalkyle, alkylaryle ou alkénylaryle, les radicaux alicycliques ou hétérocycliques, y compris les radicaux polycycliques ;
b) les radicaux alkyle ou alkényle comprenant au moins un groupe fonctionnel éther, thioéther, amine, imine, carboxyle, carbonyle, hydroxy, silyle, isocyanate ou isothiocyanate ;
c) les radicaux aryle, arylalkyle, arylalkényle, alkylaryle ou alkénylaryle, dans lesquels les noyaux aromatiques et/ou au moins un substituant du noyau comprennent des hétéroatomes tels que l'azote, l'oxygène, le soufre ;
d) les radicaux comprenant des cycles aromatiques condensés qui comprennent éventuellement au moins un hétéroatome choisi parmi l'azote, l'oxygène, le soufre ;
e) les radicaux halogénés alkyle, alkényle, aryle; arylalkyle, alkylaryle ou alkénylaryle dans lesquels le nombre d'atomes de carbone portant au moins un halogène est au plus égal au nombre d'atomes de carbone non halogénés, le carbone en $\alpha$ du groupement Y n'étant pas halogéné lorsque Y est $-SO_2-$, lesdits radicaux comprenant éventuellement des groupes fonctionnels éther, thioéther, amine, imine, carboxyle, carbonyle, hydroxy, silyle, isocyanate ou isothiocyanate ;
f) les radicaux $R_cC(R')(R'')-O-$ dans lesquels $R_c$ est un radical alkylperfluoré et R' et R" sont indépendamment l'un de l'autre un atome d'hydrogène ou un radical tel que défini en a), b), c) ou d) ci-dessus ;
g) les radicaux $(R_B)_2N-$, dans lesquels les radicaux $R_B$ identiques ou différents sont tels que définis en a), b), c), d) et e) ci-dessus, l'un des $R_B$ pouvant être un atome d'hydrogène, ou bien les deux radicaux $R_B$ forment ensemble un radical divalent qui forme un cycle avec N ;
h) les radicaux constitués par une chaîne polymère,
i) les radicaux possédant un ou plusieurs groupements ionophores cationiques et/ou un ou plusieurs groupements ionophores anioniques ;

- étant entendu qu'un substituant $R_D$ peut être un radical monovalent, une partie d'un radical multivalent portant plusieurs groupements $R_FS(O)_x-N-Y-$, ou un segment d'un polymère ;
- étant entendu que, lorsque Y est un sulfonyle et que $R_D$ est un radical tel que défini en a), $R_F$ est $R_ACF_2-$, $R_ACF_2CF_2-$, $R_ACF_2CF(CF_3)-$, $CF_3C(R_A)F-$ ou un radical perhaloalkyle ayant de 1 à 2 atomes de carbone ;
- étant entendu que lorsque qu'un radical est un polymère ou partie d'un polymère, ledit polymère est un polyalkylène portant éventuellement des substituants, un polystyrène, un poly(fluoroéthylène), un polyacrylate, un polyester, un poly(oxyalkylène), un poly(alcoxysilyle), une polyaniline, un polymère ayant des unités récurrentes dérivées de 2[2-(3-thiényl)éthoxy]éthyle ou de 3-maléimidopropyle.

**2.** Matériau à conduction ionique selon la revendication 1, **caractérisé en ce que** le cation organique onium $M^{+m}$ est choisi dans le groupe constitué par les cation $R_3O^+$ (oxonium), $NR_4^+$ (ammonium), $RC(NHR_2)_2^+$ (amidinium), $C(NHR_2)_3^+$ (guanidinium), $C_5R_6N^+$ (pyridinium), $C_3R_5N_2^+$ (imidazolium), $C_2R_4N_3^+$ (triazolium), $C_3R_7N_2^+$ (imidazolinium), $SR_3^+$ (sulfonium), $PR_4^+$ (phosphonium), $IR_2^+$ (iodonium), $(C_6R_5)_3C^+$ (carbonium), les radicaux R représentant de manière indépendante un H ou un radical choisi dans le groupe constitué par :

- les radicaux alkyles, alkényles, oxa-alkyles, oxa-alkényles, aza-alkyles, aza-alkényles, thia-alkyles, thia-alkényles, sila-alkyles, sila-alkényles, aryles, arylalkyles, alkylaryles, alkénylaryles, les radicaux dialkylamino et les radicaux dialkylazo ;
- les radicaux cycliques ou hétérocycliques comprenant éventuellement au moins une chaîne latérale comprenant des hétéroatomes tels que l'azote, l'oxygène, le soufre ;
- les radicaux cycliques ou hétérocycliques comprenant éventuellement des hétéroatomes dans le noyau aromatique ;
- les groupes comprenant plusieurs noyaux aromatiques ou hétérocycliques, condensés ou non, contenant éventuellement au moins un atome d'azote, d'oxygène, de soufre ou de phosphore ;

étant entendu que plusieurs radicaux R peuvent former ensemble des cycles aromatiques ou non, englobant éventuellement le centre portant la charge cationique.

**3.** Matériau à conduction ionique selon la revendication 2, **caractérisé en ce que** le cation onium fait partie du radical Z ou du radical $R_D$.

**4.** Matériau à conduction ionique selon la revendication 2, **caractérisé en ce que** le cation onium fait partie d'une unité récurrente d'un polymère.

**5.** Matériau à conduction ionique selon la revendication 2, **caractérisé en ce que** le cation onium est un hétérocycle cationique à caractère aromatique, comportant au moins un atome d'azote alkylé dans le cycle.

**6.** Matériau à conduction ionique selon la revendication 5, **caractérisé en ce que** le cation onium est un imidazolium, un triazolium, un pyridinium, un 4-diméthylamino-pyridinium, lesdits cations portant éventuellement un substituant sur les atomes de carbone du cycle.

**7.** Matériau à conduction ionique selon la revendication 2, **caractérisé en ce que** le cation onium est un groupement possédant une liaison -N=N-, $-N=N^{+}$, un groupement sulfonium, un groupement iodonium, ou un cation arène-ferrocénium substitué ou non, éventuellement incorporé dans une trame polymérique.

**8.** Matériau à conduction ionique selon la revendication 2, **caractérisé en ce que** le cation onium est un cation diaryliodonium, un cation dialkylaryliodonium, un cation triarylsulfonium, un cation trialkylaryle sulfonium, ou un cation phénacyl-dialkyl sulfonium substitué ou non.

**9.** Matériau à conduction ionique selon la revendication 8, **caractérisé en ce que** le cation onium fait partie d'une. chaîne polymère.

**10.** Matériau à conduction ionique selon la revendication 2, **caractérisé en ce que** M est un cation organique qui incorpore un groupement 2,2'[Azobis(2-2'-imidazolinio-2-yl)propane]$^{2+}$ ou 2,2'-Azobis(2-amidiniopropane)$^{2+}$.

**11.** Matériau à conduction ionique selon la revendication 1, **caractérisé en ce que** le cation M est un cation métallique choisi dans le groupe constitué par les cations de métaux alcalins, les cations de métaux alcalino-terreux, les cations de métaux de transition, les cations de métaux trivalents, et les cations de terres rares.

**12.** Matériau à conduction ionique selon la revendication 1, **caractérisé en ce que** le cation organo-métallique est un métallocénium, choisi dans le groupe constitué par les cations dérivés du ferrocène, du titanocène, du zirconocène, les cations indénocénium, les cations arène métallocénium, les cations des métaux de transition complexés par des ligands de type phosphine possédant éventuellement une chiralité et les cations organométalliques possédant un ou plusieurs groupements alkyles ou aryles fixés d'une manière covalente à un atome ou un groupe d'atome, lesdits cations faisant éventuellement partie d'une chaîne polymère.

**13.** Matériau à conduction ionique selon la revendication 1, **caractérisé en ce que** $R_F$ est un atome de fluor ou un radical alkyle perhalogéné ayant de 1 à 12 atomes de carbone, ou un radical alkylaryle perhalogéné ayant de 6 à 9 atomes de carbone.

**14.** Matériau à conduction ionique selon la revendication 1, **caractérisé en ce que** Z est un groupe $R_D$-$SO_2$-.

**15.** Matériau à conduction ionique selon la revendication 1, **caractérisé en ce que** $R_D$ est choisi parmi les radicaux alkyle, alkényle, oxa-alkyle, oxa-alkényle, aza-alkyle, aza-alkényle, thia-alkyle ou thia-alkényle ayant de 1 à 24 atomes de carbone, ou parmi les radicaux aryle, arylalkyle, alkylaryle ou alkénylaryle qui ont de 5 à 24 atomes de carbone et dans lesquels les chaînes latérales et/ou les noyaux aromatiques comprennent des hétéroatomes;

**16.** Matériau à conduction ionique selon la revendication 1, **caractérisé en ce que** $R_D$ est choisi parmi les radicaux alkyles ou alkényles qui ont de 1 à 12 atomes de carbone, qui comprennent éventuellement au moins un hétéroatome O, N ou S dans la chaîne principale ou dans une chaîne latérale, et/ou qui portent éventuellement un groupe hydroxy, un groupe carbonyle, un groupe aminé, un groupe carboxyle, un groupe isocyanate ou un groupe isothiocyanate.

**17.** Matériau à conduction ionique selon la revendication 1, **caractérisé en ce que** $R_D$ est partie d'un radical poly (oxyalkylène) ou d'un radical polystyrène.

**18.** Matériau à conduction ionique selon la revendication 1, **caractérisé en ce que** $R_D$ est un radical possédant un groupement iodonium, un groupement sulfonium, oxonium, ammonium, amidinium, triazolium, guanidinium, pyridinium, imidazolium, phosphonium ou carbonium, ledit groupement ionique jouant totalement ou partiellement le rôle du cation $M^{+m}$.

**19.** Matériau à conduction ionique selon la revendication 1, **caractérisé en ce que** $R_D$ possède un ou plusieurs groupements ionophores anioniques constitués par une fonction carboxylate ($-CO_2^-$), une fonction sulfonate ($-SO_3^-$), une fonction sulfonimide ($-SO_2NSO_2-$) ou une fonction sulfonamide ($-SO_2N-$).

**20.** Matériau à conduction ionique selon l'une des revendications 1, **caractérisé en ce que** $R_D$ comporte au moins une insaturation éthylénique et/ou un groupe condensable et/ou un groupe dissociable par voie thermique, par voie photochimique ou par dissociation ionique.

**21.** Matériau à conduction ionique selon l'une des revendications 1, **caractérisé en ce que** $R_D$ représente un groupe mésomorphe ou un groupe chromophore ou un polymère conducteur électronique autodopé ou un alcoxysilane hydrolysable.

**22.** Matériau à conduction ionique selon la revendication 1, **caractérisé en ce que** Z représente une unité récurrente d'une chaîne polymère.

**23.** Matériau à conduction ionique selon la revendication 1, **caractérisé en ce que** $R_D$-Y- est optiquement actif.

**24.** Matériau à conduction ionique selon la revendication 1, **caractérisé en ce que** $R_D$-Y- représente un acide aminé, ou un polypeptide optiquement ou biologiquement actif.

**25.** Matériau à conduction ionique selon la revendication 1, **caractérisé en ce que** $R_D$ représente un radical ayant une valence v supérieure à deux, comportant lui-même au moins un groupe $R_F-S(O)_x-N-$.

**26.** Matériau à conduction ionique selon la revendication 1, **caractérisé en ce que** le substituant Z est choisi dans le groupe constitué par $-OC_nF_{2n+1}$, $-OC_2F_4H$, $-SC_nF_{2n+1}$ et $-SC_2F_4H$, $-OCF=CF_2$, $-SCF=CF_2$ et $C_nF_{2n+1}CH_2-$ n étant un nombre entier de 1 à 8.

**27.** Matériau à conduction ionique selon la revendication 1, **caractérisé en ce que** Z comprend un hétérocycle, dérivé de la pyridine, de la pyrazine, de la pyrimidine, de l'oxadiazole ou du thiadiazole, fluoré ou non.

**28.** Matériau à conduction ionique selon la revendication 1, **caractérisé en ce que** le substituant $R_D$ du composé ionique comprend un groupe alkyle, un groupe aryle, un groupe alkylaryle ou un groupe arylalkyle ; un groupe mésomorphe ou un groupe comprenant au moins une insaturation éthylénique et/ou un groupe condensable et/ou un groupe dissociable par voie thermique, par voie photochimique ou par dissociation ionique ; un polymère conducteur électronique autodopé ; un alcoxysilane hydrolysable ; un piège à radicaux libres ; un dipôle dissociant ; un couple rédox ; un ligand complexant.

**29.** Matériau selon la revendication 1, **caractérisé en ce que** le substituant $R_D$ est une unité récurrente d'un polymère.

**30.** Matériau à conduction ionique selon la revendication 1, **caractérisé en ce que** le solvant est soit un solvant liquide aprotique, choisi parmi les éthers linéaires et les éthers cycliques, les esters, les nitriles, les dérivés nitrés, les amides, les sulfones, les sulfolanes, les alkylsulfamides et les hydrocarbures partiellement halogénés, soit un polymère polaire, soit un de leurs mélanges.

**31.** Matériau à conduction ionique selon la revendication 1, **caractérisé en ce que** le solvant est un polymère solvatant, réticulé ou non, portant ou non des groupements ioniques greffés.

**32.** Matériau à conduction ionique selon la revendication 31, **caractérisé en ce que** le polymère solvatant est choisi parmi les polyéthers de structure linéaire, peigne ou à blocs, formant ou non un réseau, à base de poly(oxyde d'éthylène), les copolymères contenant le motif oxyde d'éthylène ou oxyde de propylène ou allylglycidyléther, les polyphosphazènes, les réseaux réticulés à base de polyéthylène glycol réticulé par des isocyanates, les réseaux obtenus par polycondensation et portant des groupements qui permettent l'incorporation de groupements réticulables et les copolymères à blocs dans lesquels certains blocs portent des fonctions qui ont des propriétés rédox.

**33.** Matériau à conduction ionique selon la revendication 1, **caractérisé en ce que** le solvant est constitué essentiellement par un solvant liquide aprotique et un solvant polymère polaire comprenant des unités contenant au moins un hétéroatome choisi parmi le soufre, l'oxygène, l'azote et le fluor.

**34.** Matériau à conduction ionique selon la revendication 33, **caractérisé en ce que** le polymère polaire contient

principalement des unités dérivées de l'acrylonitrile, du fluorure de vinylidène, de la N-vinyipyrrolidone ou du méthacrylate de méthyle.

**35.** Matériau à conduction ionique selon la revendication 1, **caractérisé en ce qu'**il contient en outre un second sel.

**36.** Matériau à conduction ionique selon la revendication 1, **caractérisé en ce qu'**il contient en outre une charge minérale ou organique sous forme de poudre ou de fibres.

**37.** Générateur électrochimique comprenant une électrode négative et une électrode positive séparées par un électrolyte, **caractérisé en ce que** l'électrolyte est un matériau selon l'une des revendications 1 à 36.

**38.** Générateur selon la revendication 37, **caractérisé en ce que** l'électrode négative est constituée par du lithium métallique, ou par l'un de ses alliages, éventuellement sous forme de dispersion manométrique dans de l'oxyde de lithium, ou par un nitrure double de lithium et d'un métal de transition, ou par un oxyde à bas potentiel ayant pour formule générale $Li_{1+y+x/3}Ti_{2-x/3}O_4$ ($0 \leq x \leq 1$, $0 \leq y \leq 1$), ou par le carbone et les produit carbonés issus de la pyrolyse de matières organiques.

**39.** Générateur selon la revendication 37, **caractérisé en ce que** l'électrode positive est choisie parmi les oxydes de vanadium $VO_x$ ($2 \leq x \leq 2{,}5$), $LiV_3O_8$, $Li_yNi_{1-x}CO_xO_2$, ($0 \leq x \leq 1$ ; $0 \leq y \leq 1$), les spinelles de manganèse $Li_yMn_{1-x}M_xO_2$ (M = Cr, Al, V, Ni, $0 \leq x \leq 0{,}5$ ; $0 \leq y \leq 2$), les polydisulfures organiques, FeS, $FeS_2$, le sulfate de fer $Fe_2(SO_4)_3$, les phosphates et phosphosilicates de fer et de lithium de structure olivine, ou leurs produits de substitution du fer par le manganèse, utilisés seuls ou en mélanges.

**40.** Générateur selon la revendication 37 , **caractérisé en ce que** le collecteur de la cathode est en aluminium.

**41.** Supercapacité utilisant au moins une électrode de carbone à haute surface spécifique, ou une électrode contenant un polymère rédox, dans laquelle l'électrolyte est un matériau selon l'une des revendications 1 à 36.

**42.** Utilisation d'un matériau selon l'une des revendications 1 à 36 pour le dopage p ou n d'un polymère à conduction électronique.

**43.** Dispositif électrochrome, dans lequel l'électrolyte est un matériau selon l'une des revendications 1 à 36.

**44.** Matériau à conduction électronique, **caractérisé en ce qu'**il comprend un matériau à conduction ionique selon la revendication 1.

**45.** Matériau à conduction électronique selon la revendication 44, **caractérisé en ce que** la partie cationique du composé ionique est un polycation constitué par un polymère conjugué dopé "p".

**46.** Matériau à conduction électronique selon la revendication 44, **caractérisé en ce que** le substituant Z du composé ionique comprend une chaîne alkyle ayant de 6 à 20 atomes de carbone.

## Claims

**1.** An ionicially conductive material comprising an ionic compound in solution in a solvent, said compound being an amide or a salt thereof, comprising at least one anionic part associated to at least one cationic part $M^{+m}$ in sufficient number to ensure the electronic neutrality of the whole, said material being **characterized in that** $M^{+m}$ is a hydroxonium, a nitrosonium $NO^+$, an ammonium $-NH_4^+$, a metallic cation having a valency m, an organic cation having a valency m or an organometallic cation having a valency m, and **in that** the anionic part corresponds to the formula $R_F$-$SO_x$-$N^-$-Z in which :

- the $-S(O)_x-$ group represents a sulfonyl group $-SO_2-$ or a sulfinyl group -SO- ;
- $R_F$ is a perhalogenated alkyl, alkylaryl, oxa-alkyl, aza-alkyl or thia-alkyl radical, or a radical corresponding to one of the formulae $R_ACF_2$-, $R_ACF_2CF_2$-, $R_ACF_2CF(CF_3)$- or $CF_3C(R_A)F$- in which $R_A$- represents a non-perhalogenated organic radical selected from alkyl, aryl, alkylaryl or arylalkyl groups ; mesomorphous groups or groups comprising at least one ethylenic unsaturation and/or a condensable group and/or a group which is dissociable by thermal means or by photochemical means or via ionic dissociation ; electronically conducting

autodoped polymers ; hydrolyzable alkoxysilanes ; dissociating dipoles ; redox couples ; complexing ligands ; chromophorous groups ; polymeric chains bearing grafts having a carbonyl group, a sulfonyl group, a thionyl group or a phosphonyl group ; zwitterions ; amino acids or optically or biologically active polypeptides ; chiral groups ;

- Z represents an electro-attractor radical having a Hammett parameter at least equal to that of a phenyl radical, selected from :

  j) -CN, $-NO_2$, -SCN, $-N_3$, $-CF_3$, $R'_F-CH_2-$ ($R'_F$ being a perfluorinated radical), fluoroalkylthioxy radicals,
  jj) radicals comprising one or more aromatic nuclei optionally containing at least one nitrogen, oxygen, sulfur or phosphorus atom, said nuclei optionally being condensed nuclei and/or said nuclei optionally carrying at least one substituent selected from halogens, -CN, $-NO_2$, -SCN, $-N_3$, $-CF_3$, $CF_3CH_2-$, $CF_2$=CF-O-, $CF_2$=CF-S-, perfluoroalkyl groups, fluoroalkyloxy groups, fluoroalkylthioxy groups, alkyl, alkenyl, oxa-alkyl, oxa-alkenyl, aza-alkyl, aza-alkenyl, thia-alkyl, thia-alkenyl radicals, polymer radicals, radicals having at least one cationic ionophoric group and/or at least one anionic ionophoric group ;
  with the proviso that a substituent Z may be a monovalent radical, part of a multivalent radical carrying a plurality of groups $R_F-S(O)_x-N-$, or a polymer segment ;
  or

- Z is a radical $R_D$-Y- in which Y is a sulfonyl or sulfinyl group and $R_D$ is a radical selected from the group consisting of :

  a) alkyl or alkenyl radicals, aryl, arylalkyl, alkylaryl or alkenylaryl radicals, alicyclic or heterocyclic radicals, including polycyclic radicals ;
  b) alkyl or alkenyl radicals comprising at least one functional ether, thioether, amine, imine, carboxyl, carbonyl, hydroxy, silyl, isocyanate or thioisocyanate group ;
  c) aryl, arylalkyl, arylalkenyl, alkylaryl or alkenylaryl radicals, in which the aromatic nuclei and/or at least one substituent of the nucleus comprises heteroatoms selected from nitrogen, oxygen, sulfur ;
  d) radicals comprising condensed aromatic cycles which optionally comprise at least one heteroatom selected from nitrogen, oxygen, sulfur ;
  e) halogenated alkyl, alkenyl, aryl, arylalkyl, alkylaryl or alkenylaryl radicals in which the number of carbon atoms carrying at least one halogen is at least equal to the number of non-halogenated carbon atoms, the carbon in $\alpha$ position of group Y not being halogenated when Y is $-SO_2-$, said radicals optionally comprising functional ether, thioether, amine, imine, carboxyl, carbonyl, hydroxy, silyl, isocyanate or isothiocyanate groups ;
  f) radicals $R_CC(R')(R'')-O-$ in which $R_C$ is an perfluoroalkyl radical and R' and R'' are independently from one another, an hydrogen atom or a radical as defined in a), b), c) or d) above ;
  g) radicals $(R_B)_2N-$, in which the $R_B$ radicals, identical or different, are as defined in a), b), c), d) and e) above, one of the $R_B$ may be a hydrogen atom, or the two radicals $R_B$ together form a bivalent radical which forms a cycle with N ;
  h) radicals consisting of a polymer chain ;
  i) radicals having one or more cationic ionophoric groups and/or one or more anionic ionophoric groups ;

  with the proviso that a substituent $R_D$ may be a monovalent radical, part of a multivalent radical carrying a plurality of $R_FS(O)_x$-N-Y- groups, or a segment of a polymer;
  with the proviso that, when Y is a sulfonyl, and $R_D$ is a radical such as defined in a), $R_F$ is $R_ACF_2-$, $R_ACF_2CF_2-$, $R_ACF_2CF(CF_3)-$, $CF_3C(R_A)F-$ or a perhaloalkyl radical having 1 to 2 carbon atoms ;
  with the provisio that, when a radical is a polymer or part of a polymer, said polymer is a polyalkylene optionally carrying substituents, a polystyrene, a poly(fluoroethylene), a polyacrylate, a polyester, a poly(oxyalkylene), a poly-(alcoxysilyl), a polyaniline, a polymer having recurring units derived from 2[2-(3-thienyl)ethoxy]ethyl or from 3-maleimidopropyle.

2. Ionically conducting material according to claim 1, **characterized in that** the onium organic cation $M^{+m}$ is selected from the group consisting of $R_3O^+$ (oxonium), $NR_4^+$ (ammonium), $RC(NHR_2)_2^+$ (amidinium), $C(NHR_2)_3^+$ (guanidinium), $C_5R_6N^+$ (pyridinium), $C_3R_5N_2^+$ (imidazolium), $C_2R_4N_3^+$ (triazolium), $C_3R_7N_2^+$ (imidazolinium), $SR_3^+$ (sulfonium), $PR_4^+$ (phosphonium), $IR_2^+$ (iodonium), $(C_6R_5)_3C^+$ (carbonium) cations, the radicals R independently representing H or a radical selected from the group consisting of:

- alkyl, alkenyl, oxa-alkyl, oxa-alkenyl, aza-alkyl, aza-alkenyl, thia-alkyl, thia-alkenyl, sila-alkyl, sila-alkenyl, aryl,

arylalkyl, alkylaryl, alkenylaryl radicals, dialkylamino radicals and dialkylazo radicals ;
- cyclic or heterocyclic radicals optionally comprising at least one side chain comprising heteroatoms such as nitrogen, oxygen, sulfur ;
- cyclic or heterocyclic radicals optionally comprising heteroatoms in the aromatic nuclei ;
- groups comprising a plurality of aromatic or heterocyclic, condensed or noncondensed nuclei, optionally containing at least one nitrogen, oxygen, sulfur or phosphorus atom ;

with the proviso that a plurality of radicals R may together form aromatic or non aromatic cycles optionally enclosing the center carrying the cationic charge.

**3.** Ionically conducting material according to claim 2, **characterized in that** cation onium is part of the radical Z or of the radical $R_D$.

**4.** Ionically conducting material according to claim 2, **characterized in that** the onium cation is part of a recurring unit of a polymer.

**5.** Ionically conducting material according to claim 2, **characterized in that** the onium cation is a cationic heterocycle with aromatic character, having at least one alkylated nitrogen atom in the cycle.

**6.** Ionically conducting material according to claim 5, **characterized in that** the onium cation is an imidazolium, a triazolium, a pyridinium, a 4-dimethyl-amino-pyridinium, said cations optionally carrying a substituent on the carbon atoms of the cycle.

**7.** Ionically conducting material according to claim 2, **characterized in that** the onium cation is a group having a -N=N- or $-N=N^+$ bond, a sulfonium group, an iodonium group, or a substituted or non-substituted arene-ferrocenium cation, optionally incorporated in a polymer network.

**8.** Ionically conducting material according to claim 2, **characterized in that** the onium cation is a diaryliodonium cation, a dialkylaryliodonium cation, a triarylsulfonium cation, a trialkylaryl sulfonium cation, or a substituted or non-substituted phenacyl-dialkyl sulfonium cation.

**9.** Ionically conducting material according to claim 8, **characterized in that** the onium cation is part of a polymer chain.

**10.** Ionically conducting material according to claim 2, **characterized in that** M is an organic cation incorporating a group 2,2'[azobis(2-2'-imidazolinio-2-ylpropane]$^{2+}$ or 2,2'azobis(2-amidiniopropane)$^{2}$+.

**11.** Ionically conducting material according to claim 1, **characterized in that** the cation M is a metallic cation selected from the group consisting of cations of alkali metal, cations of alkali-earth metals, cations of transition metals, cations of trivalent metals, and cations of rare earth metal.

**12.** Ionically conducting material according to claim 1, **characterized in that** the organo-metallic cation is a metallocenium, selected from the group consisting of cations derived from ferrocene, titanocene, zirconocene, indenocenium cations, arene metallocenium cations, cations of transition metals complexed with ligands of phosphine type optionally having a chirality, and organometallic cations having one or more alkyl or aryl groups covalently fixed to an atom or a group of atoms, said cations optionally being part of a polymer chain.

**13.** Ionically conducting material according to claim 1, **characterized in that** $R_F$ is a fluorine atom or a perhalogenated alkyl radical having 1 to 12 carbon atoms, or a perhalogenated alkylaryl radical having 6 to 9 carbon atoms.

**14.** Ionically conducting material according to claim 1, **characterized in that** Z is a $R_D$-$SO_2$ group.

**15.** Ionically conducting material according to claim 1, **characterized in that** $R_D$ is selected from alkyl, alkenyl, oxa-alkyl, oxa-alkenyl, aza-alkyl, aza-alkenyl, thia-alkyl or thia-alkenyl radicals having 1 to 24 carbon atoms, or from aryl, arylalkyl, alkylaryl or alkenylaryl radicals having 5 to 24 carbon atoms and wherein the side chains and/or the aromatic nuclei have heteroatoms.

**16.** Ionically conducting material according to claim 1, **characterized in that** $R_D$ is selected from alkyl or alkenyl radicals having 1 to 12 carbon atoms and optionally comprising at least one heteroatom O, N or S in the main chain

or in a side chain, and/or optionally carrying a hydroxy group, a carbonyl group, an amine group, a carboxyl group, an isocyanate group or a isothiocyanate group.

**17.** Ionically conducting material according to claim 1, **characterized in that** $R_D$ is part of a poly(oxyalkylene) radical or a polystyrene radical.

**18.** Ionically conducting material according to claim 1, **characterized in that** $R_D$ is a radical having an iodonium, a sulfonium, oxonium, ammonium, amidinium, triazolium, guanidinium, pyridinium, imidazolium, phosphonium or carbonium group, said ionic group totally or partially acting as the cation $M^+$.

**19.** Ionically conducting material according to claim 1, **characterized in that** $R_D$ has one or more anionic ionophoric groups consisting of a carboxylate function ($-CO_2-$), a sulfonate function ($-SO_3-$), a sulfonimide function ($-SO_2NSO_2-$) or a sulfonamide function ($-SO_2N-$).

**20.** Ionically conducting material according to claim 1, **characterized in that** $R_D$ includes at least one ethylenic unsaturation and/or a condensable group and/or a group which is dissociable by thermal means, by photochemical means, or by ionic dissociation.

**21.** Ionically conducting material according to claim 1, **characterized in that** $R_D$ represents a mesomorphous group or a chromophorous group or a self-doped electronically conductive polymer or a hydrolyzable alkoxysilane.

**22.** Ionically conducting material according to claim 1, **characterized in that** Z represents a recurring unit of a polymer chain.

**23.** Ionically conducting material according to claim 1, **characterized in that** $R_D$-Y- is optically active.

**24.** Ionically conducting material according to claim 1, **characterized in that** $R_D$-Y- represents an amino acid, or an optically or biologically active polypeptide.

**25.** Ionically conducting material according to claim 1, **characterized in that** $R_D$ represents a radical having a valency v higher than 2, comprising at least one $R_F$-$S(O)_x$-N- group.

**26.** Ionically conducting material according to claim 1, **characterized in that** the substituent Z is selected from the group consisting of $-OC_nF_{2n+1}$, $-OC_2F_4H$, $-SC_nF_{2n+1}$ and $-SC_2F_4H$, $-OCF=CF_2$, $-SCF=CF_2$ and $C_nF2_{n+1}CH_2$- n being a whole nunber from 1 to 8.

**27.** Ionically conducting material according to claim 1, **characterized in that** Z comprises a heterocycle, derived from pyridine, pyrazine, pyrimidine, oxadiazole or thiadiazole, which is fluorinated or non-fluorinated.

**28.** Ionically conductive material according to claim 1, **characterized in that** the substituent $R_D$ of the ionic compound comprises an alkyl group, an aryl group, a alkylaryl group or an arylalkyl group; a mesomorphous group or a group comprising at least one ethylenic unsaturation and/or a condensable group and/or a group which is dissociable by thermal means, by photochemical means or by ionic dissociation ; a self-doped electronically conductive polymer ; a hydrolysable alkoxysilane ; a free radical trap; a dissociating dipole ; a redox couple ; a complexing ligand.

**29.** Ionically conductive material according to claim 1, **characterized in that** the substituent $R_D$ is a recurring unit of a polymer.

**30.** Ionically conductive material according to claim 1, **characterized in that** the solvent is either an aprotic liquid solvent, selected from linear ethers and cyclic ethers, esters, nitriles, nitro derivatives; amides, sulfones, sulfolanes, sulfamides and partially halogenated hydrocarbons, or a polar polymer, or a mixture thereof.

**31.** Ionically conductive material according to claim 1, **characterized in that** the solvent is a cross-linked or non-cross-linked solvating polymer, which may carry grafted ionic groups.

**32.** Ionically conductive material according to claim 31, **characterized in that** the solvating polymer is selected from polyethers of linear, comb or blocks structure, which may form a network, based on poly(ethylene oxide), copolymers containing ethylene oxide, propylene oxide or allyl-glycidylether units, polyphosphazenes, cross-linked networks based on polyethylene glycol crosslinked with isocyanates, networks obtained by polycondensation and carrying groups which enable the incorporation of cross-linkable groups and block copolymers in which some blocks carry functions which have redox properties.

**33.** Ionically conductive material according to claim 1, **characterized in that** the solvent essentially consists of a liquid aprotic solvent and a polar polymer solvent comprising units containing at least one heteroatom selected from sulfur, oxygen, nitrogen and fluorine.

**34.** Ionically conductive material according to claim 33, **characterized in that** the polar polymer mainly contains units derived from acrylonitrile, vinylidene fluoride, N-vinylpyrrolidone or methyl methacrylate.

**35.** Ionically conductive material according to claim 1, **characterized in that** it additionally contains at least one second salt.

**36.** Ionically conductive material according to claim 1, **characterized in that** it additionally contains a mineral or organic filler in the form of powder or fibres.

**37.** Electrochemical generator comprising a negative electrode and a positive electrode both separated by an electrolyte, **characterized in that** the electrolyte is a material according to one of claims 1 to 36.

**38.** Generator according to claim 37, **characterized in that** the negative electrode consists of metallic lithium, or an alloy thereof, optionally in the form of nanometric dispersion in lithium oxide, or a double nitride of lithium and a transition metal, or an oxide with low potential having the general formula $Li_{1+y+x/3}Ti_{2-x/3}O_4$ ($0 \leq x \leq 1$, $0 \leq y \leq 1$), or carbon and carbon products originating from pyrolysis of organic materials.

**39.** Generator according to claim 37, **characterized in that** the positive electrode is selected from vanadium oxides $VO_x$ ($2 \leq x \leq 2.5$), $LiV_3O_8$, $Li_yNi_{1-x}CO_xO_2$, ($0 \leq x \leq 1$ ; $0 \leq y \leq 1$), spinels of manganese $Li_yMn_{1-x}M_xO_2$ (M = Cr, Al, V, Ni, $0 \leq x \leq 0{,}5$ ; $0 \leq y \leq 5$ 2), organic polydisulfides FeS, $FeS_2$, iron sulfate $Fe_2(SO_4)_3$, phosphates and phosphosilicates of iron and lithium of olivine structure, or substituted products where iron is substituted by manganese, used alone or in admixtures.

**40.** Generator according to claim 37, **characterized in that** the collector of the cathode is made of aluminum.

**41.** Supercapacitor utilizing at least one carbon electrode with high specific surface, or an electrode containing a redox polymer, in which the electrolyte is a material according to one of claims 1 to 36.

**42.** Use of a material according to one of claims 1 to 36 for p or n doping of an electronically conductive polymer.

**43.** Electrochrome device in which the electrolyte is a material according to one of claims 1 to 36.

**44.** Electronically conductive material **characterized in that** it comprises an ionically conducting material according to claim 1.

**45.** Electronically conductive material according to claim 44, **characterized in that** the cationic part of the ionic compound is a polycation consisting of a doped conjugated polymer "p".

**46.** Electronically conductive material according to claim 44, **characterized in that** the substituent Z of the ionic compound comprises an alkyl chain having 6 to 20 carbon atoms.

**Patentansprüche**

**1.** Ionenleitendes Material, das eine ionische Verbindung in Lösung in einem Lösungsmittel umfasst, wobei die ionische Verbindung aus einem Amid oder einem Salz davon besteht, umfassend einen anionischen Anteil, der mit zumindest einem kationischen Anteil $M^{+m}$ in ausreichender Zahl assoziiert ist, um elektrische Neutralität des Ganzen zu gewährleisten, wobei das Material **dadurch gekennzeichnet ist, dass** das Kation $M^{+m}$ ein Hydroxonium-, ein Nitrosonium- ($NO^+$), ein Ammonium- ($-NH_4^+$), ein Metallkation mit der Wertigkeit m, ein organisches Oniumkation mit der Wertigkeit m oder ein organometallisches Kation mit der Wertigkeit m ist, sowie dadurch, dass der anionische Anteil der Formel $R_F$-$SO_x$-$N^-$-Z entspricht, worin:

- die Gruppe -$S(O)_x$- eine Sulfonylgruppe -$SO_2$- oder eine Sulfinylgruppe -SO- darstellt;
- $R_F$ ein perhalogenierter Alkyl-, Alkylaryl, Oxaalkyl-, Azaalkyl- oder Thiaalkylrest ist oder ein Rest, der einer der Formeln $R_ACF_2$-, $R_ACF_2CF_2$-, $R_ACF_2CF(CF_3)$- oder $CF_3C$-$(R_A)F$- entspricht, worin $R_A$- für einen nicht perhalogenierten organischen Rest steht, der aus Alkyl-, Aryl- Alkylaryl- oder Arylalkylresten; mesomorphen Gruppen oder Gruppen, die zumindest eine ethylenische Unsättigung und/oder eine kondensierbare Gruppe und/oder eine thermisch, photochemisch oder durch ionische Dissoziation dissoziierbare Gruppe umfassen; eigendotierten elektronenleitenden Polymeren; hydrolysierbaren Alkoxysilanen; dissoziierbaren Dipolen; Redoxpaaren; komplexierenden Liganden; chromophoren Gruppen; Polymerketten, die Pfropfäste aufweisen, die eine Carbonylgruppe; eine Sulfonylgruppe, eine Thionylgruppe oder eine Phosphonylgruppe umfassen; Zwitterionen; optisch oder biologisch aktiven Aminosäuren oder Polypeptiden; und chiralen Gruppen ausgewählt ist;
- Z einen Elektronen-anziehenden Rest mit einem Hammett-Parameter darstellt, der zumindest dem eines Phenylrests entspricht, ausgewählt aus:

  j) -CN, -$NO_2$, -SCN, -$N_3$, -$CF_3$, $R'_FCH_2$- (wobei $R'_F$ ein perfluorierter Rest ist), Fluoralkylthioxy-Resten,
  jj) Resten mit einem oder mehreren aromatischen Kernen, die gegebenenfalls zumindest ein Stickstoff-, Sauerstoff-, Schwefel- oder Phosphoratom enthalten, wobei die Kerne gegebenenfalls kondensierte Kerne sind und/oder die Kerne gegebenenfalls zumindest einen Substituenten aufweisen, der aus Halogenen, -CN, -$NO_2$, -SCN, -$N_3$, -$CF_3$, $CF_3CH_2$-, $CF_2$=CF-O-, $CF_2$=CF-S-, Perfluoralkylgruppen, Fluoralkyloxygruppen, Fluoralkylthioxygruppen, Alkyl-, Alkenyl, Oxaalkyl-, Oxaalkenyl-, Azaalkyl-, Azaalkenyl-, Thiaalkyl-, Thiaalkenylresten, Polymerresten, sowie Resten, die zumindest eine kationische ionophore Gruppe und/oder zumindest eine anionische ionophore Gruppe aufweisen, ausgewählt ist;

  mit der Maßgabe, dass ein Substituent Z ein einwertiger Rest, eine Einheit eines mehrwertigen Rests, der mehrere Gruppen $R_F$-$S(O)_x$-N- aufweist, oder ein Polymersegment sein kann;
  oder aber:
- Z ein Rest $R_D$-Y- ist, worin Y eine Sulfonyl- oder Sulfinylgruppe ist und $R_D$ ein Rest ist, der aus der aus folgenden bestehenden Gruppe ausgewählt ist:

  a) Alkyl- oder Alkenylresten, Aryl-, Arylalkyl-, Alkylaryl- oder Alkenylarylresten, alizyklischen oder heterozyklischen Resten, einschließlich polyzyklischen Resten;
  b) Alkyl- oder Alkenylresten, die zumindest eine funktionelle Ether-, Thioether-, Amino-, Imino-, Carboxyl-, Carbonyl- Hydroxy-, Silyl-, Isocyanat- oder Isothiocyanatgruppe umfassen;
  c) Aryl-, Arylalkyl-, Arylalkenyl-, Alkylaryl- oder Alkenylarylresten, worin die aromatischen Kerne und/oder zumindest ein Kernsubstituent Heteroatome, wie z.B. Stickstoff, Sauerstoff oder Schwefel, umfassen;
  d) Resten, die kondensierte aromatische Ringe umfassen, die gegebenenfalls zumindest ein aus Stickstoff, Sauerstoff und Schwefel ausgewähltes Heteroatom umfassen,
  e) halogenierten Alkyl-, Alkenyl-, Aryl-, Arylalkyl- Alkylaryl- oder Alkenylarylresten, worin die Anzahl der Kohlenstoffatome, die zumindest ein Halogen tragen, zumindest gleich der Anzahl an nicht halogenierten Kohlenstoffatomen ist, wobei der $\alpha$-Kohlenstoff der Gruppe Y nicht halogeniert ist, wenn Y -$SO_2$- ist, wobei die Reste gegebenenfalls funktionelle Ether-, Thioether-, Amino-, Imino-, Carboxyl-, Carbonyl, Hydroxy-, Silyl-, Isocyanat- oder Isothiocyanatgruppen umfassen;
  f) Resten $R_CC(R')(R'')$-O-, worin $R_C$ ein perfluorierter Alkylrest ist und R' und R'' unabhängig voneinander ein Wasserstoffatom oder ein Rest wie zuvor unter a), b), c) oder d) definiert ist;
  g) Resten $(R_B)_2N$-, worin die Reste $R_B$, die identisch oder unterschiedlich sein können, wie zuvor unter a), b), c), d) und e) definiert sind, wobei ein $R_B$ ein Wasserstoffatom sein kann, oder die beiden Reste $R_B$ gemeinsam einen zweiwertigen Rest bilden, der mit N einen Ring bildet;
  h) Resten, die aus einer Polymerkette bestehen;
  i) Resten, die eine oder mehrere kationische ionophore Gruppen und/oder eine oder mehrere anionische ionophore Gruppen aufweisen;

- mit der Maßgabe, dass ein Substituent $R_D$ ein einwertiger Rest, eine Einheit eines mehrwertigen Rests, der mehrere Gruppen $R_FS(O)_x$-N-Y- aufweist, oder ein Polymersegment sein kann;
- mit der Maßgabe, dass, wenn Y ein Sulfonyl ist und $R_D$ ein Rest wie unter a) definiert ist, $R_F$ $R_ACF_2$-, $R_ACF_2CF_2$-, $R_ACF_2CF(CF_3)$-, $CF_3C(R_A)F$- oder ein perhalogenierter Alkylrest mit 1 bis 2 Kohlenstoffatomen

ist;

- mit der Maßgabe, dass, wenn ein Rest ein Polymer oder Teil eines Polymers ist, das Polymer ein Polyalkylen, das gegebenenfalls Substituenten aufweist, ein Polystyrol, ein Poly(fluorethylen), ein Polyacrylat, ein Polyester, ein Poly(oxyalkylen), ein Poly(alkoxysilan), ein Polyanilin oder ein Polymer mit Grundeinheiten ist, die von 2-[2-(3-Thienyl)ethoxy]ethylen oder 3-Maleimidopropyl abgeleitet sind.

**2.** Ionenleitendes Material nach Anspruch 1, **dadurch gekennzeichnet, dass** das organische Oniumkation $M^{+m}$ aus der aus den Kationen $R_3O^+$ (Oxonium), $NR_4^+$ (Ammonium), $RC(NHR_2)_2^+$ (Amidinium), $C(NHR_2)_3^+$ (Guanidinium), $C_5R_6N^+$ (Pyridinium), $C_3R_5N_2^+$ (Imidazolium), $C_2R_4N_3^+$ (Triazolium), $C_3R_7N_2^+$ (Imidazolinium), $SR_3^+$ (Sulfonium), $PR_4^+$ (Phosphonium), $IR_2^+$ (Iodonium) und $(C_6R_5)_3C^+$ (Carbonium) bestehenden Gruppe ausgewählt ist, wobei die Reste R unabhängig voneinander ein H oder einen Rest darstellen, der aus der aus Folgenden bestehenden Gruppe ausgewählt ist:

- Alkyl-, Alkenyl-, Oxaalkyl-, Oxaalkenyl-, Azaalkyl-, Azaalkenyl-, Thiaalkyl-, Thiaalkenyl-, Silaalkyl-, Silaalkenyl, Aryl-, Arylalkyl-, Alkylaryl-, Alkenylarylresten, Dialkylaminoresten und Dialkylazoresten;
- zyklischen oder heterozyklischen Resten, die gegebenenfalls zumindest eine Seitenkette umfassen, die Heteroatome, wie z.B. Stickstoff, Sauerstoff oder Schwefel, umfasst;
- zyklischen oder heterozyklischen Resten, die gegebenenfalls Heteroatome im aromatischen Kern umfassen;
- Gruppen, die mehrere kondensierte oder nichtkondensierte, aromatische oder heterozyklische Kerne umfassen, die gegebenenfalls zumindest ein Stickstoff-, Sauerstoff-, Schwefel- oder Phosphoratom enthalten;

mit der Maßgabe, dass mehrere Reste R gegebenenfalls gemeinsam aromatische Ringe bilden können, die gegebenenfalls das die kationische Ladung tragende Zentrum umfassen.

**3.** Ionenleitendes Material nach Anspruch 2, **dadurch gekennzeichnet, dass** das Oniumkation Bestandteil von Rest Z oder von Rest $R_D$ ist.

**4.** Ionenleitendes Material nach Anspruch 2, **dadurch gekennzeichnet, dass** das Oniumkation Bestandteil einer Grundeinheit eines Polymers ist.

**5.** Ionenleitendes Material nach Anspruch 2, **dadurch gekennzeichnet, dass** das Oniumkation ein kationischer Heterozyklus mit aromatischer Beschaffenheit ist, der im Ring zumindest ein alkyliertes Stickstoffatom aufweist.

**6.** Ionenleitendes Material nach Anspruch 5, **dadurch gekennzeichnet, dass** das Oniumkation Imidazolium, Triazolium, Pyridinium oder 4-Dimethylaminopyridinium ist, wobei die Kationen gegebenenfalls einen Substituenten auf den Kohlenstoffatomen des Rings tragen.

**7.** Ionenleitendes Material nach Anspruch 2, **dadurch gekennzeichnet, dass** das Oniumkation eine Gruppe ist, die eine -N=N- oder $-N=N^+$-Bindung, eine Sulfoniumgruppe, eine Iodoniumgruppe oder ein substituiertes oder unsubstituiertes Aren-Ferrocen-Kation ist, das gegebenenfalls in einem Polymereinschluss enthalten ist.

**8.** Ionenleitendes Material nach Anspruch 2, **dadurch gekennzeichnet, dass** das Oniumkation ein substituiertes oder unsubstituiertes Diaryliodoniumkation, Dialkylaryliodoniumkation, Triarylsulfoniumkation, Trialkylarylsulfoniumkation oder Phenacyldialkylsulfoniumkation ist.

**9.** Ionenleitendes Material nach Anspruch 8, **dadurch gekennzeichnet, dass** das Oniumkation Teil einer Polymerkette ist.

**10.** Ionenleitendes Material nach Anspruch 2, **dadurch gekennzeichnet, dass** M ein organisches Kation ist, das eine Gruppe 2,2'-[Azobis(2,2'-imidazolinio-2-yl)propan]$^{2+}$ oder 2,2'-Azobis(2-amidiniopropan)$^{2+}$ umfasst.

**11.** Ionenleitendes Material nach Anspruch 1, **dadurch gekennzeichnet, dass** das Kation M ein Metallkation ist, das aus der aus Alkalimetallkationen, Erdalkalimetallkationen, Übergangsmetallkationen, dreiwertigen Metallkationen und Seltenderdkationen bestehenden Gruppe ausgewählt ist.

**12.** Ionenleitendes Material nach Anspruch 1, **dadurch gekennzeichnet, dass** das organometallische Kation ein Metallocenium ist, das aus der aus den von Ferrocen, Titanocen, Zirconocen abgeleiteten Kationen, Indenoceniumkationen, Arenmetalloceniumkationen, Kationen von Übergangsmetallen, die mit Liganden vom Phosphintyp komplexiert sind, die gegebenenfalls Chiralität aufweisen, und organometallischen Kationen, die ein oder mehrere Alkyl- oder Arylgruppen aufweisen, die kovalent an ein Atom oder eine Atomgruppe gebunden sind, bestehenden Gruppe ausgewählt ist, wobei die Kationen gegebenenfalls Teil einer Polymerkette sind.

**13.** Ionenleitendes Material nach Anspruch 1, **dadurch gekennzeichnet, dass** $R_F$ ein Fluoratom oder ein perhalogenierter Alkylrest mit 1 bis 12 Kohlenstoffatomen oder ein perhalogenierter Alkylarylrest mit 6 bis 9 Kohlenstoffatomen ist.

**14.** Ionenleitendes Material nach Anspruch 1, **dadurch gekennzeichnet, dass** Z eine Gruppe $R_D$-$SO_2$- ist.

**15.** Ionenleitendes Material nach Anspruch 1, **dadurch gekennzeichnet, dass** $R_D$ aus Alkyl-, Alkenyl-, Oxaalkyl-, Oxaalkenyl-, Azaalkyl-, Azaalkenyl-, Thiaalkyl- oder Thiaalkenylresten mit 1 bis 24 Kohlenstoffatomen ausgewählt ist oder aus Aryl-, Arylalkyl-, Alkylaryl- oder Alkenylarylresten, die 5 bis 24 Kohlenstoffatome aufweisen und in denen die Seitenketten und/oder die aromatischen Kerne Heteroatome umfassen.

**16.** Ionenleitendes Material nach Anspruch 1, **dadurch gekennzeichnet, dass** $R_D$ aus Alkyl- oder Alkenylresten mit 1 bis 12 Kohlenstoffatomen ausgewählt ist, die gegebenenfalls zumindest ein Heteroatom O, N oder S in der Hauptkette oder in einer Seitenkette umfassen, und/oder die gegebenenfalls eine Hydroxygruppe, eine Carbonylgruppe, eine Aminogruppe, einer Carboxylgruppe, eine Isocyanatgruppe oder eine Isothiocyanatgruppe tragen.

**17.** Ionenleitendes Material nach Anspruch 1, **dadurch gekennzeichnet, dass** $R_D$ Teil eines Poly(oxyalkylen)rests oder eines Polystyrolrests ist.

**18.** Ionenleitendes Material nach Anspruch 1, **dadurch gekennzeichnet, dass** $R_D$ ein Rest ist, der eine Iodoniumgruppe, eine Sulfonium-, Oxonium-, Ammonium-, Amidinium-, Triazolium-, Guanidinium-, Pyridinium-, Imidazolium-, Phosphonium- oder Carboniumgruppe aufweist, wobei die ionische Gruppe vollständig oder teilweise die Rolle von Kation $M^{+m}$ übernimmt.

**19.** Ionenleitendes Material nach Anspruch 1, **dadurch gekennzeichnet, dass** $R_D$ eine oder mehrere, anionische, ionophore Gruppen aufweist, die durch eine Carboxylatfunktionalität ($-CO_2^-$), eine Sulfonatfunktionalität ($-SO_3^-$), eine Sulfonimidfunktionalität ($-SO_2NSO_2-$) oder eine Sulfonamidfunktionalität ($-SO_2N-$) gebildet werden.

**20.** Ionenleitendes Material nach Anspruch 1, **dadurch gekennzeichnet, dass** $R_D$ zumindest eine ethylenische Unsättigung und/oder eine kondensierbare Gruppe und/oder eine thermisch, photochemisch oder durch ionische Dissoziation dissoziierbare Gruppe umfasst.

**21.** Ionenleitendes Material nach Anspruch 1, **dadurch gekennzeichnet, dass** $R_D$ eine mesomorphe Gruppe oder eine chromophore Gruppe oder ein eigendotiertes elektronenleitendes Polymer oder ein hydrolysierbares Alkoxysilan darstellt.

**22.** Ionenleitendes Material nach Anspruch 1, **dadurch gekennzeichnet, dass** Z eine Grundeinheit einer Polymerkette darstellt.

**23.** Ionenleitendes Material nach Anspruch 1, **dadurch gekennzeichnet, dass** $R_D$-Yoptisch aktiv ist.

**24.** Ionenleitendes Material nach Anspruch 1, **dadurch gekennzeichnet, dass** $R_D$-Yeine Aminosäure oder ein optisch oder biologisch aktives Polypeptid darstellt.

**25.** Ionenleitendes Material nach Anspruch 1, **dadurch gekennzeichnet, dass** $R_D$ einen Rest mit einer Wertigkeit v > 2 darstellt, der selbst zumindest eine Gruppe $R_F$-$S(O)_x$-N- trägt.

**26.** Ionenleitendes Material nach Anspruch 1, **dadurch gekennzeichnet, dass** der Substituent Z aus der aus $-OC_nF_{2n+1}$, $-OC_2F_4H$, $-SC_nF_{2n+1}$ und $-SC_2F_4H$, $-OCF=CF_2$, $-SCF=CF_2$ und $C_nF_{2n+1}CH_2$- bestehenden Gruppe ausgewählt ist, wobei n eine ganze Zahl von 1 bis 8 ist.

**27.** Ionenleitendes Material nach Anspruch 1, **dadurch gekennzeichnet, dass** Z einen Heterozyklus umfasst, der von Pyridin, Pyrazin, Pyrimidin, Oxadiazol oder Thiadiazol abgeleitet ist, und der gegebenenfalls fluoriert ist.

**28.** Ionenleitendes Material nach Anspruch 1, **dadurch gekennzeichnet, dass** der Substituent $R_D$ der ionischen Verbindung eine Alkylgruppe, eine Arylgruppe, eine Alkylarylgruppe oder eine Arylalkylgruppe; eine mesomorphe Gruppe oder eine Gruppe, die zumindest eine ethylenische Unsättigung aufweist, und/oder eine kondensierbare Gruppe und/oder eine thermisch, photochemisch oder durch ionische Dissoziation dissoziierbare Gruppe; ein eigendotiertes elektronenleitendes Polymer; ein hydrolysierbares Alkoxysilan; einen Radikalfänger; einen dissoziierbaren Dipol; ein Redoxpaar; oder einen komplexierenden Liganden umfasst.

**29.** Ionenleitendes Material nach Anspruch 1, **dadurch gekennzeichnet, dass** der Substituent $R_D$ eine Grundeinheit eines Polymers ist.

**30.** Ionenleitendes Material nach Anspruch 1, **dadurch gekennzeichnet, dass** das Lösungsmittel entweder ein flüssiges, aprotisches Lösungsmittel, das aus linearen und zyklischen Ethern, Esterns, Nitrilen, Nitroderivaten, Amiden, Sulfonen, Sulfolanen, Alkylsulfamiden, und Kohlenwasserstoffen die teilweise halogeniert sind, oder ein polares Polymer oder ein Gemisch davon ist.

**31.** Ionenleitendes Material nach Anspruch 1, **dadurch gekennzeichnet, dass** das Lösungsmittel ein gegebenenfalls vernetztes, solvatisierendes Polymer ist, das gegebenenfalls aufgepfropfte ionische Gruppen aufweist.

**32.** Ionenleitendes Material nach Anspruch 31, **dadurch gekennzeichnet, dass** das solvatisierende Polymer aus Polyethern mit linearer, kammförmiger oder blockförmiger Struktur, die gegebenenfalls ein Netzwerk bilden auf Basis von Poly(ethylenoxid), Copolymeren, die eine Ethylenoxid- oder Propylenoxid- oder Allylglycidylether-Einheit enthalten, Polyphosphazenen, vernetzten Netzwerken auf Basis von Polyethylenglykol, das durch Isocyanate vernetzt ist, Netzwerken, die durch Polykondensation erhalten werden und Gruppen aufweisen, die den Einbau vernetzbarer Gruppen ermöglichen, und Blockcopolymeren ausgewählt sind, in denen bestimmte Blöcke Funktionalitäten enthälten, die Redoxeigenschaften aufweisen.

**33.** Ionenleitendes Material nach Anspruch 1, **dadurch gekennzeichnet, dass** das Lösungsmittel im Wesentlichen aus einem flüssigen aprotischen Lösungsmittel und einem polaren Polymerlösungsmittel besteht, das Einheiten umfasst, die zumindest ein aus Schwefel, Sauerstoff, Stickstoff und Fluor ausgewähltes Heteroatom enthalten.

**34.** Ionenleitendes Material nach Anspruch 33, **dadurch gekennzeichnet, dass** das polare Polymer vorwiegend Einheiten enthält, die von Acrylnitril, Vinylidenfluorid, N-Vinylpyrrolidon oder Methylmethacrylat herrühren.

**35.** Ionenleitendes Material nach Anspruch 1, **dadurch gekennzeichnet, dass** es weiters ein zweites Salz enthält.

**36.** Ionenleitendes Material nach Anspruch 1, **dadurch gekennzeichnet, dass** es weiters einen mineralischen oder organischen Füllstoff in Pulver- oder Faserform enthält.

**37.** Elektrochemischer Generator, der eine negative Elektrode und eine positive Elektrode umfasst, die durch einen Elektrolyten voneinander getrennt sind, **dadurch gekennzeichnet, dass** der Elektrolyt ein Material nach einem der Ansprüche 1 bis 36 ist.

**38.** Generator nach Anspruch 37, **dadurch gekennzeichnet, dass** die negative Elektrode aus metallischem Lithium oder einer Legierung davon, gegebenenfalls in Form einer nanometrischen Dispersion in Lithiumoxid, oder aus einem Lithium-Übergangsmetall-Doppelnitrid oder aus einem Oxid mit niedrigem Potential der allgemeinen Formel $Li_{1+y+x/3}Ti_{2-x/3}O_4$ ($0 \leq x \leq 1$, $0 \leq y \leq 1$), oder aus Kohlenstoff und kohlenstoffhältigen Produkten der Pyrolyse organischer Materialien besteht.

**39.** Generator nach Anspruch 37, **dadurch gekennzeichnet, dass** die positive Elektrode aus Vanadiumoxiden $VO_x$ ($2 \leq x \leq 2.5$), $LiV_3O_8$, $Li_yNi_{1-x}CO_xO_2$, ($0 \leq x \leq 1$ ; $0 \leq y \leq 1$), Manganspinellen $Li_yMn_{1-x}M_xO_2$ (M = Cr, Al, V, Ni, $0 \leq x \leq 0{,}5$ ; $0 \leq y \leq 5$ 2), organischen Polydisulfiden FeS, $FeS_2$, Eisensulfat $Fe_2(SO_4)_3$, Eisen- und Lithiumphosphaten und - phophosilikaten mit Olivinstruktur oder Produkten davon, die durch Ersetzen von Eisen durch Mangan erhalten sind, alleine oder als Gemisch ausgewählt ist.

**40.** Generator nach Anspruch 37, **dadurch gekennzeichnet, dass** der Stromabnehmer der Kathode aus Aluminium besteht.

**41.** Superkondensator, bei dem zumindest eine Kohlenstoffelektrode mit großer spezifischer Oberfläche oder eine

Elektrode eingesetzt wird, die ein Redoxpolymer enthält, worin der Elektrolyt ein Material nach einem der Ansprüche 1 bis 36 ist.

**42.** Verwendung eines Materials nach einem der Ansprüche 1 bis 36 zur p- oder n-Dotierung eines elektronenleitenden Polymers.

**43.** Elektrochrome Vorrichtung, in der der Elektrolyt ein Material nach einem der Ansprüche 1 bis 36 ist.

**44.** Elektronenleitendes Material, **dadurch gekennzeichnet, dass** es ein ionenleitendes Material nach Anspruch 1 enthält.

**45.** Elektronenleitendes Material nach Anspruch 44, **dadurch gekennzeichnet, dass** der kationische Anteil der ionischen Verbindung ein Polykation ist, das aus einem p-dotierten konjugierten Polymer besteht.

**46.** Elektronenleitendes Material nach Anspruch 44, **dadurch gekennzeichnet, dass** der Substituent Z der ionischen Verbindung eine Alkylkette mit 6 bis 20 Kohlenstoffatomen umfasst.

FIGURE 1

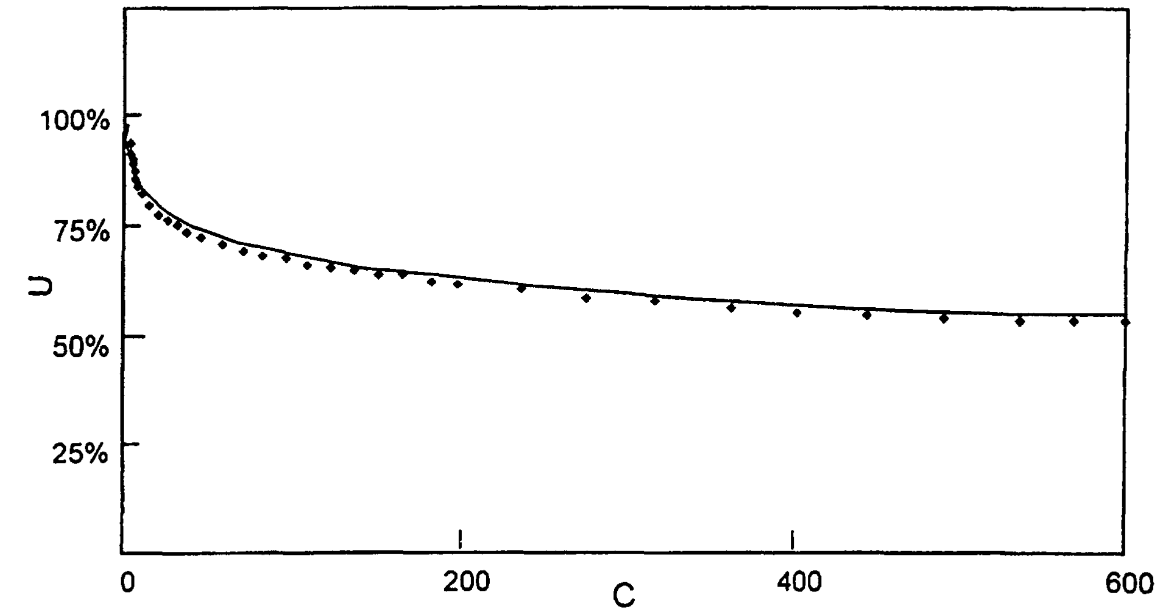


Figure 2